# EUROPEAN PATENT SPECIFICATION

(11) **EP 4 181 832 B1**
(45) Date of publication and mention of the grant of the patent: **11.03.2026**
(21) Application number: 21789985.5
(22) Date of filing: 16.09.2021
(51) Int. Cl.: A61F 2/24

(54) **PROSTHETIC VALVE SYSTEMS AND APPARATUSES**
PROTHESENKLAPPENSYSTEME UND -VORRICHTUNGEN
SYSTÈMES DE PROTHÈSE VALVULAIRE ET APPAREILS

(30) Priority: 18.09.2020 US 202062706944 P
(43) Date of publication of application: 24.05.2023
(73) Proprietor: Edwards Lifesciences Corporation, Irvine, CA 92614-5688 (US)
(72) Inventor: DASS, Ajay Kumar, Irvine, CA 92614 (US); LANDON, David Robert, Irvine, CA 92614 (US); SCHEINBLUM, Taylor Jacob, Newport Beach, CA 92660-3287 (US); LUONG, Hieu Minh, Westminster, CA 92683 (US)
(74) Representative: Venner, Julia Ann
(86) International application number: PCT/US2021/050718
(87) International publication number: WO 2022/061017

(56) References cited:
- WO-A1-2017/151566
- US-A1- 2014 243 966
- US-A1- 2015 142 104
- US-A1- 2019 053 895

## Description

### BACKGROUND

### Field

Certain embodiments disclosed herein relate generally to implants, including prosthetic valves for implantation.

### Background

Human heart valves, which include the aortic, pulmonary, mitral and tricuspid valves, function essentially as one-way valves operating in synchronization with the pumping heart. The valves allow blood to flow downstream, but block blood from flowing upstream. Diseased heart valves exhibit impairments such as narrowing of the valve or regurgitation, which inhibit the valves' ability to control blood flow. Such impairments reduce the heart's blood-pumping efficiency and can be a debilitating and life threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open heart surgery. Replacement valves are being designed to be delivered through minimally invasive procedures and even percutaneous procedures. Such replacement valves often include a tissue-based valve body that is connected to an expandable frame that is then delivered to the native valve's annulus.

These replacement valves are often intended to at least partially block blood flow. However, a problem occurs when blood flows around the valve on the outside of the prosthesis. For example, in the context of replacement heart valves, paravalvular leakage (PVL) has proven particularly challenging. An additional challenge relates to the ability of such prostheses to be secured relative to intralumenal tissue, e.g., tissue within any body lumen or cavity, in an atraumatic manner.

WO 2017/151566 describes methods, devices and systems for anchoring and/or sealing a heart valve prosthesis using inflatable elements.

US 2015/0142104 describes a prosthetic heart valve for replacing a native valve including a collapsible and expandable stent and a valve assembly disposed within the stent, the valve assembly including a plurality of leaflets and a cuff annularly disposed about the stent. The cuff includes a surplus portion capable of forming a sealing structure having a diameter greater than a diameter of the proximal end of the stent when deployed.

US 2019/0053895 describes a delivery assembly comprising an annular frame, a leaflet structure positioned within the frame and secured thereto, and an outer skirt positioned around an outer surface of the frame. The outer skirt can comprise pericardial tissue having a fibrous parietal layer defining a first surface of the outer skirt and a serous parietal layer defining a second surface of the outer skirt.

US 2014/0243966 describes a transcatheter valve prosthesis including an expandable tubular stent, a prosthetic valve within the stent, and an anti-paravalvular leakage component coupled to and encircling the tubular stent. The anti-paravalvular leakage component includes a radially-compressible annular scaffold, which is a sinusoidal patterned ring of self-expanding material, and an impermeable membrane extending over the annular scaffold. Alternatively, the anti-paravalvular leakage component includes a plurality of self-expanding segments and an annular sealing element coupled to inner surfaces of the segments.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention.

Embodiments of prosthetic valves may be directed to improvements in anchoring to an implantation site and/or sealing flow at an implantation site, which may comprise a native valve. Embodiments may be configured to conform to a shape of a native valve, which may be a non-circular shape. Such native valves may include native valve annuli having oval shapes and/or shapes that include one or more recesses.

Improvements in anchoring to implantation sites are disclosed as well.

Embodiments as disclosed herein may include a prosthetic valve configured to be deployed to a native valve. The prosthetic valve may include a plurality of prosthetic valve leaflets. The prosthetic valve may include a first anchor coupled to the plurality of prosthetic valve leaflets and configured to be positioned radially outward of an outward facing surface of a leaflet of the native valve. The prosthetic valve may include a second anchor coupled to the plurality of prosthetic valve leaflets and configured to abut against an inward facing surface of the leaflet of the native valve.

Embodiments as disclosed herein may include a method. The method may include delivering a prosthetic valve to a native valve, the prosthetic valve including a plurality of prosthetic valve leaflets and a first anchor and a second anchor. The method may include retaining a leaflet of the native valve between the first anchor positioned radially outward of an outward facing surface of the leaflet and the second anchor abutting against an inward facing surface of the leaflet of the native valve.

Embodiments as disclosed herein may include a prosthetic valve configured to be deployed to a native valve. The prosthetic valve may include a plurality of prosthetic valve leaflets. The prosthetic valve may include a frame coupled to the plurality of prosthetic valve leaflets. The prosthetic valve may include one or more sealing prongs protruding radially outward from the frame and each configured to reduce fluid flow at a portion of the native valve.

Embodiments as disclosed herein may include a method. The method may include deploying a prosthetic valve to a native valve. The prosthetic valve may include a plurality of prosthetic valve leaflets, a frame coupled to the plurality of prosthetic valve leaflets, and one or more sealing prongs each configured to protrude radially outward from the frame and each configured to reduce fluid flow at a portion of the native valve.

Embodiments as disclosed herein may include a prosthetic valve configured to be deployed to a native valve. The prosthetic valve may include a plurality of prosthetic valve leaflets. The prosthetic valve may include one or more distal anchors coupled to the plurality of prosthetic valve leaflets and configured to extend over a distal tip of a leaflet of the native valve and horizontally from the distal tip of the leaflet to a tip of the respective distal anchor.

Embodiments as disclosed herein may include a method. The method may include deploying a prosthetic valve to a native valve. The prosthetic valve may include a plurality of prosthetic valve leaflets, and one or more distal anchors coupled to the plurality of prosthetic valve leaflets and configured to extend over a distal tip of a leaflet of the native valve and horizontally from the distal tip of the leaflet to a tip of the respective distal anchor.

Embodiments as disclosed herein may include a prosthetic valve configured to be deployed to a native valve. The prosthetic valve may include a plurality of prosthetic valve leaflets. The prosthetic valve may include one or more distal anchors coupled to the plurality of prosthetic valve leaflets and configured to extend over a distal tip of a leaflet of the native valve and couple to a ventricular heart wall.

Embodiments as disclosed herein may include a method. The method may include delivering a prosthetic valve to a native valve, the prosthetic valve including a plurality of prosthetic valve leaflets and one or more distal anchors. The method may include extending the one or more distal anchors over a distal tip of a leaflet of the native valve. The method may include coupling the one or more distal anchors to a ventricular heart wall.

Embodiments as disclosed herein may include a prosthetic valve configured to be deployed to a native valve. The prosthetic valve may include a plurality of prosthetic valve leaflets. The prosthetic valve may include a braid material coupled to the plurality of prosthetic valve leaflets and configured to have a shape that varies to vary an outer diameter of the prosthetic valve.

Embodiments as disclosed herein may include a method. The method may include deploying a prosthetic valve to a native valve. The prosthetic valve may include a plurality of prosthetic valve leaflets and a braid material coupled to the plurality of prosthetic valve leaflets and configured to have a shape that varies to vary an outer diameter of the prosthetic valve.

Embodiments as disclosed herein may include a prosthetic valve configured to be deployed to a native valve. The prosthetic valve may include a plurality of prosthetic valve leaflets. The prosthetic valve may include an anchor coupled to the plurality of prosthetic valve leaflets and configured to couple to a portion of a heart. The prosthetic valve may include an indicator mechanism configured to indicate the coupling of the anchor to the portion of the heart.

Embodiments as disclosed herein may include a method. The method may include visualizing an indicator mechanism coupled to a prosthetic valve including a plurality of prosthetic valve leaflets. The method may include determining a coupling of an anchor of the prosthetic valve to a portion of a patient's heart based on the visualized indicator mechanism.

Embodiments as disclosed herein may include a prosthetic valve configured to be deployed to a native valve. The prosthetic valve may include a plurality of prosthetic valve leaflets. The prosthetic valve may include an inner frame supporting the plurality of prosthetic valve leaflets. The prosthetic valve may include a sealing body positioned radially outward from the inner frame and configured to seal against a portion of the native valve and configured to have an adjustable outer diameter.

Embodiments as disclosed herein may include a method. The method may include deploying a prosthetic valve to a native valve. The prosthetic valve may include a plurality of prosthetic valve leaflets, an inner frame supporting the plurality of prosthetic valve leaflets, and a sealing body positioned radially outward from the inner frame and configured to seal against a portion of the native valve and configured to have an adjustable outer diameter.

Embodiments as disclosed herein may include a prosthetic valve configured to be deployed to a native valve. The prosthetic valve may include a plurality of prosthetic valve leaflets. The prosthetic valve may include an inner frame supporting the plurality of prosthetic valve leaflets. The prosthetic valve may include an inflatable body positioned radially outward from the inner frame and configured to seal against a portion of the native valve.

Embodiments as disclosed herein may include a method. The method may include deploying a prosthetic valve to a native valve. The prosthetic valve may include a plurality of prosthetic valve leaflets, an inner frame supporting the plurality of prosthetic valve leaflets, and an inflatable body positioned radially outward from the inner frame and configured to seal against a portion of the native valve.

Embodiments as disclosed herein may include a prosthetic valve configured to be deployed to a native valve. The prosthetic valve may include a plurality of prosthetic valve leaflets. The prosthetic valve may include a sealing body positioned radially outward from the plurality of prosthetic valve leaflets and including one or more strands configured to seal the sealing body against a portion of the native valve.

Embodiments as disclosed herein may include a method. The method may include deploying a prosthetic valve to a native valve. The prosthetic valve may include a plurality of prosthetic valve leaflets, and a sealing body positioned radially outward from the plurality of prosthetic valve leaflets and including one or more strands configured to seal the sealing body against a portion of the native valve.

Embodiments as disclosed herein may include a prosthetic valve configured to be deployed to a native valve. The prosthetic valve may include a plurality of prosthetic valve leaflets. The prosthetic valve may include a frame supporting the plurality of prosthetic valve leaflets. The prosthetic valve may include a frameless sealing body positioned radially outward from the plurality of prosthetic valve leaflets and configured to seal against a portion of the native valve.

Embodiments as disclosed herein may include a method. The method may include deploying a prosthetic valve to a native valve. The prosthetic valve may include a plurality of prosthetic valve leaflets, a frame supporting the plurality of prosthetic valve leaflets, and a frameless sealing body positioned radially outward from the plurality of prosthetic valve leaflets and configured to seal against a portion of the native valve.

Embodiments as disclosed herein may include a prosthetic valve configured to be deployed to a native valve. The prosthetic valve may include a plurality of prosthetic valve leaflets. The prosthetic valve may include a valve body supporting the plurality of prosthetic valve leaflets. The prosthetic valve may include one or more anchors configured to anchor the valve body to the native valve and each having a diameter configured to increase.

Embodiments as disclosed herein may include a method. The method may include deploying a prosthetic valve to a native valve. The prosthetic valve may include a plurality of prosthetic valve leaflets, a valve body supporting the plurality of prosthetic valve leaflets, and one or more anchors configured to anchor the valve body to the native valve and each having a diameter configured to increase.

Embodiments as disclosed herein may include a prosthetic valve configured to be deployed to a native valve. The prosthetic valve may include a plurality of prosthetic valve leaflets. The prosthetic valve may include a valve body having a proximal portion and a distal portion and supporting the plurality of prosthetic valve leaflets. The prosthetic valve may include one or more anchors positioned at the distal portion of the valve body, each of the one or more anchors having a first portion extending proximally to a tip of the respective anchor, the first portion passing through at least a portion of the valve body.

Embodiments as disclosed herein may include a method. The method may include deploying a prosthetic valve to a native valve. The prosthetic valve may include a plurality of prosthetic valve leaflets, a valve body having a proximal portion and a distal portion and supporting the plurality of prosthetic valve leaflets, and one or more anchors positioned at the distal portion of the valve body, each of the one or more anchors having a first portion extending proximally to a tip of the respective anchor, the first portion passing through at least a portion of the valve body.

Embodiments as disclosed herein may include a prosthetic valve configured to be deployed to a native valve. The prosthetic valve may include a plurality of prosthetic valve leaflets. The prosthetic valve may include an inner frame supporting the plurality of prosthetic valve leaflets and having a proximal portion and a distal portion. The prosthetic valve may include a plurality of anchors positioned at the distal portion of the inner frame and configured to couple to the native valve. The prosthetic valve may include a sealing body positioned radially outward from the inner frame and having a proximal end and a distal end that is coupled to the distal portion of the inner frame such that the sealing body is configured to move with respect to the inner frame.

Embodiments as disclosed herein may include a method. The method may include deploying a prosthetic valve to a native valve. The prosthetic valve may include a plurality of prosthetic valve leaflets, an inner frame supporting the plurality of prosthetic valve leaflets and having a proximal portion and a distal portion, a plurality of anchors positioned at the distal portion of the inner frame and configured to couple to the native valve, and a sealing body positioned radially outward from the inner frame and having a proximal end and a distal end that is coupled to the distal portion of the inner frame such that the sealing body is configured to move with respect to the inner frame.

Embodiments as disclosed herein may include a system for delivery of a prosthetic valve into a portion of a patient's body. The system may include an elongate shaft configured for insertion into a portion of the patient's body. The system may include a capsule coupled to the elongate shaft and configured to at least partially release the prosthetic valve, the capsule extending about an axis and including at least two sides surrounding the axis, a first side of the at least two sides configured to retract axially relative to a second side of the at least two sides.

Embodiments as disclosed herein may include a method. The method may include utilizing a delivery system to deploy a prosthetic valve to a native valve. The delivery system may include an elongate shaft configured for insertion into a portion of a patient's body, and a capsule coupled to the elongate shaft and configured to at least partially release the prosthetic valve, the capsule extending about an axis and including at least two sides surrounding the axis, a first side of the at least two sides configured to retract axially relative to a second side of the at least two sides.

### BRIEF DESCRIPTION OF THE DRAWINGS

Features and advantages of the systems, apparatuses, and methods as disclosed herein will become appreciated as the same become better understood with reference to the specification, claims, and appended drawings wherein:
FIG. 1 illustrates a perspective view of a prosthetic valve according to an embodiment of the present disclosure.
FIG. 2 illustrates a side perspective view of the prosthetic valve shown in FIG. 1.
FIG. 3 illustrates a cross sectional schematic view of the prosthetic valve shown in FIG. 1.
FIG. 4 illustrates a schematic view of a delivery apparatus passing into a portion of a patient's body according to an embodiment of the present disclosure.
FIG. 5 illustrates a schematic view of a prosthetic valve being deployed according to an embodiment of the present disclosure.
FIG. 6 illustrates a schematic view of the prosthetic valve shown in FIG. 5 being deployed.
FIG. 7 illustrates a schematic view of the prosthetic valve shown in FIG. 5 being deployed.
FIG. 8 illustrates a schematic view of the prosthetic valve shown in FIG. 5 being deployed.
FIG. 9 illustrates a schematic top view of a valve annulus according to an embodiment of the present disclosure.
FIG. 10 illustrates a plan view of a frame according to an embodiment of the present disclosure.
FIG. 11 illustrates a cross sectional schematic view of a prosthetic valve according to an embodiment of the present disclosure.
FIG. 12 illustrates a top schematic view of the prosthetic valve shown in FIG. 11.
FIG. 13 illustrates a top schematic view of the prosthetic valve shown in FIG. 11 with a sealing skirt positioned thereon.
FIG. 14 illustrates a top schematic view of the prosthetic valve shown in FIG. 11 positioned within a valve annulus according to an embodiment of the present disclosure.
FIG. 15 illustrates a top schematic view of the prosthetic valve shown in FIG. 11 positioned within a valve annulus and with a sealing skirt positioned thereon according to an embodiment of the present disclosure.
FIG. 16 illustrates a cross sectional schematic view of a prosthetic valve according to an embodiment of the present disclosure.
FIG. 17 illustrates a side perspective view of a prosthetic valve according to an embodiment of the present disclosure.
FIG. 18 illustrates a bottom perspective view of the prosthetic valve shown in FIG. 17 according to an embodiment of the present disclosure.
FIG. 19 illustrates a cross sectional schematic view of a prosthetic valve implanted within a native heart valve according to an embodiment of the present disclosure.
FIG. 20 illustrates a cross sectional schematic view of a prosthetic valve implanted within a native heart valve according to an embodiment of the present disclosure.
FIG. 21 illustrates a cross sectional schematic view of a prosthetic valve implanted within a native heart valve according to an embodiment of the present disclosure.
FIG. 22 illustrates a plan view of a frame according to an embodiment of the present disclosure.
FIG. 23 illustrates a plan view of a frame according to an embodiment of the present disclosure.
FIG. 24 illustrates a schematic view of an anchor tip approaching a ventricular wall according to an embodiment of the present disclosure.
FIG. 25 illustrates a cross sectional schematic view of a prosthetic valve according to an embodiment of the present disclosure.
FIG. 26 illustrates a perspective view of an anchor tip according to an embodiment of the present disclosure.
FIG. 27 illustrates a cross sectional schematic view of a prosthetic valve implanted within a native heart valve according to an embodiment of the present disclosure.
FIG. 28 illustrates a perspective view of a braid material according to an embodiment of the present disclosure.
FIG. 29 illustrates a perspective view of the braid material shown in FIG. 28 elongated.
FIG. 30 illustrates a cross sectional schematic view of a prosthetic valve according to an embodiment of the present disclosure.
FIG. 31 illustrates a top schematic view of the prosthetic valve shown in FIG. 30.
FIG. 32 illustrates a top schematic view of the prosthetic valve shown in FIG. 31 with a varied outer diameter.
FIG. 33 illustrates a cross sectional schematic view of a prosthetic valve according to an embodiment of the present disclosure.
FIG. 34 illustrates a top schematic view of the prosthetic valve shown in FIG. 33.
FIG. 35 illustrates a top schematic view of the prosthetic valve shown in FIG. 34 with a varied outer diameter.
FIG. 36 illustrates a cross sectional view of a portion of the prosthetic valve shown in FIG. 33.
FIG. 37 illustrates a cross sectional schematic view of a prosthetic valve according to an embodiment of the present disclosure.
FIG. 38 illustrates a cross sectional schematic view of a prosthetic valve missing capture of a leaflet of a native valve according to an embodiment of the present disclosure.
FIG. 39 illustrates a cross sectional schematic view of the prosthetic valve shown in FIG. 38 capturing a leaflet.
FIG. 40 illustrates a side view of an indicator mechanism according to an embodiment of the present disclosure.
FIG. 41 illustrates a side view of the indicator mechanism shown in FIG. 40.
FIG. 42 illustrates a side view of an indicator mechanism according to an embodiment of the present disclosure.
FIG. 43 illustrates a side view of the indicator mechanism shown in FIG. 42.
FIG. 44 illustrates a side view of an indicator mechanism according to an embodiment of the present disclosure.
FIG. 45 illustrates a side view of the indicator mechanism shown in FIG. 44.
FIG. 46 illustrates a side view of an indicator mechanism according to an embodiment of the present disclosure.
FIG. 47 illustrates a side view of the indicator mechanism shown in FIG. 46.
FIG. 48 illustrates a side view of the indicator mechanism shown in FIG. 46.
FIG. 49 illustrates a plan view of a tip of an anchor according to an embodiment of the present disclosure.
FIG. 50 illustrates a cross sectional schematic view of a prosthetic valve according to an embodiment of the present disclosure.
FIG. 51 illustrates a perspective schematic view of a prosthetic valve according to an embodiment of the present disclosure.
FIG. 52 illustrates a close up view of a strand entering a channel according to an embodiment of the present disclosure.
FIG. 53A illustrates a perspective schematic view of the prosthetic valve shown in FIG. 51 according to an embodiment of the present disclosure.
FIG. 53B illustrates a perspective schematic view of a prosthetic valve according to an embodiment of the present disclosure.
FIG. 54 illustrates a cross sectional schematic view of the prosthetic valve shown in FIG. 51 positioned within a native heart valve.
FIG. 55 illustrates a cross sectional schematic view of the prosthetic valve shown in FIG. 54 positioned within a native heart valve.
FIG. 56 illustrates a top schematic view of the prosthetic valve shown in FIG. 51 positioned within a valve annulus.
FIG. 57 illustrates a cross sectional schematic view of a prosthetic valve according to an embodiment of the present disclosure.
FIG. 58 illustrates a top cross sectional schematic view of the prosthetic valve shown in FIG. 57 according to an embodiment of the present disclosure.
FIG. 59 illustrates a top cross sectional schematic view of the prosthetic valve shown in FIG. 57 according to an embodiment of the present disclosure.
FIG. 60 illustrates a schematic view of a plurality of spacer bodies according to an embodiment of the present disclosure.
FIG. 61 illustrates a perspective schematic view of a prosthetic valve according to an embodiment of the present disclosure.
FIG. 62 illustrates a perspective schematic view of a prosthetic valve according to an embodiment of the present disclosure.
FIG. 63 illustrates a perspective schematic view of a prosthetic valve according to an embodiment of the present disclosure.
FIG. 64 illustrates a perspective schematic view of the prosthetic valve shown in FIG. 63.
FIG. 65 illustrates a perspective schematic view of a prosthetic valve according to an embodiment of the present disclosure.
FIG. 66 illustrates a perspective schematic view of the prosthetic valve shown in FIG. 65.
FIG. 67 illustrates a cross sectional schematic view of the prosthetic valve shown in FIG. 65.
FIG. 68 illustrates a cross sectional schematic view of the prosthetic valve shown in FIG. 65.
FIG. 69 illustrates a cross sectional schematic view of a prosthetic valve according to an embodiment of the present disclosure.
FIG. 70 illustrates a cross sectional schematic view of the prosthetic valve shown in FIG. 69.
FIG. 71 illustrates a cross sectional schematic view of the prosthetic valve shown in FIG. 69.
FIG. 72 illustrates a cross sectional schematic view of a prosthetic valve according to an embodiment of the present disclosure.
FIG. 73 illustrates a cross sectional schematic view of a prosthetic valve positioned within a native heart valve according to an embodiment of the present disclosure.
FIG. 74 illustrates a top cross sectional schematic view of the prosthetic valve shown in FIG. 73.
FIG. 75 illustrates a cross sectional schematic view of the prosthetic valve shown in FIG. 73 positioned within a native heart valve.
FIG. 76 illustrates a top cross sectional schematic view of the prosthetic valve shown in FIG. 73.
FIG. 77 illustrates a side view of an anchor according to an embodiment of the present disclosure.
FIG. 78 illustrates a side view of the anchor shown in FIG. 77 according to an embodiment of the present disclosure.
FIG. 79 illustrates a cross sectional view of a strand according to an embodiment of the present disclosure.
FIG. 80 illustrates a cross sectional view of the strand shown in FIG. 79 with an expanded diameter according to an embodiment of the present disclosure.
FIG. 81 illustrates a cross sectional schematic view of a prosthetic valve according to an embodiment of the present disclosure.
FIG. 82 illustrates a cross sectional schematic view of a prosthetic valve according to an embodiment of the present disclosure.
FIG. 83 illustrates a top schematic view of the prosthetic valve shown in FIG. 82.
FIG. 84 illustrates a cross sectional schematic view of a prosthetic valve according to an embodiment of the present disclosure.
FIG. 85 illustrates a top schematic view of the prosthetic valve shown in FIG. 84.
FIG. 86 illustrates a cross sectional schematic view of a prosthetic valve according to an embodiment of the present disclosure.
FIG. 87 illustrates a cross sectional schematic view of the prosthetic valve shown in FIG. 86.
FIG. 88 illustrates a perspective view of the prosthetic valve shown in FIG. 86.
FIG. 89 illustrates a top view of the prosthetic valve shown in FIG. 86.
FIG. 90 illustrates a schematic view of the prosthetic valve shown in FIG. 86 being deployed according to an embodiment of the present disclosure.
FIG. 91 illustrates a schematic view of the prosthetic valve shown in FIG. 86 being deployed according to an embodiment of the present disclosure.
FIG. 92 illustrates a schematic view of the prosthetic valve shown in FIG. 86 deployed according to an embodiment of the present disclosure.
FIG. 93 illustrates a perspective view of a delivery apparatus according to an embodiment of the present disclosure.
FIG. 94 illustrates a perspective view of a capsule of the delivery apparatus shown in FIG. 93.
FIG. 95 illustrates a perspective schematic view of the capsule shown in FIG. 93, with a side retracted.
FIG. 96 illustrates a perspective schematic view of a capsule of a delivery apparatus, with a side retracted.
FIGS. 97A-97F illustrate a deployment sequence of a prosthetic valve utilizing the capsule shown in FIG. 93.

### DETAILED DESCRIPTION

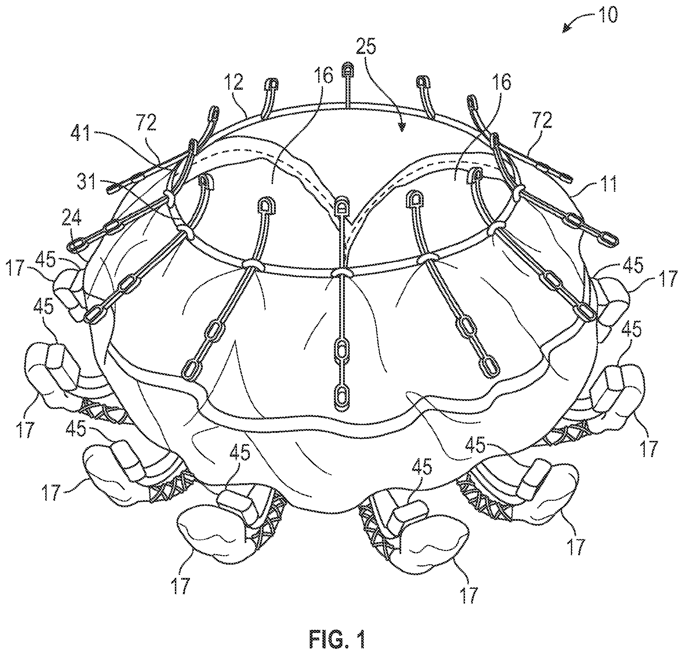

FIG. 1 illustrates a perspective view of a prosthetic valve 10 in the form of a replacement heart valve. The prosthetic valve 10 may be configured to be deployed within a portion of a patient's body. The prosthetic valve 10, for example, may be deployed to an annulus of a native valve, which may comprise a native mitral valve or a native tricuspid valve. In embodiments other implantation locations may be utilized such as within an aortic or pulmonary valve, or in other valve or locations within a patient's body as desired.

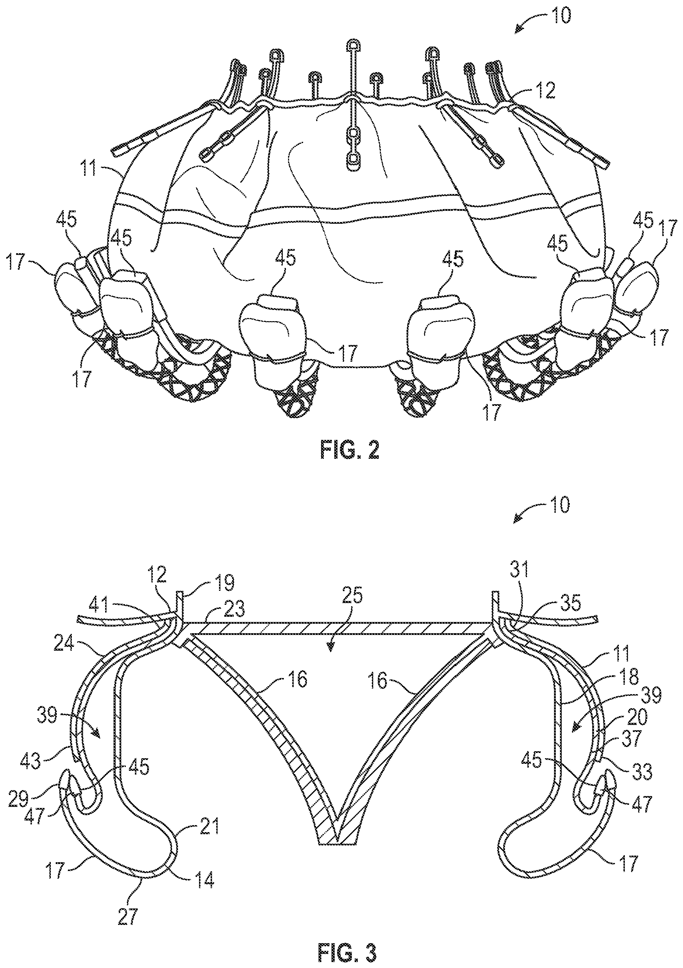

The prosthetic valve 10 may include a proximal end 12 and a distal end 14 (marked in FIG. 3), and a length therebetween. The prosthetic valve 10 may further include a plurality of prosthetic valve leaflets 16 configured to surround a flow channel for controlling flow through the valve 10. The prosthetic valve leaflets 16 may be configured to move between opened and closed states to mimic and replace the operation of native valve leaflets.

In embodiments, the prosthetic valve leaflets 16 may be coupled to a frame. The frame may include an inner frame or valve frame 18 as shown in a cross sectional view in FIG. 3 and may include an outer frame 20 as shown in FIG. 3, which may be part of a sealing body 11.

FIG. 3 illustrates a cross sectional schematic view of the valve 10. As marked in FIG. 3, the valve frame 18 may include a proximal portion including a proximal end 19 and a distal portion including a distal end 21. The valve frame 18 may have a curved configuration, comprising a curved body that curves radially outward between the proximal end 19 and the distal end 21, or may have another configuration in embodiments as desired.

The proximal portion of the valve frame 18 may be coupled to a proximal portion of the plurality of prosthetic valve leaflets 16. The valve frame 18 may support the prosthetic valve leaflets 16. The prosthetic valve leaflets 16 may be coupled to the valve frame 18 and may extend radially inward from the valve frame 18. The prosthetic valve leaflets 16 may couple to the valve frame 18 via an intermediate body 23 that may support the prosthetic valve leaflets 16 and may couple the leaflets 16 to the valve frame 18 via sutures or another method as desired.

The prosthetic valve leaflets 16 may surround a flow channel 25 as marked in FIG. 3 and may move between open and closed states to control flow through the flow channel 25. As shown in FIG. 3, the proximal end of the prosthetic valve 10 may comprise an inflow end of the valve 10, and the distal end of the prosthetic valve 10 may comprise an outflow end, although other configurations may be utilized as desired.

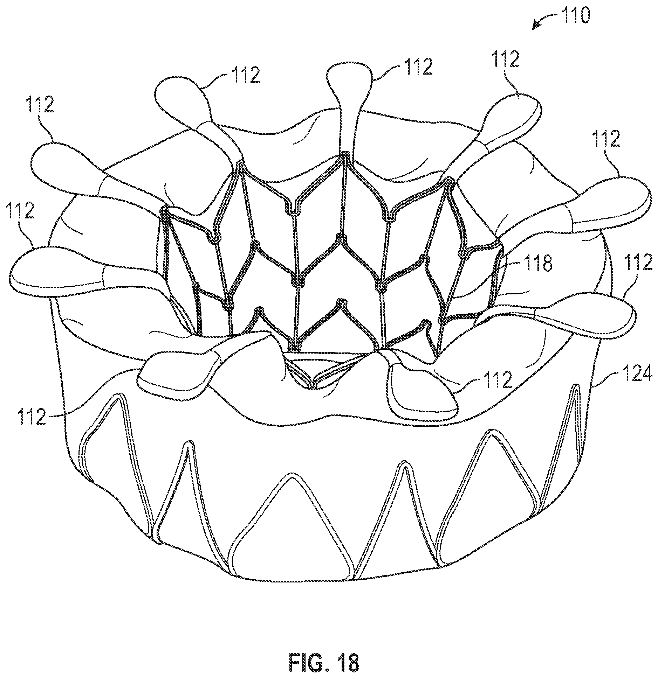

The valve frame 18 may include a plurality of struts spaced from each other with spaces. Such a configuration is shown, for example, in FIG. 18 (with struts of the valve frame 118 visible in FIG. 18). Such a configuration may allow the valve frame 18 to move between an undeployed, unexpanded, or linearized configuration to a deployed or expanded configuration. For example, the valve frame 18 may expand radially outward to move to the deployed or expanded configuration, with the length of the valve frame 18 decreasing due to the increased diameter of the valve frame 18. Other configurations of valve frames 18 may be utilized as desired.

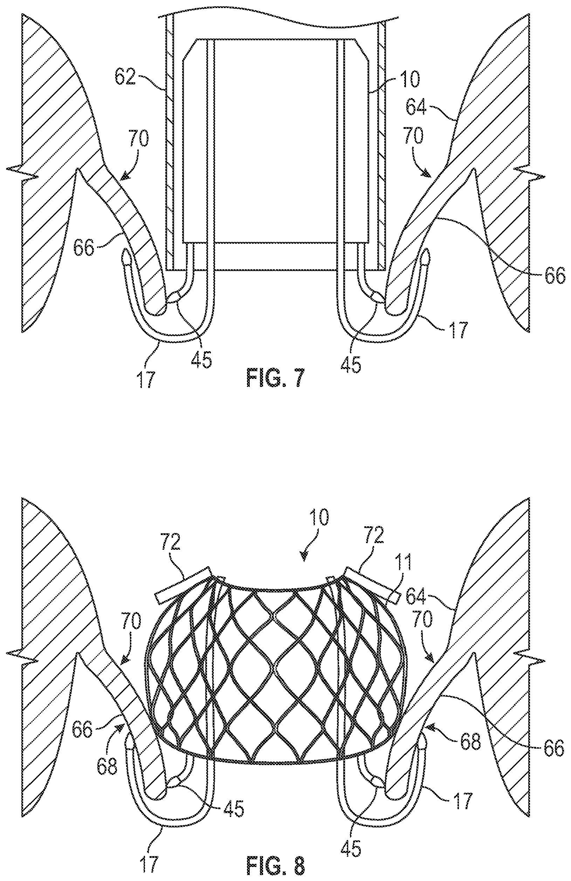

Referring to FIG. 1, the prosthetic valve 10 may include one or more anchors 17 that may be coupled to the plurality of prosthetic valve leaflets 16 and each may be configured to anchor to a portion of a patient's heart. The anchors 17 may particularly be configured to anchor to the native valve leaflets of the patient's heart. The anchors 17 may extend around the native valve leaflets to anchor to the native valve leaflets. The anchors 17 may be configured to be positioned radially outward of an outward facing surface 68 of a leaflet 66 of the native valve as shown in FIG. 8 for example. The anchors 17 may comprise distal anchors positioned at the distal end 14 of the valve 10, or in embodiments may be positioned in another position as desired.

The anchors 17 may each extend radially outward from the flow channel 25 and radially outward from the prosthetic valve leaflets 16 of the valve 10. FIG. 3, for example, illustrates that the anchors 17 may be coupled to the distal portion of the valve frame 18 comprising an inner frame of the valve 10. The anchors 17 may each include a proximal portion 27 and a distal portion 29, with the proximal portion 27 coupled to the valve frame 18 and the distal portion 29 comprising a tip of the respective anchor 17. The anchors 17 may extend vertically from the proximal portion 27 to the tip at the distal portion 29 when the valve 10 is deployed.

As shown in FIG. 3, each anchor 17 is configured as a protruding arm configured to extend distally and then curve in a proximal direction to the tip of the respective one of the anchors 17. Such a configuration may allow the anchor 17 to extend around a native leaflet and around the distal tip of the leaflet, to hook the native leaflet and be positioned radially outward of an outward facing surface of a leaflet of the native valve. The anchor 17 may thus resist a force applied in the atrial or proximal direction to the valve 10 and may anchor the valve 10 within the native valve annulus. Other configurations of anchors 17 may be utilized in embodiments as desired.

The anchors 17 are shown in FIGS. 1-3 in a deployed or expanded configuration, in which the tips of the anchors 17 extend proximally. In embodiments, the anchors 17 may be configured to be in undeployed, unexpanded, or linearized configuration in which the tips of the anchors 17 extend distally. Upon deployment, the anchors 17 may be configured to move from the undeployed configuration radially outward to the deployed configuration, with the tips flipped towards the proximal direction. Such an operation may allow the anchors 17 to flip over the native valve leaflets to anchor to the native valve leaflets during deployment. Other deployment methods for the anchors 17 may be utilized in embodiments as desired.

Referring to FIG. 1, the valve 10 may include a sealing body 11. The sealing body 11 may be positioned radially outward from the leaflets 16 and may comprise the outer surface of the valve 10. The sealing body 11 may define the outer diameter of the valve 10 and may comprise the outer periphery of the valve 10. The sealing body 11 may include a proximal portion having a proximal end 31 and may include a distal portion having a distal end 33 (marked in FIG. 3).

The sealing body 11 may include a frame 20 and a sealing skirt 24, or in embodiments may comprise only a frame or only a sealing skirt as desired. The sealing skirt 24 is shown in FIG. 1. The frame 20 may comprise an outer frame that is positioned radially outward from the inner valve frame 18. The sealing skirt 24 may be coupled to the frame 20 and may comprise the outer portion of the sealing body 11 as shown in FIG. 1.

Referring to FIG. 3, the outer frame 20 comprises at least a portion of the sealing body 11 that is configured to apply a seal to a portion of a heart. The outer frame 20 may have a proximal portion 35 that couples to the proximal end 19 of the valve frame 18. The proximal portion 35 may extend radially outward from the proximal end 19 of the valve frame 18 and from the prosthetic valve leaflets 16. A distal portion 37 of the frame 20 may be spaced from the prosthetic valve leaflets 16 and the valve frame 18 with a gap 39. The gap 39 may be positioned between the outer frame 20 of the sealing body 11 and a distal portion of the valve frame 18. The valve frame 18 accordingly may comprise an inner frame and the frame 20 of the sealing body 11 may comprise an outer frame positioned radially outward of the inner valve frame 18 and surrounding the inner valve frame 18 and the prosthetic valve leaflets 16.

As shown in FIG. 3, the outer frame 20 may have a length that extends distally to a lesser distance than the distal end of the valve frame 18. As such, the outer frame 20 may be shorter than the valve frame 18. The outer frame 20 may further have a curved configuration that curves outward from the valve frame 18, with a greatest diameter of the outer frame 20 being at the distal portion of the outer frame 20.

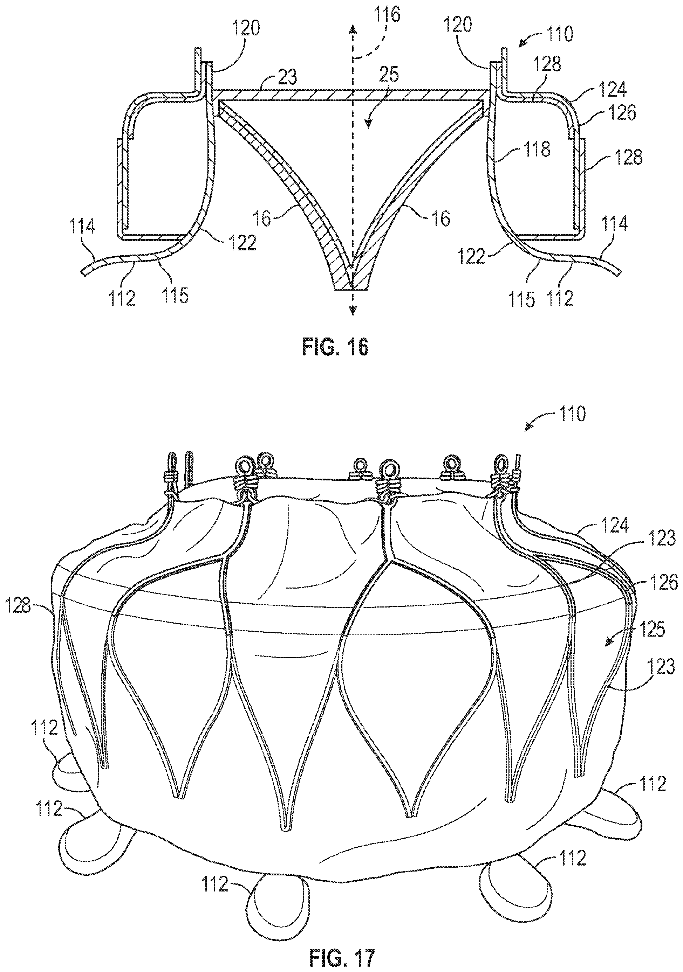

The outer frame 20 of the sealing body 11 may include a plurality of struts forming the frame 20, with spaces between the struts. Such a configuration is shown in FIG. 17 for example, with the struts 123 of the outer frame 126 separated by spaces 125. Such a configuration utilized with the frame 20 may allow the frame 20 to move between an undeployed, unexpanded, or linearized configuration to a deployed or expanded configuration as shown in FIG. 1, in which the outer frame 20 and sealing body 11 have a curved bulbous shape. As with the valve frame 18, the length of the outer frame 20 of the sealing body 11 may decrease as the diameter of the outer frame 20 of the sealing body 11 increases during deployment. The diameter of the outer frame 20 of the sealing body 11 may radially expand outward from the inner valve frame 18 simultaneously, or at a different time or rate of expansion as the inner valve frame 18 in embodiments.

The sealing body 11 may include a sealing skirt 24 that may extend around the inner valve frame 18 and the prosthetic valve leaflets 16. The sealing skirt 24 may be coupled to the frame 20 of the sealing body or may be free from the frame 20 in embodiments.

The sealing skirt 24 may have a proximal portion 41 that is coupled to the proximal portion of the frame 20 of the sealing body 11 and may be coupled to the proximal portion of the valve frame 18. The skirt 24 may have a distal portion 43 (marked in FIG. 3) that may be coupled to the distal end of the frame 20, and in embodiments may be coupled to the inner valve frame 18 or one or more of the anchors 17. As shown in FIG. 3, the anchors 17 may be configured to extend radially outward from the inner valve frame 18 and across the gap 39 to the tip of the respective anchor 17.

The sealing skirt 24 may be made of a material that resists fluid flow therethrough, such as a cloth material, woven material, or other material such as a polymer or other material that resists fluid flow therethrough. A variety of materials may be utilized for the skirt 24 as desired.

The sealing body 11 may be configured to abut a portion of the patient's heart to reduce fluid flow. For example, the sealing body 11 may abut a surface of a patient's native valve leaflet to reduce fluid flow between the sealing body 11 and the native leaflet. The sealing body 11 may be configured to abut other portions of the patient's heart to reduce fluid flow as desired.

One or more anchors 45 may be coupled to the plurality of prosthetic valve leaflets 16. The anchors 45 may be configured to abut against an inward facing surface 70 of a leaflet 66 of a native valve, as shown for example in FIG. 8. The anchors 45 may be coupled to the frame of the prosthetic valve 10 and, in particular, to the outer frame 20. The anchors 45 may be coupled to the distal portion 37 of the outer frame 20.

The anchors 45 may extend radially outward from the prosthetic valve 10 and radially outward from the prosthetic valve leaflets 16. The anchors 45 may be positioned at the same circumferential position as the anchors 17 as shown in FIG. 1, or in embodiments may be circumferentially staggered from the positions of the anchors 17. In an embodiment in which the anchors 45 are positioned at the same circumferential position as the anchors 17, the respective distal tips of the anchors 45, 17 may meet as shown in FIG. 1 for example.

The anchors 45, 17 may each comprise protrusions that extend radially outward from the outer frame 20 and the inner valve frame 18. The anchors 45 may comprise protruding arms extending from the distal portion of the outer frame 20 towards the anchors 17. The anchors 45 may curve from the outer frame 20 to a distal portion 47 of the anchor 45 comprising a distal tip of the anchor 45. The anchors 45, for example, may each curve distally from the outer frame 20 as shown in FIG. 3 and then proximally to the distal tip of the anchor 45 toward the distal tip of the opposing anchor 17.

In embodiments, one or more of the anchors 45, 17 may be biased to provide a force towards each other and may be configured to retain a body, such as a native heart valve leaflet, between the anchors 45, 17. The anchors 45, 17 may be configured to contact each other in the absence of a body positioned between the anchors 45, 17, as shown in FIG. 1 for example. The anchors 45, 17 may be biased towards each other to pinch a body such as a native heart valve leaflet between the anchors 45, 17. One or more of the anchors 45, 17, for example, may be made of a shape memory material that may be shape set with a bias towards each other. The shape memory material may comprise nitinol or another shape memory material in embodiments.

The anchors 45 may comprise inner anchors of the prosthetic valve 10 and the anchors 17 may comprise outer anchors of the prosthetic valve 10.

The anchors 45, 17 may be configured to be deployed sequentially, with the anchors 17 being first deployed and the anchors 45 then being deployed. Such a configuration may allow the anchors 17 to first be placed into position radially outward of a native heart valve leaflet and then the anchors 45 to be placed into position radially inward of the native heart valve leaflet to retain the leaflet therebetween. In embodiments, the outer frame 20 may be deployed after the anchors 17 are deployed, to allow for the sequential deployment of the anchors 45.

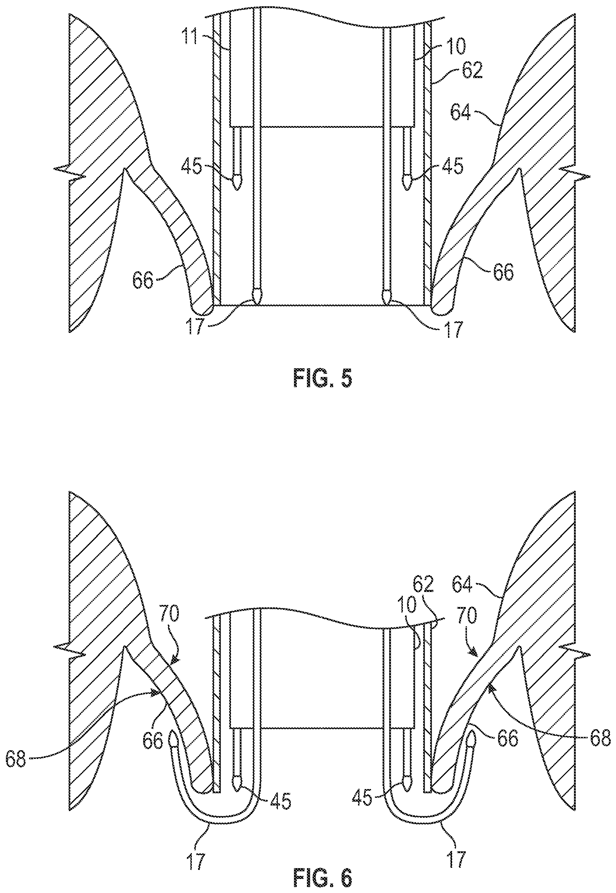

FIG. 7, for example, illustrates the outer frame 20 being deployed after the anchors 17 are deployed. The prosthetic valve 10 may be configured to move from an undeployed configuration as shown in FIG. 5 to a deployed configuration as shown in FIG. 8. The anchors 17 may be configured to move relatively towards the anchors 45 when the prosthetic valve 10 moves from the undeployed configuration to the deployed configuration.

In embodiments, the anchors 45, 17 may be deployed simultaneously.

The anchors 45, 17 may be utilized to retain a native heart valve leaflet between the anchors 45, 17 such that the native heart valve leaflet does not move during or following deployment of the prosthetic valve 10. The anchors 45, 17 may beneficially reduce the possibility of a body such as a native heart valve leaflet shifting position (which may include slipping out from the anchors) during or following deployment of the prosthetic valve 10. The anchors 45, 17 may further enhance securement of the prosthetic valve 10 to the native heart valve.

FIG. 2 illustrates a side view of the prosthetic valve 10. The anchors 45, 17 are shown to extend radially outward from the distal portion of the frame. The anchors 45, 17 are biased to be in contact with each other in the absence of a body such as a native heart valve leaflet between the anchors 45, 17.

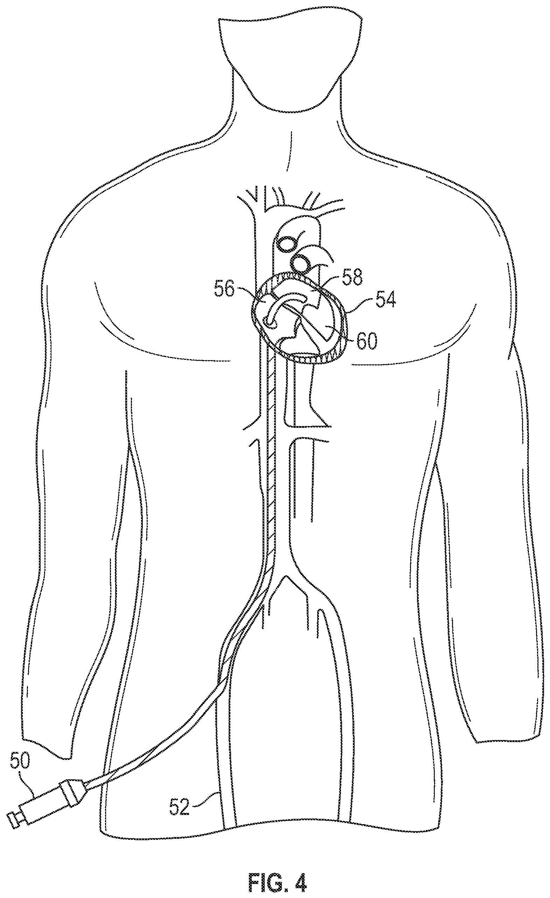

FIGS. 4-8 illustrate an exemplary method of deploying the prosthetic valve 10. In embodiments, the method may be modified as desired, including removing steps, adding steps, or utilizing steps, systems, or apparatuses from various other embodiments as desired.

The method may include delivering a prosthetic valve to a native valve of a patient's body. Referring to FIG. 4, a delivery apparatus 50 may be passed percutaneously into a patient's body in a minimally invasive manner. In other embodiments, more invasive means may be utilized as desired.

The delivery apparatus 50 may be utilized for transcatheter delivery of the valve. The delivery apparatus 50 may pass transvenous through the femoral artery 52 or another portion of the patient's vasculature. For example, transjugular entry or other methods of entry may be utilized as desired. The delivery apparatus 50 may pass to the patient's heart 54.

The delivery apparatus 50 may be used to deliver the valve to the tricuspid valve, and as such, may be positioned within the right atrium 56 of the patient's heart for delivery to the tricuspid valve. In an embodiment in which delivery is to the mitral valve, the delivery apparatus 50 may pass transseptal to the left atrium 58 for delivery to the mitral valve. The delivery apparatus 50 may advance towards the left ventricle 60 of the patient's heart for mitral delivery.

FIG. 5 illustrates the prosthetic valve 10 positioned within a capsule 62 of the delivery apparatus 50 for approach to a valve, such as the native mitral valve 64. The valve 10 may be passed out of the capsule 62 of the delivery apparatus 50 to be deployed to the native valve. The anchors 17 of the prosthetic valve 10 are shown in an undeployed or linearized state in FIG. 5 and the anchors 45 are also shown in an undeployed or linearized state. The sealing body 11 may be restrained from radially expanding outward by the constrictive force of the capsule 62. Additional features of the valve 10 are excluded from view in FIGS. 5-7.

The capsule 62 is shown positioned within the mitral valve annulus, between the native mitral valve leaflets 66. The capsule 62 may be in another position for deployment in other embodiments.

FIG. 6 illustrates the valve 10 being deployed, with the capsule 62 being retracted relative to the prosthetic valve 10. The anchors 17 are shown to extend radially outward from the capsule 62 and extend around the distal tips of the leaflets 66 of the native mitral heart valve 64. The anchors 17 extend around the leaflets 66 to be positioned radially outward of an outward facing surface 68 of the leaflets 66 of the native mitral heart valve 64.

In the configuration shown in FIG. 6, the anchors 45 of the outer frame 20 may be restrained from deployment by the capsule 62. As such, the anchors 45 may not yet be in contact with the inward facing surface 70 of the leaflets 66 of native mitral heart valve 64. The anchors 45 accordingly may be deployed sequentially from the anchors 17, to allow for sequential controlled deployment of the anchors 45. The anchors 17 may be first deployed and then the anchors 45 may be deployed. Such a feature may allow the anchors 17, 45 to more easily retain the native heart valve leaflet 66 between the anchors 17, 45. The outer frame 20, for example, may be restrained from deployment by the capsule 62, which may restrain expansion of the anchors 45 coupled to the outer frame 20. In embodiments, however, the anchors 17, 45 may be deployed simultaneously. In embodiments, the anchors 45 may first be deployed and then the anchors 17 may be deployed.

FIG. 7 illustrates the valve 10 continuing to be deployed, with the capsule 62 continuing to retract relative to the valve 10. The anchors 45 coupled to the outer frame 20 are released from the capsule 62 and extend radially outward toward the leaflets 66 of the native mitral heart valve 64. The anchors 45 may abut the inward facing surface 70 of the leaflets 66.

FIG. 8 illustrates the valve 10 deployed and with additional features of the valve 10 visible, including a pattern of the outer frame 20. The anchors 17 are positioned radially outward of the outward facing surface 68 of the leaflets 66. The anchors 45 abut against the inward facing surface 70 of the leaflet 66 of the native mitral heart valve 64. The anchors 17, 45 are utilized to anchor the prosthetic valve 10 to the native mitral heart valve 64. The anchors 17, 45 retain the leaflet 66 between the anchors 17, 45, to provide for greater securement of the valve 10 to the leaflets 66 and to reduce the possibility of the leaflet 66 moving relative to the valve 10. The anchors 17, 45 may apply a force towards each other and may pinch the leaflet 66 between the anchors 17, 45.

Additional anchors may be utilized in embodiments. For example, as shown in FIGS. 1 and 8, atrial or proximal anchors 72 may be utilized for enhanced securement of the valve 10 within the native heart valve 64.

Variations in the prosthetic valve 10 and the configuration of the anchors 17, 45 may be utilized in embodiments. For example, the position of the anchors 17, 45 and coupling location of the anchors 17, 45 to the prosthetic valve 10 may be varied in embodiments. The anchors 45, 17 are shown being deployed to a native mitral valve, however other deployment locations such as native tricuspid valves, or other deployment locations may be utilized as desired. Other deployment methods for the prosthetic valve 10 may be utilized in embodiments.

The features of the anchors 45, 17 and the inner valve frame 18 and the sealing body 11, including the outer frame 20 may be utilized in other embodiments disclosed herein. The anchors 45, 17, may be utilized solely, or may be utilized to anchor other embodiments of prosthetic valves disclosed herein to a deployment location.

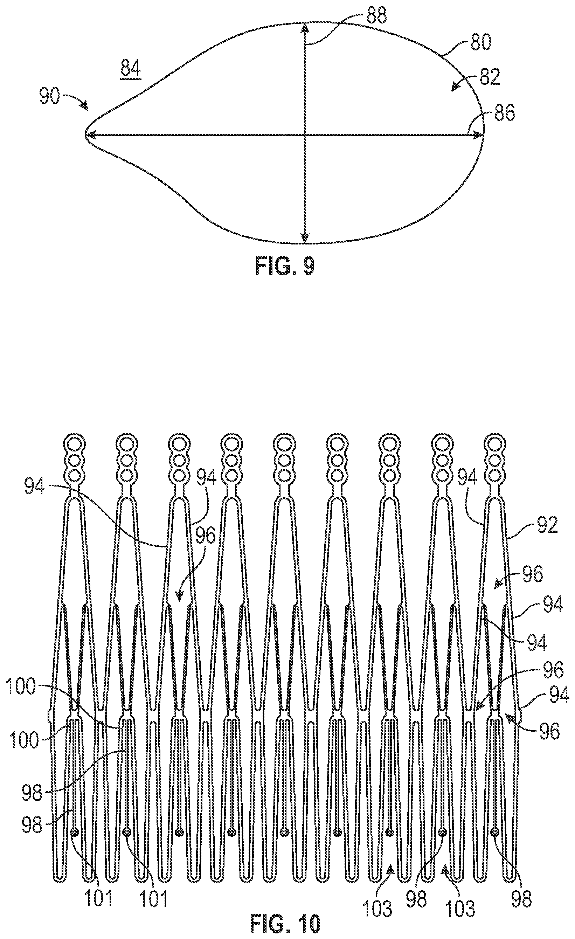

FIG. 9 illustrates a top schematic view of a valve annulus, which may comprise a mitral valve annulus 80. The valve annulus, however, may comprise various other forms of valve annuli, including a tricuspid valve annulus, an aortic valve annulus, or a pulmonary valve annulus, among other forms of valve annuli. As shown in FIG. 9, a flow channel 82 that may allow fluid flow may be surrounded by the valve annulus 80. A heart wall 84 may be positioned exterior of the annulus 80.

As shown, the annulus 80 may have a non-circular shape. The non-circular shape may be an oval shape. The annulus 80 may have a major axis 86 and a smaller minor axis 88. The oval shape may be symmetrical along only the major axis 86 thus forming an ovoid shape or may have no axis of symmetry. For example, the annulus 80 may have a varied shape that is irregular along the perimeter of the annulus 80. The annulus 80 may include one or more recesses 90 as shown in FIG. 9 that may be positioned at commissure points of leaflets of the native valve, or may correspond to areas of calcification, among other features.

A circular prosthetic heart valve implant may have difficulty deploying to the non-circular shape of the annulus 80 and conforming to the shape of the annulus 80. Further, the presence of recesses 90 may enhance the difficulty of a circular prosthetic heart valve implant to seal the annulus 80 at the recess 90.

Embodiments as disclosed herein may be configured to address an annulus 80 that may be non-circular or may include one or more recesses 90, or a combination of both features.

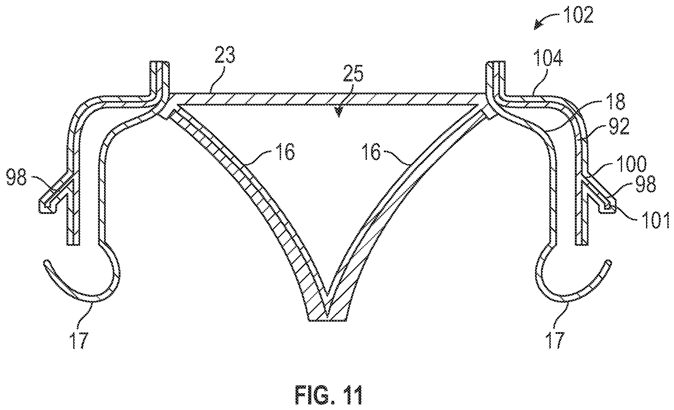

FIG. 10, for example, illustrates a plan view of a pattern of a frame 92 that may be utilized according to embodiments herein. The frame 92 may comprise a frame of a sealing body that may be utilized to reduce fluid flow. The frame 92 may comprise an outer frame of a valve that extends around an inner frame 18 of the valve, as marked in the cross sectional view of FIG. 11.

The frame 92 may include a plurality of struts 94 separated from each other by openings 96. The configuration of struts 94 and openings 96 may allow the frame 92 to form a non-circular shape. The non-circular shape may comprise an oval shape, which may be an elliptical shape or may comprise an ovoid shape such as a "D" shape. The configuration of struts 94 and openings 96 may allow the frame 92 to deflect from a circular shape to an oval shape to conform to an oval shape of an annulus. As such, as the valve is deployed, the frame 92 may move to an oval shape that conforms to the shape of the annulus.

The frame 92, for example, may include a closed row of cells. The closed row of cells may be configured to allow the frame 92 to vary in shape from a circular shape to a non-circular shape.

One or more sealing prongs 98 may be coupled to the frame 92. The sealing prongs 98 may comprise elongate bodies that may extend from a proximal portion 100 of the respective sealing prong 98 to a distal tip 101 of the respective sealing prong 98. The sealing prongs 98 may extend to their respective distal tips 101 from the closed row of cells of the frame 92.

The sealing prongs 98 are shown positioned within spaces 103 of the outer frame 92 between struts 94. As such, the distal tips 101 of the sealing prongs 98 may be shorter than the distal end of the frame 92 with the frame 92 in a flattened configuration. The proximal portions 100 of the respective sealing prongs 98 may couple to a juncture of the struts 94 at a mid-portion of the frame 92 between a distal end and a proximal end of the frame 92.

The sealing prongs 98 may be configured to extend radially outward from the spaces 103. The sealing prongs 98 may comprise protrusions that extend outward from the frame 92 to the distal tip 101 of the respective sealing prong 98.

FIG. 11, for example, illustrates a cross sectional view of a prosthetic valve 102 including the frame 92 shown in FIG. 10. The prosthetic valve 102 is configured to be deployed to a native valve. The frame 92 forms an outer frame that is positioned radially outward of and extends circumferentially around an inner valve frame 18 that supports a plurality of prosthetic valve leaflets 16. The inner valve frame 18 may have distal anchors 17 coupled thereto that may be configured to anchor to native valve leaflets in a similar manner as the anchors 17 shown in FIG. 3.

The frame 92 is coupled to the plurality of prosthetic valve leaflets 16. The frame 92 is shown with the sealing prongs 98 protruding radially outward from the frame 92. The sealing prongs 98 may be angled in a distal direction from the proximal portion 100 to the distal tip 101 of the respective sealing prong 98. Each sealing prong 98 is configured to reduce fluid flow at a portion of a native valve. In embodiments, a sealing skirt 104 may be coupled to the sealing prongs 98. The sealing skirt 104 may extend over the sealing prongs 98 and circumferentially about the prosthetic valve 10 between circumferentially adjacent sealing prongs 98. As such, the sealing skirt 104 may reduce fluid flow at the position of the sealing prongs 98.

The sealing prongs 98 may each be configured to be flexible. The sealing prongs 98 may be configured to deflect radially inward relative to the frame 92. Further, the sealing prongs 98 in embodiments may be configured to deflect circumferentially. The deflection of the sealing prongs 98 may be to accommodate a surface of the native valve upon deployment of the prosthetic valve 102. The prongs 98 for example, may be made of a shape memory material that may be shape set with a bias radially outward from the frame 92. The bias may be overcome with contact with the native valve annulus. The shape memory material may comprise nitinol or another shape memory material in embodiments.

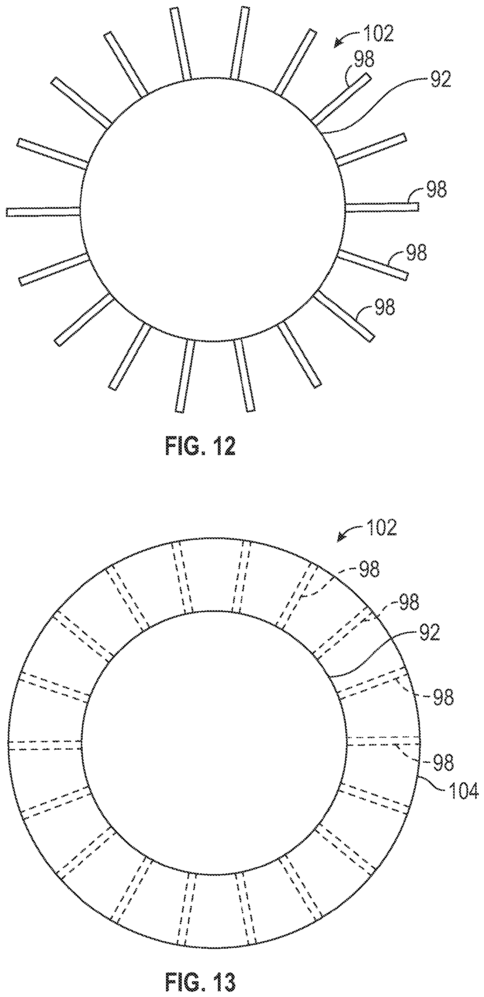

FIG. 12 illustrates a top view representing the protrusion of the sealing prongs 98 relative to the outer frame 92. The sealing skirt 104 is excluded from view in FIG. 12. The sealing prongs 98 are circumferentially spaced from each other and may be equally spaced or may have varied spacing as desired. The distance that each sealing prong 98 protrudes from the outer frame 92 may be equal as shown in FIG. 12, or may be varied as desired. The number of sealing prongs 98 may further be varied as desired. For example, a greater variation in the shape of the valve annulus may result in a greater number of sealing prongs 98 being utilized. A greater number of sealing prongs 98 may produce greater conformability of the prosthetic valve to the shape of the native valve annulus.

FIG. 13 illustrates the top view of FIG. 12 with the sealing skirt 104 positioned over the sealing prongs 98. The sealing skirt 104 may form the outermost layer of the valve 102 and may extend between circumferentially adjacent sealing prongs 98. In embodiments, the configuration of the sealing skirt 104 may be varied as desired. For example, the sealing skirt 104 may be positioned interior of the sealing prongs 98 in embodiments.

Upon deployment, the sealing prongs 98 may deflect to accommodate a surface of the native valve, which may be the native valve annulus. The deflection may be radially inward or may be circumferential in response to the shape of the native valve annulus. The deflection may conform the sealing prongs 98 to a shape of a portion of the native valve annulus. Further, the outer frame 92 may deflect to conform to a shape of the native valve annulus, which may be a non-circular shape, including an oval shape (which may comprise an elliptical shape or an ovoid shape including a "D" shape).

The valve 102 may be delivered to the native valve in a similar manner as the prosthetic valve 10 shown in FIG. 4. For example, a delivery apparatus may be utilized to deploy the valve 102 to the native valve.

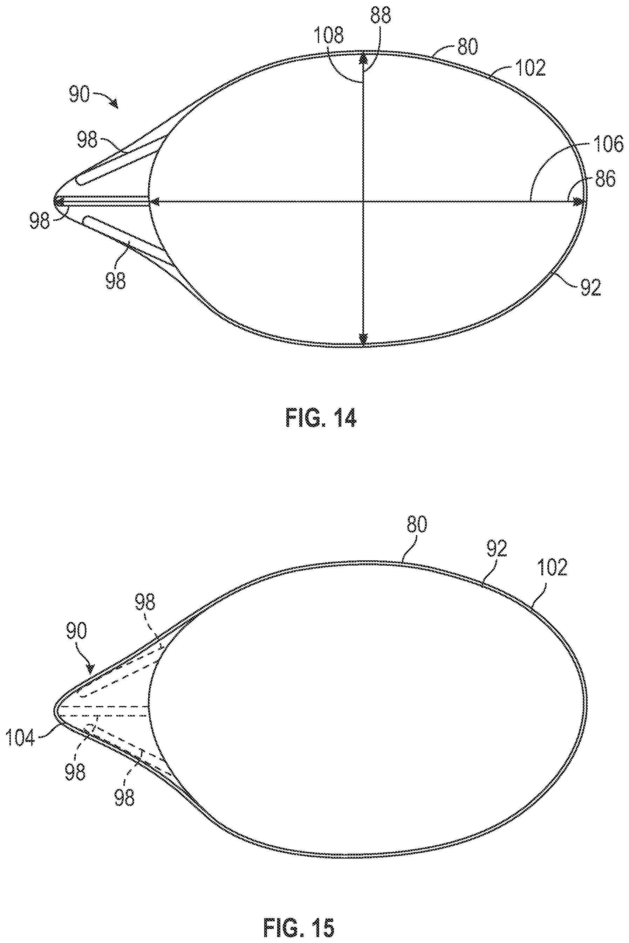

FIG. 14 illustrates a top schematic view of the valve 102 positioned within the native valve annulus 80 shown in FIG. 9. The sealing skirt 104 shown in FIG. 13 is excluded from view. The outer frame 92 may be flexible and configured to move from a circular configuration as shown in FIG. 12 to an oval configuration shown in FIG. 14. The outer frame 92 may move to conform to the shape of a portion of the native valve annulus 80. A major axis 106 of the outer frame 92 may align with the major axis 86 of the native valve annulus 80 and a minor axis 108 of the outer frame 92 may align with the minor axis 88 of the native valve annulus 80. The outer frame 92 may have an elliptical shape as shown in FIG. 14, or may have an ovoid shape, or other shape, according to embodiments.

The inner valve frame 18 marked in FIG. 11, notably, may retain a circular shape as the outer frame 92 moves to the oval shape. Thus, the outer frame 92 may have a shape that varies independent of the shape of the inner valve frame 18. In embodiments, the shape of the inner valve frame 18 may vary with the outer frame 92.

The sealing prongs 98 may be utilized to reduce fluid flow at a portion of the native valve. Such a portion may comprise a recess 90 as shown in FIG. 14 or may comprise another portion as desired. As shown in FIG. 14, sealing prongs 98 may deflect radially inward relative to the outer frame 92 to accommodate the native valve. Thus, one or more, or a majority, of the sealing prongs 98 may deflect radially inward. Sealing prongs 98 may be positioned within the spaces 103 of the outer frame 92 marked in FIG. 10 and thus a majority of the sealing prongs 98 shown in FIG. 12 are not visible in FIG. 14 due to the radial deflection inward. The sealing prongs 98 may continue to be biased radially outward, however, to apply a seal to the native valve between the outer surface of the outer frame 92 and the inner surface of the native valve.

Sealing prongs 98 may protrude into recesses 90 of the native valve. FIG. 14, for example, illustrates three sealing prongs 98 that have not been deflected into the outer frame 92, and continue to protrude radially outward from the outer frame 92. A central sealing prong 98 extends along the major axis 86 of the native valve and adjacent sealing prongs 98 are deflected circumferentially towards the recess 90 and the central sealing prong 98. As such, the sealing prongs 98 deflect to accommodate and enter the recess 90 of the native valve. The sealing prongs 98 reduce fluid flow at the recess 90.

FIG. 15 illustrates the view shown in FIG. 14, with the sealing skirt 104 shown in view. The sealing prongs 98 may prop the sealing skirt 104 into the recess 90 to thus reduce fluid flow at the recess 90.

The configuration of the sealing prongs 98 may provide a variety of benefits, including reducing fluid flow at positions of the native valve that the outer frame is not positioned in (such as a recess shown in FIG. 15). The sealing prongs 98 may be flexible to conform to the configuration of the native valve at the position of the sealing prongs 98. Such a feature may allow the overall diameter of the outer frame to be reduced, to be a lesser diameter than the major axis 86 of the native valve, yet still seal at a major axis 86 of the native valve. Further, sealing may be provided for irregular shapes of valve annuli, including a variety of recesses of different sizes positioned along the perimeter of the valve annulus.

The embodiment of prosthetic valve 102 may be utilized in a mitral valve as disclosed herein, or may be utilized in other deployment locations such as a native tricuspid valve, or other deployment locations. For example, the sealing prongs 98 and/or outer frame 92 may be applied to a valve configured to deploy to a native aortic valve or pulmonary valve, to enhance sealing at the aortic valve or pulmonary valve.

The configuration of the sealing prongs 98 and the outer frame 92 may be utilized solely, or may be utilized in combination with other embodiments disclosed herein. For example, the sealing prongs 98 and outer frame 92 may be utilized with an embodiment shown in FIG. 1, including use of the anchors 17, 45, or any other embodiment of prosthetic valve disclosed herein.

FIG. 16 illustrates a cross sectional schematic view of a prosthetic valve 110 configured to be deployed to a native valve. The prosthetic valve 110 may include one or more distal anchors 112 that are each configured to extend over a distal tip of a native valve leaflet and horizontally from the distal tip of the leaflet to a tip 114 of the respective distal anchor 112. The prosthetic valve 110 may extend around a central axis 116, and the distal anchors 112 may extend perpendicular with respect to the central axis 116.

The distal anchors 112 may be coupled to a valve frame 118 that may support prosthetic valve leaflets 16 as shown in FIG. 3 for example. The valve frame 118 in embodiments may curve inward from the proximal end 120 of the valve frame 118 to the distal end 122 of the valve frame 118. The distal anchors 112 may be coupled to the distal portion of the valve frame 118 including the distal end 122. The distal anchors 112 may be coupled to the prosthetic valve leaflets 16.

A sealing body 124 may extend circumferentially around the valve frame 118 and may serve to reduce fluid flow at the position of the sealing body 124. The sealing body 124 may include a frame 126 that may comprise an outer frame 126 extending circumferentially around the inner valve frame 118 and positioned radially outward of the valve frame 118 and may include a sealing skirt 128.

FIG. 17, for example, illustrates a side perspective view of the prosthetic valve 110 illustrating the sealing body 124 and the distal anchors 112 protruding radially outward from the sealing body 124. The outer frame 126 of the sealing body 124 may include a plurality of struts 123 separated by spaces 125.

FIG. 18 illustrates a bottom perspective view of the prosthetic valve 110 showing the distal anchors 112 positioned radially outward from the valve frame 118. The anchors 112 may be co-planar with each other, in a plane that extends perpendicular to the central axis 116 marked in FIG. 16. The anchors 112 may be circumferentially spaced from each other with an equal spacing or at another spacing as desired.

The anchors 112 may extend horizontally relative to the distal tips of the native valve leaflets such that anchoring to the native valve leaflets does not require vertical extension of the anchors 112 to anchor to the native valve leaflet. Such vertical extension is shown for example in FIG. 8 with the anchors 17. Thus, the possibility of missed capture of a native leaflet may be reduced, as the anchors 112 do not vertically hook the native leaflets.

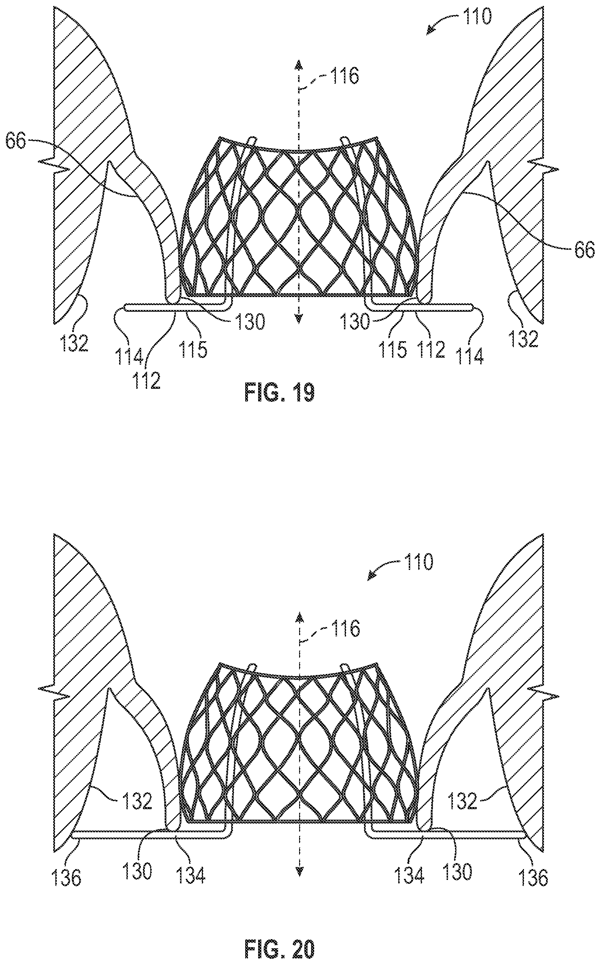

FIG. 19, for example, illustrates the distal anchors 112 extending horizontally from the distal tips 130 of the native valve leaflets 66. The distal anchors 112 may extend at an angle of ninety degrees with respective to the distal tips 130 or another amount as desired. Arm portions 115 of the distal anchors 112 contact the distal tips 130 and extend from the valve frame 118 to the respective tip of the anchors 112. The arm portions 115 may have a linear shape or may have a curved configuration as shown in FIG. 16 for example. The contact between the anchor 112 and the distal tip 130 accordingly anchors the prosthetic valve 110 to the native valve and resists a force to the valve in a proximal or atrial direction.

The prosthetic valve 110 may be delivered to the native valve in a similar manner as the prosthetic valve 10 shown in FIG. 4. For example, a delivery apparatus may be utilized to deploy the valve 110 to the native valve.

The distal tips 114 of the distal anchors 112 as shown in FIG. 19 may be configured to be positioned between the native valve leaflets 66 and a heart wall comprising the ventricular wall 132. Such a configuration may reduce possible adverse effects that may be produced by contacting a heart wall.

The distal anchors 112 may be further configured to pass through chordae coupled to the native valve leaflets 66, to reduce the possibility of trauma to the chordae and to further anchor the prosthetic valve 110 to the native valve. The prosthetic valve may be chordal sparing in embodiments.

In embodiments, the distal tips 114 may be configured to contact the ventricular wall 132. FIG. 20, for example, illustrates an embodiment including distal anchors 134 extending horizontally from the distal tip 130 of the native valve leaflet 66. The distal tips 136 of the anchors 134 contact the ventricular wall 132 of the heart to further anchor the prosthetic valve 110 to the native valve.

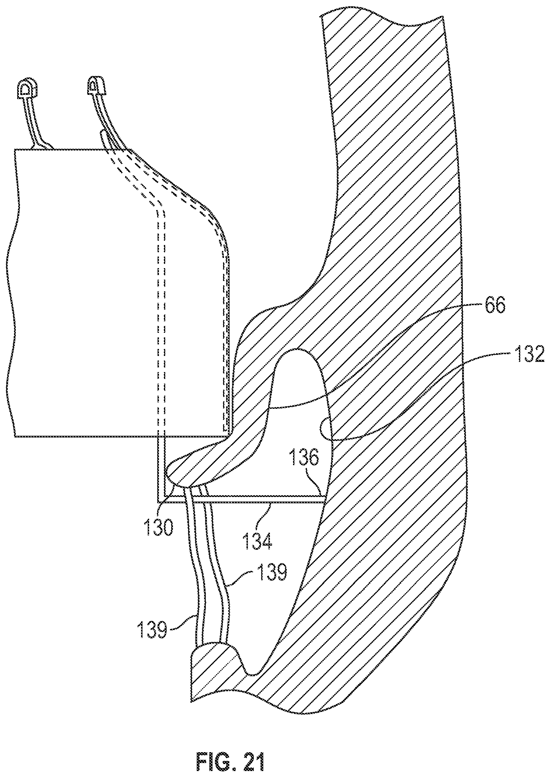

FIG. 21, for example, illustrates a close up view of such a configuration in which the distal anchor 134 is contacted to the ventricular wall 132. The distal anchor 134 extends horizontally from the distal tip 130 of the native valve leaflet 66. The distal anchor 134 passes between the chordae 139 coupled to the native valve leaflet 66.

The distal tips 136 of the anchors 134 may be configured to be atraumatic to the surfaces of the ventricular wall 132 in embodiments. For example, the distal tips 136 may have smooth contact surfaces that may be flattened or curved and are not configured to penetrate the ventricular wall 132. The distal tips 136 may be covered in embodiments. In embodiments, the distal tips 136 may be configured to be flexible to allow for reduction of possible trauma to the ventricular wall 132 upon contact.

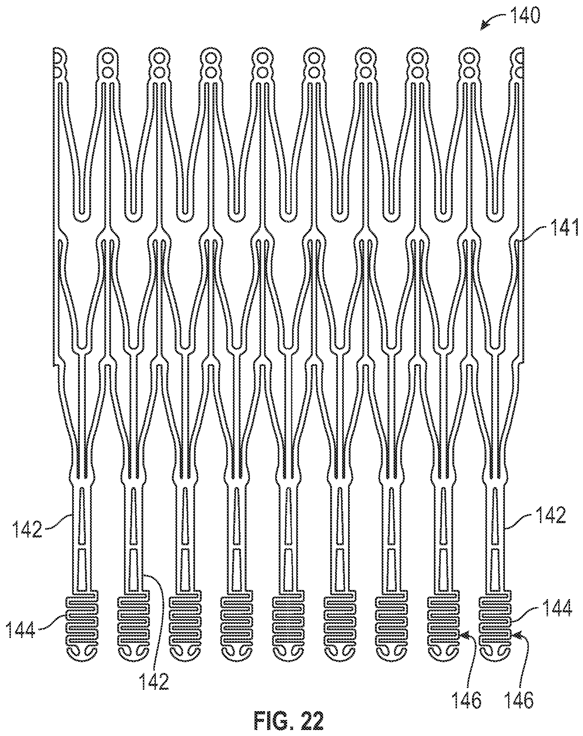

FIG. 22, for example, illustrates a pattern of a frame 140 having distal anchors 142 configured similarly as the anchors 134 shown in FIG. 21 and configured to contact a ventricular heart wall for anchoring. The frame 140 may be a flattened pattern of an inner frame, similar to the inner frame 118 marked in FIG. 16. The upper portion 141 of the frame 140 may support the prosthetic valve leaflets 16. The distal anchors 142 may deflect radially outward from the upper portion 141 of the frame 140 from the flattened configuration shown in FIG. 22 and may extend perpendicular to the frame 140 and central axis of the valve. The distal tips 144 of the distal anchors 142 may be configured to be flexible, and able to flex upon contact with the ventricular heart wall. A distal tip 144 of the anchors 142 may have a smooth curved contact surface for atraumatic contact with the ventricular wall. The distal tips 144 may include an undulating feature 146 or "rachis" feature that allows for flex of the distal tips 144. The amount of flex may be controlled by the thickness of material forming the undulating feature 146 and the spacings between adjacent undulations.

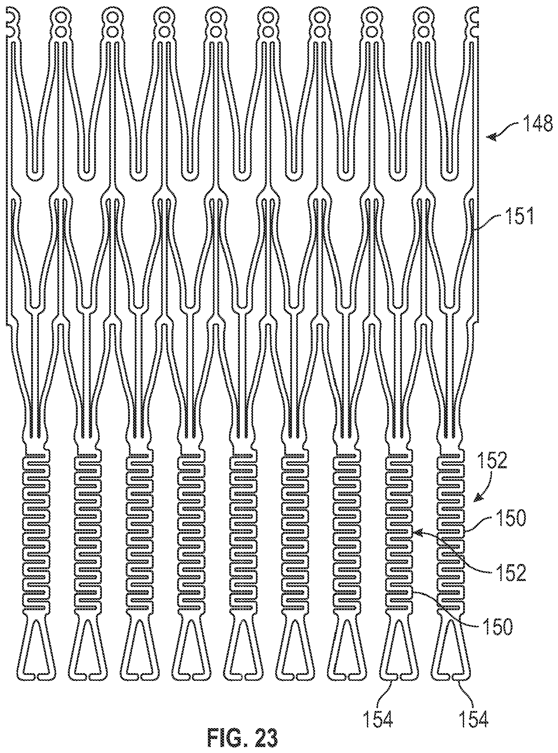

FIG. 23, for example, illustrates a pattern of a frame 148 in which the majority of the length of the distal anchors 150 comprises an undulating feature 152. The frame 148 may be a pattern of an inner frame, similar to the inner frame 118 marked in FIG. 16. The upper portion 151 of the frame 148 may support the prosthetic valve leaflets 16. The distal anchors 150 may deflect radially outward from the upper portion 151 of the frame 148 from the flattened configuration shown in FIG. 23 and may extend perpendicular to the frame 148 and central axis of the valve.

The flexibility of the anchors 150 shown in FIG. 23 may be greater than the flexibility of the anchors 142 shown in FIG. 22. A distal tip 154 of the anchors 150 may have a smooth flattened contact surface for atraumatic contact with the ventricular wall.

The flexible anchors may further allow for each anchor to have a similar length, yet have anchors with a shorter distance to the ventricular wall flex upon contact with the ventricular wall. Anchors having a longer distance to the ventricular wall may remain unflexed. In embodiments, the lengths of the anchors may be varied to produce a desired fit within a portion of the heart.

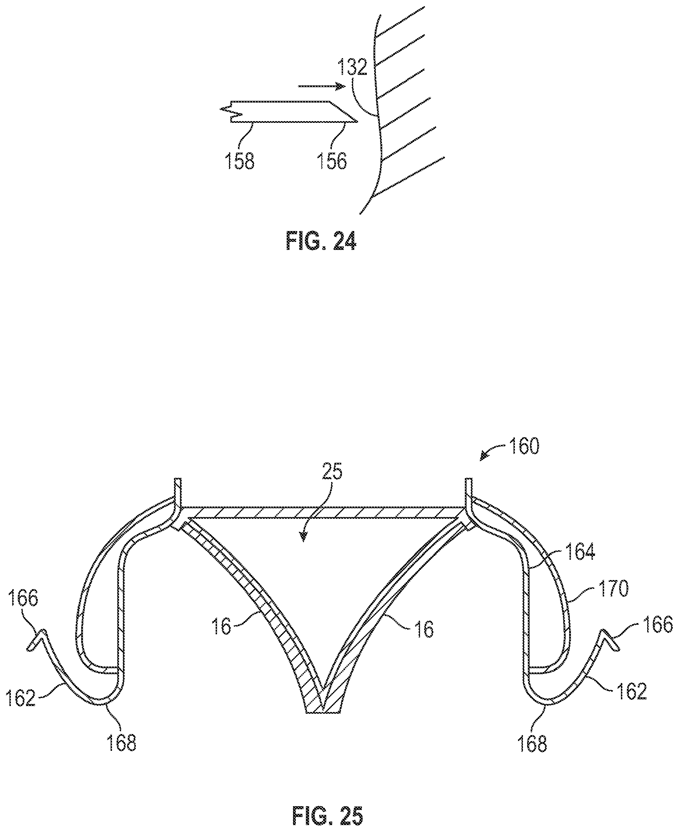

In embodiments, the anchors may be configured to puncture a portion of a ventricular wall. FIG. 24 for example illustrates a tip 156 of an anchor 158 may comprise a puncturing tip 156 configured to enter the ventricular wall 132 for anchoring.

Variations in the configurations of the anchors and the tips of the anchors may be utilized as desired.

The embodiments of prosthetic valves may be utilized in a mitral valve as disclosed herein or may be utilized in other deployment locations such as a native tricuspid valve, or other deployment locations.

The configuration of the anchors may be utilized solely or may be utilized in combination with other embodiments disclosed herein. For example, the anchors may be utilized with an embodiment as shown in FIG. 11, for example, in lieu of the anchors 17. The anchors may be utilized with the other embodiments of prosthetic valves disclosed herein.

FIG. 25 illustrates a cross sectional schematic view of a prosthetic valve 160 configured to be deployed to a native valve. The prosthetic valve 160 may include one or more distal anchors 162 configured to extend over a distal tip of a leaflet of a native valve and couple to a ventricular heart wall. The distal anchors 162 may extend radially outward from a valve frame 164 that may be configured similarly as the valve frame 18 shown in FIG. 3 and may support prosthetic valve leaflets 16. The distal anchors 162 may be coupled to the prosthetic valve leaflets 16.

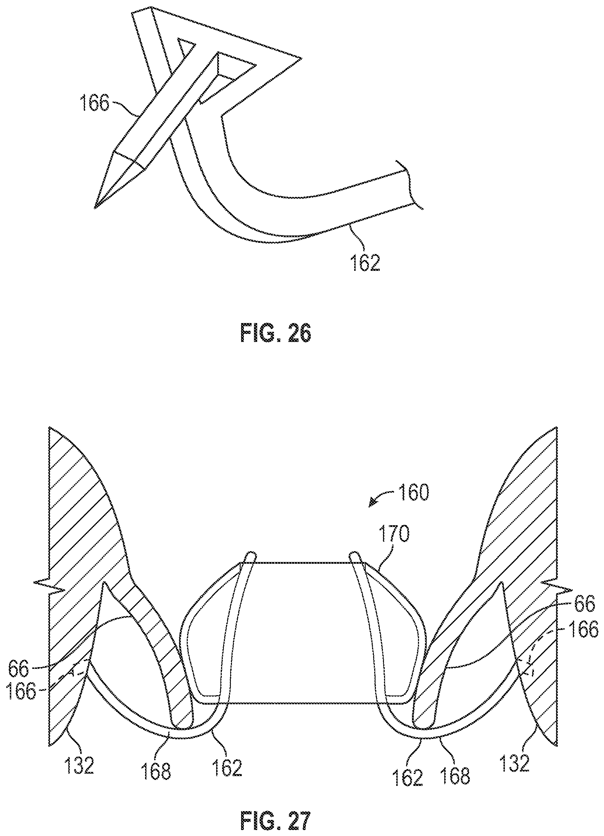

The distal anchors 162 may each include a tip 166 that may comprise a puncturing tip 166 for puncturing the ventricular heart wall. The tip 166 may be configured to resist a force applied to the prosthetic valve 160 in a ventricular or distal direction. The tip 166, for example, may include an angled puncturing tip 166 as marked in FIG. 26 that may be angled in a distal direction. As such, a force applied to the prosthetic valve 160 in the ventricular direction may be resisted by the angle of the puncturing tip 166 in the distal direction. The tip 166 for example, may comprise a protrusion with a sharpened edge for penetrating the ventricular wall.

Referring again to FIG. 25, each distal anchor 162 may be configured to contact and anchor to a distal tip of a leaflet of the native valve. For example, an arm portion 168 of the anchor 162 may be positioned distal of the distal tip of the native valve leaflet and may contact the distal tip of the native valve leaflet. Such anchoring may resist a force applied to the prosthetic valve 160 in the atrial or proximal direction.

FIG. 27, for example, illustrates the prosthetic valve 160 deployed to a native valve. The tips 166 pass into the ventricular wall 132 to resist a force applied to the prosthetic valve 160 in the ventricular or distal direction. The arm portions 168 extend over the tips of the leaflets 66 to resist a force applied to the prosthetic valve 160 in the atrial or proximal direction. The anchoring for the prosthetic valve 160 accordingly may be provided entirely by the distal anchors 162 in embodiments.

The distal anchors 162 may each have different lengths or may have the same length in embodiments. Further, the distal anchors 162 may be flexible to allow for deflection due to varied spacing towards the ventricular wall.

Referring again to FIG. 25, a sealing body 170 may be positioned radially outward from the frame 164 and the prosthetic valve leaflets 16 and may extend circumferentially around the frame 164. The sealing body 170 may comprise a frameless sealing body that may contact a portion of the heart to reduce fluid flow at that position. The sealing body 170 may seal against a portion of the native valve. The sealing body 170 for example may comprise only a sealing skirt or another frameless body that may comprise the sealing body 170. The frame of the sealing body 170 may be excluded because the anchoring feature may be provided by the anchors 162.

Thus, as shown in FIG. 27, a frameless sealing body 170 may be configured to seal to the heart valve. The frameless sealing body 170 may comprise a billowing skirt that is configured to inflate to fill the skirt against the heart valve and form a seal with the heart valve annulus. In embodiments, one or more supports may hold the billowing skirt against the native heart valve.

The prosthetic valve 160 may be delivered to the native valve in a similar manner as the prosthetic valve 10 shown in FIG. 4. For example, a delivery apparatus may be utilized to deploy the valve 160 to the native valve. The distal anchors 162 may extend over the distal tip of the leaflets of the native valve. The distal anchors 162 may be coupled to the ventricular heart wall.

The frameless sealing body 170 may be configured to conform to the shape of the native valve annulus, which may comprise a non-circular shape, including an oval shape (which may comprise an elliptical shape or an ovoid shape including a "D" shape). The frameless sealing body 170 may abut against a portion of the native valve. The lack of a frame may allow the frameless sealing body 170 to more readily conform to the shape of the native valve annulus, which may include recesses as shown in FIG. 9 for example.

Variations in the configurations of the sealing body, the anchors, and the tips of the anchors may be utilized as desired.

The configuration of the anchors or the sealing body may be utilized solely, or may be utilized in combination with other embodiments disclosed herein. The anchors may be utilized in any other embodiment of prosthetic valve disclosed herein. For example, the puncturing tips 166 may be utilized with other embodiments disclosed herein, including the embodiment shown in FIG. 24. The puncturing tips 166 may further be utilized in combination with the anchors 17, 45 shown in FIG. 1, for example. Either of the anchors may include the prong of the tip 166, which may be utilized to enhance securement of the anchors 17, 45 to the native leaflet or other body.

The embodiments of prosthetic valves may be utilized in a mitral valve as disclosed herein or may be utilized in other deployment locations such as a native tricuspid valve, or other deployment locations.

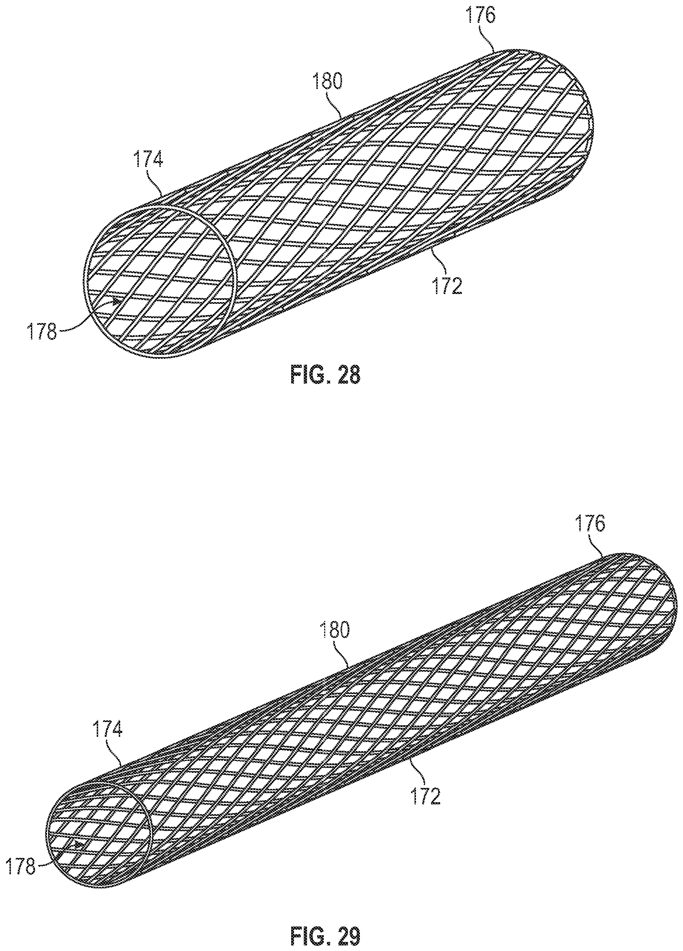

FIG. 28 illustrates an embodiment of a braid material 172 that may be utilized in embodiments herein. The braid material 172 may be configured as a braided tube as shown in FIG. 28. The braided tube may have a first end 174 and a second end 176 and a length therebetween. In embodiments, the braided tube may have a central channel 178 that is surrounded by a wall 180 of the braided tube.

The braid material 172 may be configured to be flexible and may have a shape that varies. The shape may vary in response to a local shape applied to the braid material 172. Thus, if the braid material 172 is pressed against a rounded surface, the braid material 172 may deform to accommodate the rounded surface.

In embodiments, the braid material 172 in the form of a tube may be configured to have dimensions that vary in response to a force applied to the braid material 172. For example, a length of the tube may increase in response to a radially compressive force applied to the tube. FIG. 29, for example, illustrates the length of the tube having increased with a corresponding decrease in the diameter of the tube, in response to a radially compressive force applied to the tube. Other shapes of the tube may result from a compressive force being applied to the tube in embodiments.

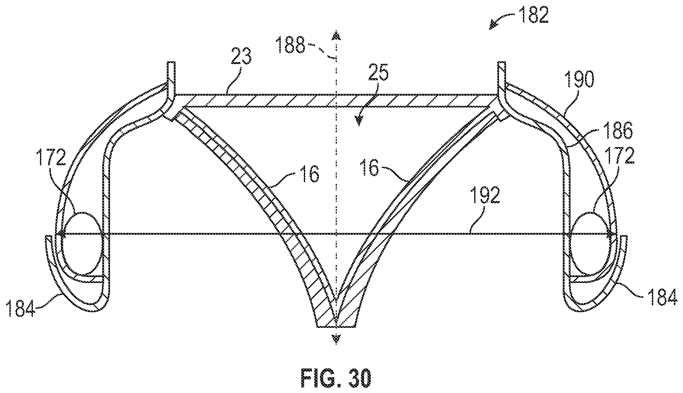

The braid material 172 may be utilized with prosthetic valve embodiments disclosed herein. FIG. 30, for example, illustrates an embodiment of a prosthetic valve 182 configured to be deployed to a native valve. The prosthetic valve 182 may include the braid material 172 having a shape that varies to vary an outer diameter 192 of the prosthetic valve 182.

The prosthetic valve 182, for example, may include distal anchors 184 coupled to a valve frame 186 that may support prosthetic valve leaflets 16 in a similar manner as discussed in regard to FIG. 3. The distal anchors 184 may be configured to anchor to leaflets of a native valve. The braid material 172 may be coupled to the prosthetic valve leaflets 16 and positioned to extend circumferentially relative to the prosthetic valve leaflets 16. One or more tubes of braid material 172 may extend circumferentially around the valve frame 186 and may curve around the central axis 188 of the prosthetic valve 182.

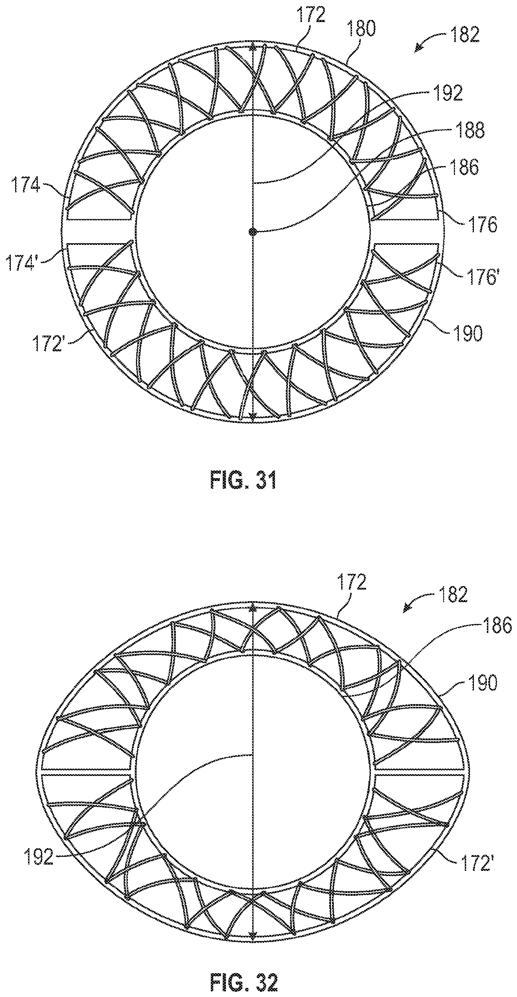

FIG. 31, for example, illustrates a top schematic view of the prosthetic valve 182 showing a tube of braid material 172 curving around the valve frame 186. Another tube of braid material 172' may also curve around the valve frame 186. The ends of the respective tubes may be adjacent each other, with an end 176 of a tube positioned adjacent an end 176' of the other tube and an end 174 of a tube positioned adjacent an end 174' of the other tube. One or more tubes of braid material 172, 172' may extend around the central axis 188 of the prosthetic valve 182.

In embodiments, a sealing body such as a sealing skirt 190 may be positioned around the braid material 172, 172' and may reduce fluid flow at the position of the sealing skirt 190. The braid material 172, 172' may be positioned between the valve frame 186 and the sealing body. The sealing skirt 190 may comprise the outer surface of the prosthetic valve 182 as shown in FIG. 30 for example.

The braid material 172, 172' may be utilized to vary the outer diameter 192 of the prosthetic valve 182. The braid material 172, 172' may have a shape that varies, and as such, the outer diameter 192 of the prosthetic valve 182 may vary with the braid material 172, 172'.

FIG. 32, for example, illustrates the shape of the braid material 172, 172' varying in response to being placed in an oval shape. The oval shape, for example, may be the shape of a native heart valve in embodiments. The braid material 172, 172' may conform to the shape of the native heart valve annulus to form an oval outer surface of the prosthetic valve 182. Notably, the shape of the valve frame 186 may remain circular, with the shape of the braid material 172, 172' and sealing skirt 190 varying. The braid material 172, 172' accordingly may be utilized to allow the sealing skirt 190 to conform to the shape of a native heart valve, which may be oval or another non-circular shape.

The sealing skirt 190 in embodiments, may be a frameless sealing skirt, with structural support provided by the braid material 172, 172'. The braid material 172, 172' accordingly may support the sealing skirt 190 against the native valve annulus to provide a seal at the annulus.

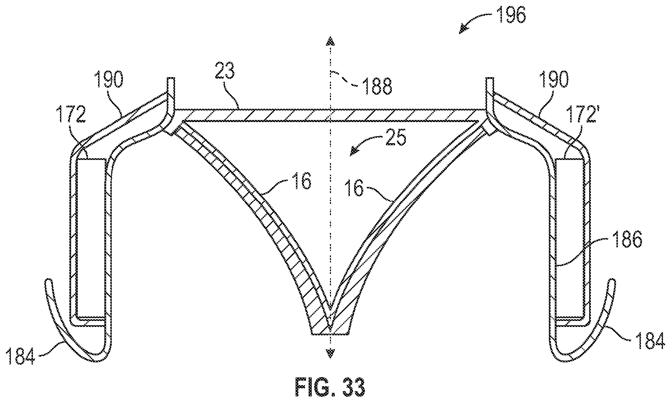

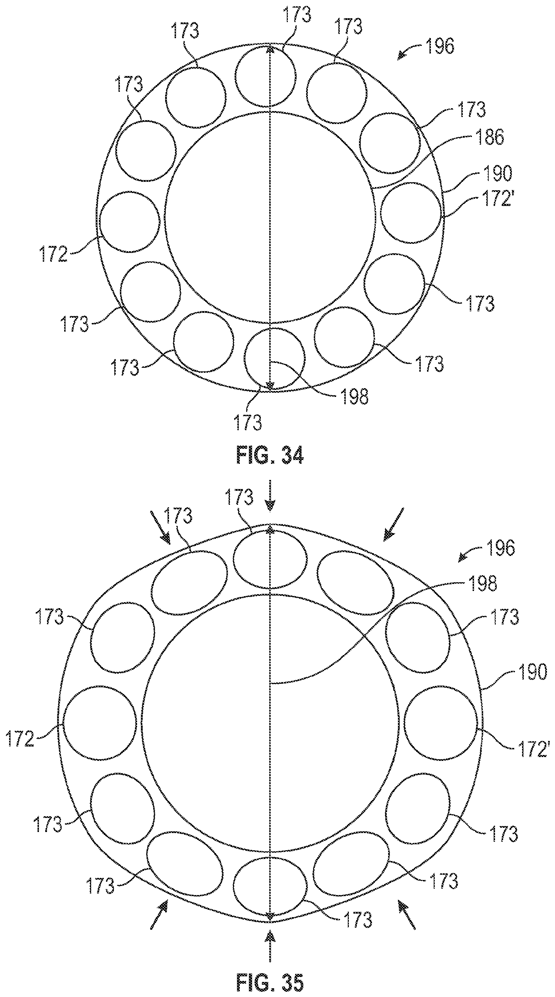

The braid material may be oriented axially with a prosthetic valve in embodiments. FIG. 33, for example, illustrates an embodiment in which the braid material 172, 172' in the form of braided tubes are oriented axially relative to the prosthetic valve leaflets 16. The braided tubes may be positioned at circumferentially spaced positions about the valve frame 186, with each tube extending axially. FIG. 34, for example, illustrates a top view of the prosthetic valve 196 in which the braided tubes are shown extending axially and are positioned circumferentially about the valve frame 186. The braided tubes may comprise the tubes 172, 172' and may comprise the additional tubes 173 that are configured similarly as the tubes 172, 172'. The tubes may be circumferentially spaced equally from each other as shown in FIG. 34, or other spacings may be utilized as desired.

The tubes 172, 172', 173 may each be configured to have a shape that varies independent from each other. For example, a variation in a shape of the tube 172 may not necessarily result in a variation in the shape of an adjacent tube 173. The variation in the shape of the prosthetic valve 196 accordingly may be localized to the variation in the shape of the tube proximate that location.

FIG. 35, for example, illustrates such a feature, with the tubes 173 being compressed to provide a localized variation in the shape of the prosthetic valve 196, and a variation in the outer diameter 198 of the prosthetic valve 196. The shape of the tubes 172, 172', however, may remain constant in response to the variation in the shape of the tubes 173. The tubes 172, 172', 173 may be utilized to allow the sealing skirt 190 to conform to the shape of a native heart valve, which may be a non-circular shape such as an oval shape.

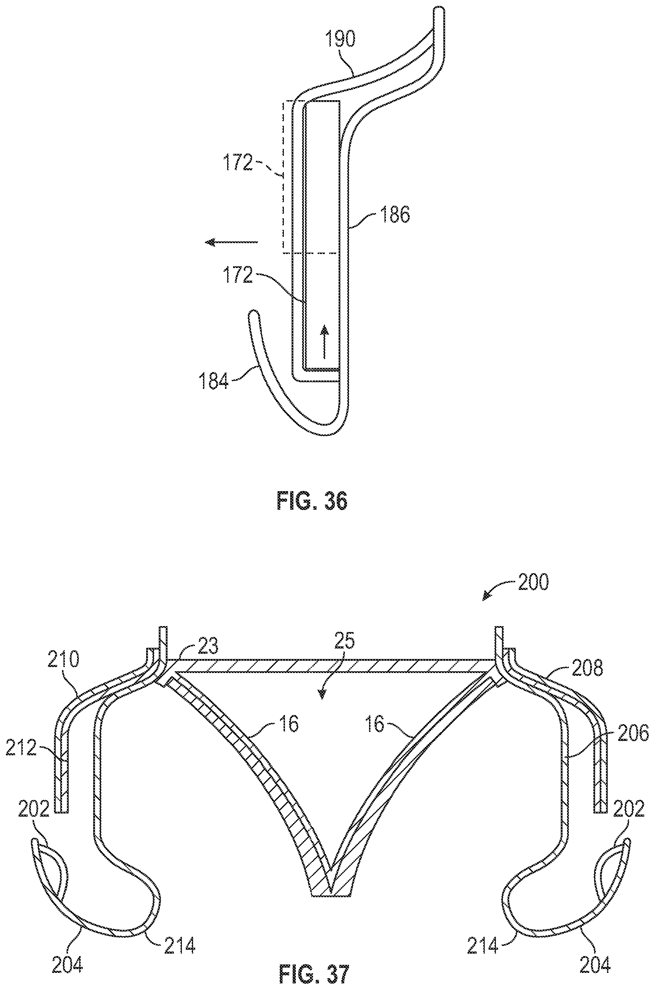

A length of the respective tubes may vary in response to a compressive force being applied to the tubes. A length of the tube 172, for example, may increase in response to a reduction in the diameter of the tube 172 due to a radially compressive force. The sealing skirt 190 may be configured to be flexible to allow for elongation and contraction of the length of the respective 172. As shown in FIG. 36, when the prosthetic valve 196 is in a compressed or linearized state, for example prior to deployment, the tubes may be compressed and may have a relatively greater length (as shown in solid lines) than when the prosthetic valve 196 is deployed. The tubes may expand radially outward and have a length that reduces upon deployment (as shown in dashed lines). The tubes may be configured to have a proximal end that is fixed in position relative to the valve frame 186, or another portion of the tubes may be fixed in embodiments.

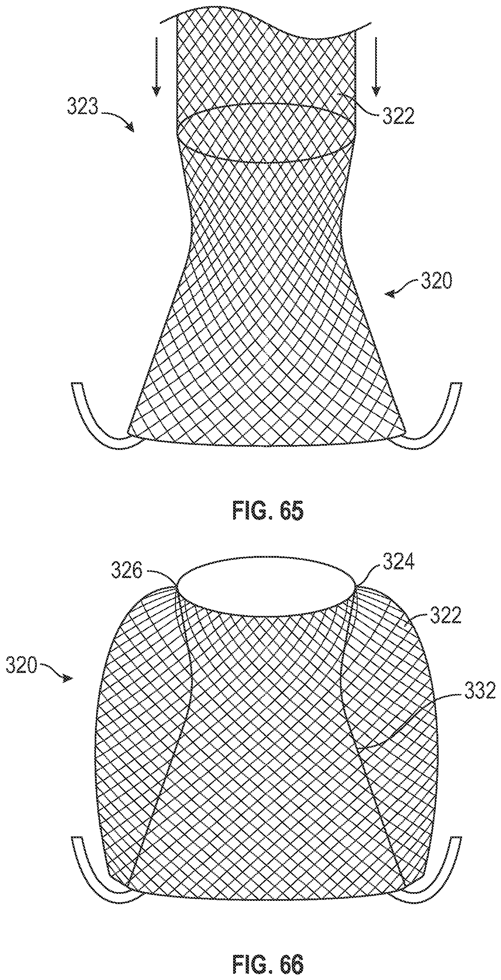

In embodiments, the braid material disclosed herein may be attached to another portion of the prosthetic valve, or may be floating within the sealing body as desired. FIGS. 65-68, for example, illustrate an embodiment in which the braid material forms an axially extending tube. The axially extending tube has an outer wall surrounding a central cavity, and the plurality of prosthetic valve leaflets and the inner frame is positioned within the central cavity.

The braid material may comprise a shape memory material, which may comprise nitinol or another form of shape memory material. The braid material may be biased to resist a variation in the shape of the braid material. Other materials, such as metals, or composite fibers, among others, may be utilized in embodiments.

The prosthetic valves may be delivered to the native valve in a similar manner as the prosthetic valve 10 shown in FIG. 4. For example, a delivery apparatus may be utilized to deploy the valve to the native valve. The distal anchors 184 may extend over the distal tip of the leaflets of the native valve.

The shape of the prosthetic valves may be configured to conform to the shape of the native valve annulus, which may comprise a non-circular shape, including an oval shape (which may comprise an elliptical shape or an ovoid shape including a "D" shape). The braid material may allow the prosthetic valves to more readily conform to the shape of the native valve annulus.

Further, embodiments of conformable prosthetic valves disclosed herein, including but not limited to the embodiments of FIGS. 9-15 and 25-36 may allow for reduced rigidity of the respective sealing bodies, allowing for an improved conformability to the shape of the native valve annulus. The force applied by the sealing bodies may further be reduced from an embodiment including a rigid outer frame. As such, reduced stress to the native valve annulus may be provided, which may reduce the possibility of electrical conduction issues within the patient's heart that may be caused by stress to the native valve annulus. The sealing prongs of the embodiment of FIGS. 9-15, as well as the outer frame of the embodiment of FIGS. 9-15, may provide a reduced outward force to the native valve annulus than may be provided with a more rigid frame. Further, a braid material, for example, may provide a reduced outward force to the native valve annulus than may be provided with a rigid frame. The braid material may have a reduced stiffness compared to a rigid frame.

Variations in the configurations of the braid material may be utilized as desired.

The configuration of the braid material may be utilized solely or may be utilized in combination with other embodiments disclosed herein. The braid material may be utilized with any embodiment of prosthetic valve disclosed herein. For example, the braid material may be utilized with the sealing bodies shown in FIGS. 11 and 25, among other embodiments.

The embodiments of prosthetic valves may be utilized in a mitral valve as disclosed herein or may be utilized in other deployment locations such as a native tricuspid valve, or other deployment locations. For example, the braid material, including the configuration of the tubes, may be applied to a valve configured to deploy to a native aortic valve or pulmonary valve, to enhance sealing at the aortic valve or pulmonary valve.

FIG. 37 illustrates a cross sectional schematic view of an embodiment of a prosthetic valve 200 configured to be deployed to a native valve. The prosthetic valve 200 may include one or more anchors 204 coupled to a plurality of prosthetic valve leaflets 16 and configured to couple to a portion of a heart. The prosthetic valve 200 may include one or more indicator mechanisms 202 configured to indicate the coupling of the anchor 204 to the portion of the heart. The portion of the heart may comprise a leaflet or other portion as desired.

The prosthetic valve 200 may include a valve frame 206 supporting prosthetic valve leaflets 16. The prosthetic valve 200 may include a sealing body 208 for reducing fluid flow, which may include a skirt 210 and an outer frame 212 as desired.

The anchors 204 may comprise distal anchors, configured similarly as the anchors 17 shown in FIG. 3, or may have another configuration. For example, the anchors 204 may be configured as any other embodiment of anchor disclosed herein or may have another configuration.

The indicator mechanisms 202 may be positioned on the anchors 204 in embodiments. The indicator mechanisms 202 for example, may be positioned at the distal ends of the anchors 204 or may have another position, such as at the arm portion 214 of the anchors 204 that may hook under the distal tips of valve leaflets. Other forms of anchors 204 such as proximal anchors or atrial anchors may be configured to utilize the indicator mechanisms 202 as well.

The indicator mechanisms 202 may be configured to be visualized to determine whether the anchors 204 have properly captured a desired portion of the heart, which may comprise the native valve leaflets. The indicator mechanisms 202 may be configured to move or otherwise have a varied appearance under visualization to indicate that capture has occurred. For example, the indicator mechanisms 202 may be configured to deflect upon capture to indicate that capture has occurred.

The indicator mechanisms 202 may comprise protrusions that may be depressed upon capture. The indicator mechanism 202 may be positioned on the anchors 204 such that the captured portion of the heart moves the indicator mechanism 202. Other locations for the indicator mechanisms 202, such as on the frame of the valve may be utilized if desired. The indicator mechanisms 202 may comprise springs configured to flex inward upon capture and configured to extend outward or remain extended outward upon a missed capture or lack of capture by the anchor 204.

The indicator mechanism 202 may be configured to indicate coupling while being visualized under ultrasound (including echocardiography) and/or fluoroscopy. Beneficially, the indicator mechanism 202 may be configured to be visualized utilizing fluoroscopy, which may otherwise exclude the clear appearance of any tissue, including native heart valve leaflets. As such, fluoroscopy may be more effectively utilized to visualize the capture of tissue due to the visualized movement of the indicator mechanism 202. In embodiments, ultrasound may be utilized to visualize the indicator mechanism as well as the tissue of the patient's body (such as a native heart valve leaflet).

The prosthetic valves may be delivered to the native valve in a similar manner as the prosthetic valve 10 shown in FIG. 4. For example, a delivery apparatus may be utilized to deploy the valve to the native valve. The distal anchors 204 may extend over the distal tip of the leaflets of the native valve.

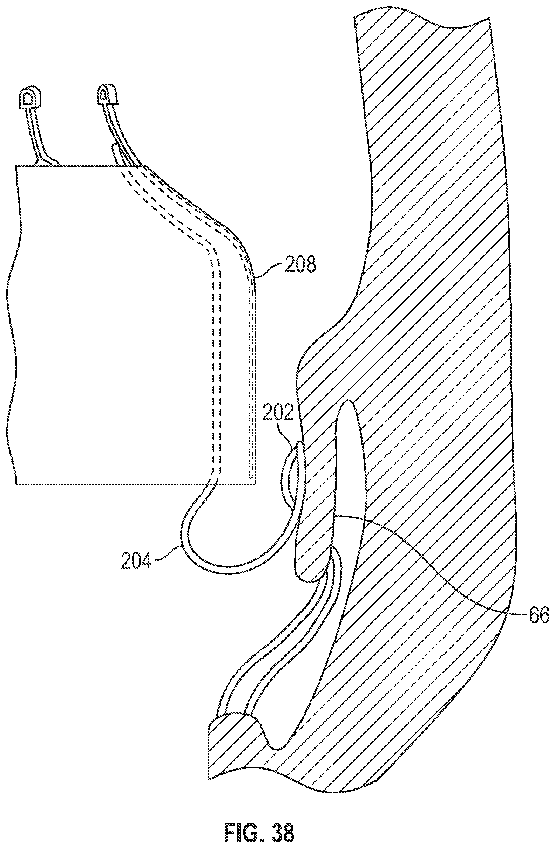

FIG. 38 illustrates a missed capture of a leaflet 66 by an anchor 204. The anchor 204 may be positioned between the sealing body 208 and the leaflet 66 and may prop open a fluid passageway that may result in paravalvular leakage (PVL). The indicator mechanism 202 may be visualized via fluoroscopy and/or ultrasound to determine a coupling of the anchor 204 to the portion of the patient's heart via the visualized indicator mechanism 202. The visualization may show that the indicator mechanism 202 has not moved, and thus missed capture of the leaflet 66 has occurred. The anchor 204 may then be redeployed or other remedial measures may take place to account for the missed capture of the leaflet 66.

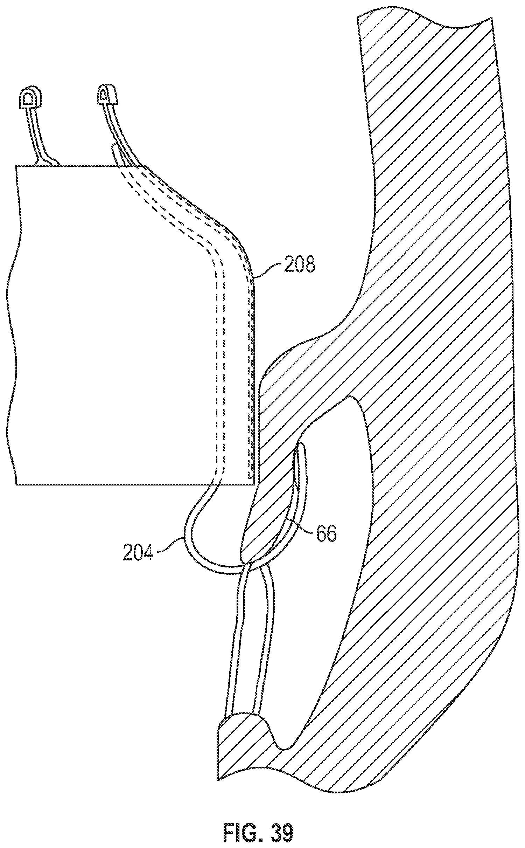

FIG. 39 illustrates an embodiment of a proper capture of the leaflet 66 by the anchor 204. The indicator mechanism 202 has been moved by the captured leaflet 66. The anchor 204 is positioned radially outward of an outward facing surface of the leaflet of the native valve. The movement of the indicator mechanism 202 may be visualized or the lack of presence of the indicator mechanism 202 may be visualized to determine that proper capture has occurred. As discussed, fluoroscopy may beneficially be utilized to visualize the movement of the indicator mechanism 202 or the lack of presence of the indicator mechanism, even though the leaflet 66 may not be visible under fluoroscopy.

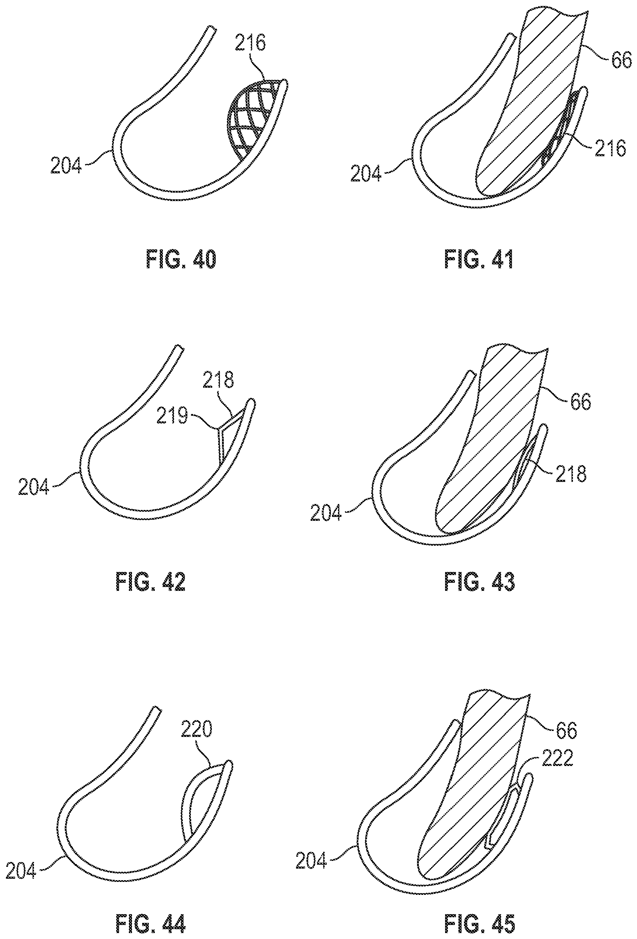

Variations in the indicator mechanisms may be provided. FIG. 40, for example, illustrates an embodiment in which the indicator mechanism 216 may comprise a skirt that may be billowing outward in FIG. 40, yet may be depressed in FIG. 41 to indicate capture of the leaflet 66.

FIGS. 42-45 illustrate embodiments in which an indicator mechanism may move to enhance or reduce an edge, which may vary an appearance of the indicator mechanism under ultrasound (or fluoroscopy).

FIG. 42 illustrates an embodiment in which the indicator mechanism 218 may be configured to reduce an edge 219 upon capture of a leaflet 66. The edge 219 may appear brightly under ultrasound imaging, and the lack of a brightly imaged edge 219 may indicate proper capture. For example, as shown in FIG. 43, the edge 219 of the indicator mechanism 218 may be reduced to reduce the brightness under ultrasound imaging following capture of the leaflet 66. A medical technician accordingly may determine that capture has occurred by the brightness of the edge 219 being reduced in ultrasound imaging.

FIG. 44 illustrates an embodiment in which the indicator mechanism 220 may be configured to produce an edge 222 upon capture of a leaflet 66. The indicator mechanism 220 in FIG. 44 may have a smooth rounded shape, and upon being depressed by the leaflet 66 may produce an edge 222. FIG. 45, for example, illustrates the indicator mechanism 220 depressed and the edge 222 produced. The edge 222 may appear brightly under ultrasound imaging, and the presence of a brightly imaged edge 222 may indicate proper capture. A medical technician accordingly may determine that capture has occurred by the brightness of the edge 222 being enhanced in ultrasound imaging.

Other configurations of indicator mechanisms may be utilized according to embodiments herein, including coil springs, leaf springs, bodies that are filled with material (which may be tissue) during capture, among other forms of indicator mechanisms.

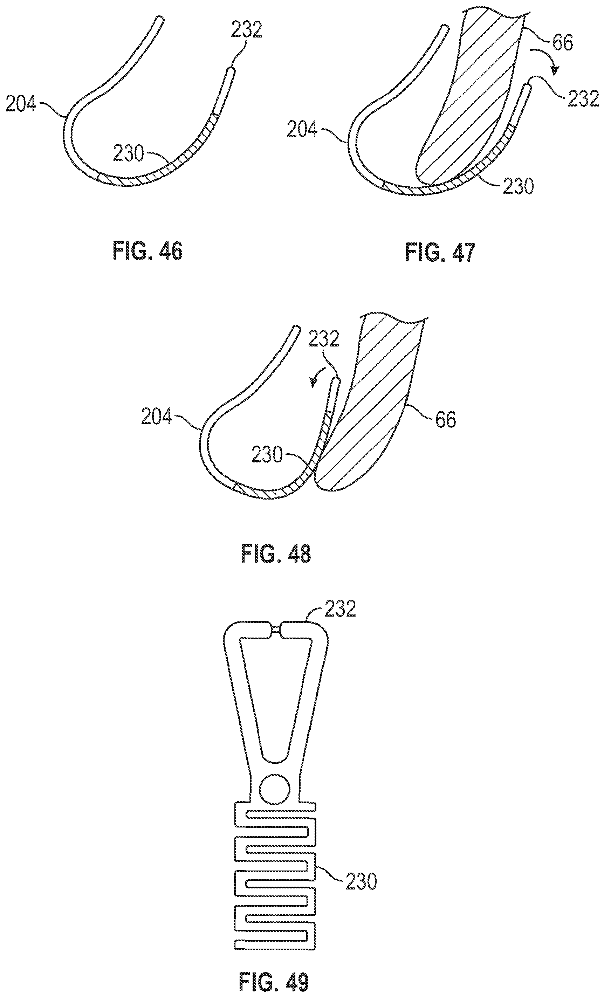

FIG. 46 illustrates an embodiment of an indicator mechanism 230 comprising a flexible portion of an anchor 204. The flexible portion of the anchor 204 may comprise an arm of the anchor, which may produce a flexible tip 232 for the anchor 204. The indicator mechanism 230 may be configured to allow the anchor 204 to deflect radially outward or inward depending on the position of the anchor 204 relative to a portion of a heart. The indicator mechanism 230 may be configured to move in response to an anatomic force.

For example, referring to FIG. 47, upon capture of a leaflet 66, the indicator mechanism 230 may allow the anchor 204 to deflect outward, as indicated by the arrow in FIG. 47. The anchor 204 may deflect outward upon coupling of the anchor 204 to the leaflet 66 of the native valve. Such movement may be visualized utilizing methods disclosed herein, particularly including fluoroscopy. Fluoroscopy may beneficially be utilized to visualize the movement of the indicator mechanism 230, even though the leaflet 66 may not be visible under fluoroscopy.

The indicator mechanism 230 may be utilized to visualize a missed capture of the leaflet 66. FIG. 48, for example, illustrates a missed capture, similar to the missed capture shown in FIG. 38. The anatomic force applied by the leaflet 66 to the anchor 204 may deflect the anchor 204 radially inward. Such movement may be visualized utilizing methods disclosed herein, particularly including fluoroscopy.

In embodiments, the indicator mechanism 230 may have a variety of forms, including one or more reduced thickness portions of the anchor 204 or other materials that may be utilized for the indicator mechanism 230 to produce flexibility. Referring to FIG. 49, in embodiments, the indicator mechanism 230 may comprise an undulating feature of the anchor 204 or "rachis" feature that allows for flex of the anchor tip 232. The amount of flex may be controlled by the thickness of material forming the undulating feature 230 and the spacings between adjacent undulations. Various other forms of indicator mechanisms may be utilized as desired. In embodiments, a flexible portion of the anchor 507 may reduce the possibility of stiff contact with a heart wall that may adversely impact electrical conduction.

The indicator mechanisms may be utilized solely or may be utilized in combination with other embodiments disclosed herein. The indicator mechanisms may be utilized with any embodiment of prosthetic valve disclosed herein. For example, the indicator mechanisms may be utilized with one or more of the anchors 17, 45 shown in FIG. 1, among other anchors disclosed herein. One or more of the anchors 17, 45 may include an indicator mechanism, which may be utilized to visualize coupling of the anchors 17, 45 to the native leaflet or other body. Other anchors or prosthetic valves disclosed herein may utilize the indicator mechanisms.

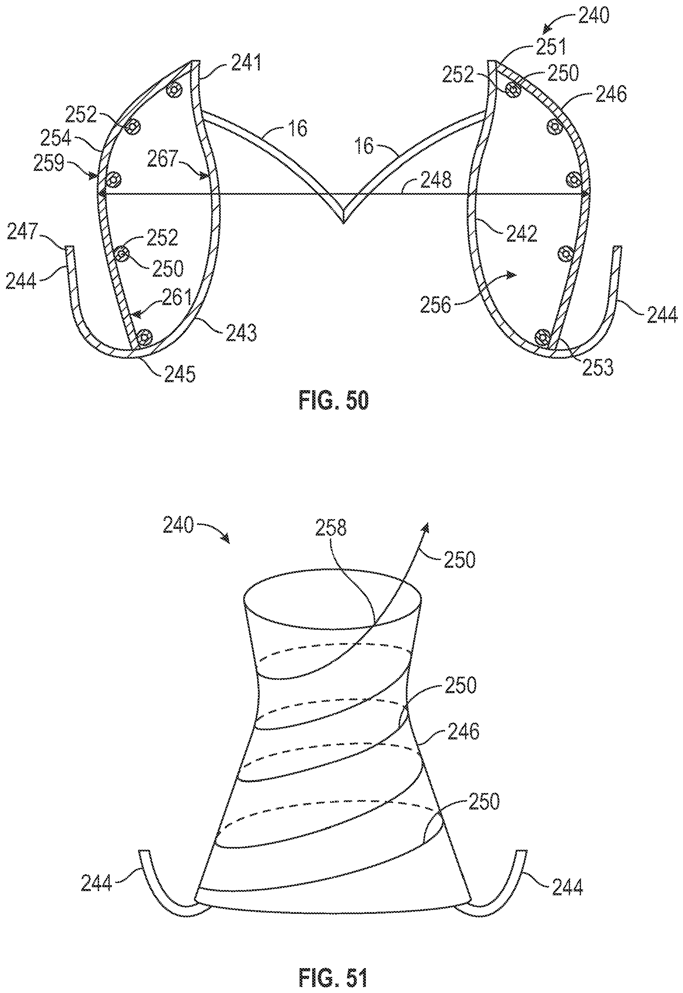

FIG. 50 illustrates a cross sectional schematic view of a prosthetic valve 240 that may include a valve frame or inner frame 242 and one or more anchors 244. The inner frame 242 may support a plurality of prosthetic valve leaflets 16.

The inner frame 242 may include a proximal portion 241 and a distal portion 243. The anchors 244 may be coupled to the distal portion 243 of the inner frame 242. The anchors 244 may each include a proximal portion 245 and a distal portion 247, with the proximal portion 245 coupled to the inner frame 242 and the distal portion 247 comprising a tip of the respective anchor 244. The anchors 244 may extend vertically from the proximal portion 245 to the tip at the distal portion 247 when the prosthetic valve 240 is deployed. The anchors 244 may be configured to hook around a distal tip of a native valve leaflet to anchor the prosthetic valve 240. The anchors 244 may be configured similarly as the anchors 17 shown in FIG. 3 for example. Other configurations of anchors may be utilized in embodiments as desired.

The prosthetic valve 240 may include a sealing body 246 that may be positioned radially outward from the inner frame 242 and configured to seal against a portion of a native valve. In embodiments, the sealing body 246 may be configured to have an adjustable outer diameter 248. The sealing body 246 may comprise a frameless sealing body in embodiments.

The sealing body 246 may include one or more strands 250 that may be configured to adjust the outer diameter 248 of the sealing body 246. The one or more strands 250 may be configured to seal the sealing body 246 against a portion of the native valve. In embodiments, the sealing body 246 may include one or more channels 252 configured to receive the one or more strands 250 and configured for the one or more strands 250 to slide relative to.

The sealing body 246 may include a proximal end 251 and a distal end 253, with the proximal end 251 of the sealing body 246 coupled to a proximal portion 241 of the inner frame 242 and the distal end 253 of the sealing body 246 coupled to the distal portion 243 of the inner frame 242. The sealing body 246 may be configured to be propped outward by the one or more strands 250 such that a gap 256 is positioned between the sealing body 246 and the inner frame 242. The inner frame 242 may be configured similarly as other forms of valve frames or inner frames disclosed herein, and may have a cylindrical shape surrounding a flow channel, or another shape as desired.

The sealing body 246 may include a sealing skirt 254 in embodiments. The sealing skirt 254 may have an outer surface 259 configured to contact a surface of a native valve. The one or more channels 252 may be coupled to the sealing skirt 254 and may be stitched or otherwise coupled to an interior surface 261 or other portion of the sealing skirt 254 in embodiments. The one or more channels 252, for example, may comprise cloth channels or may have another form as desired. The one or more channels 252 may have a variety of configurations, including a configuration of a coil extending circumferentially about the prosthetic valve leaflets 16, and may be configured as a helical coil as shown in FIGS. 50-53 for example. In embodiments, the one or more channels 252 may have other configurations as desired. For example, the one or more channels 252 and the one or more strands 250 may be positioned external to the sealing skirt 254 and may extend along the outer surface 259 of the sealing skirt 254. Other configurations may be utilized as desired.

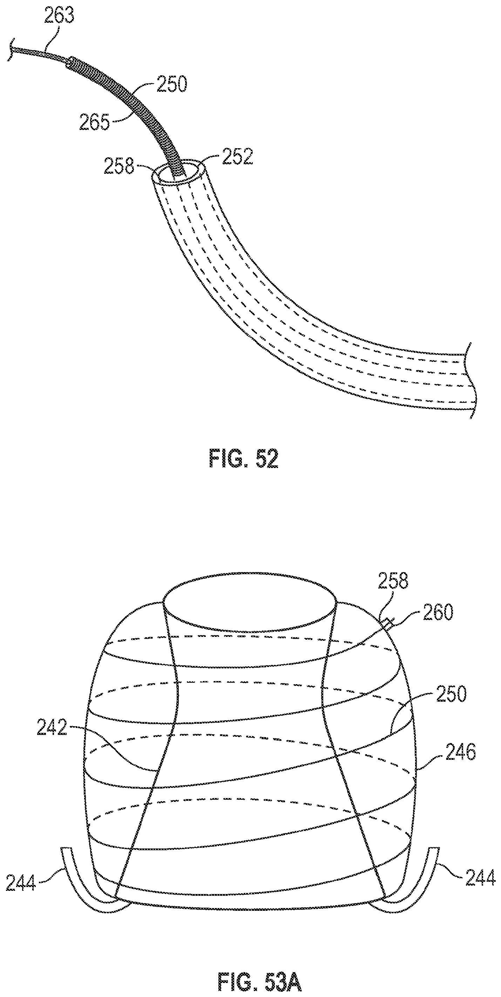

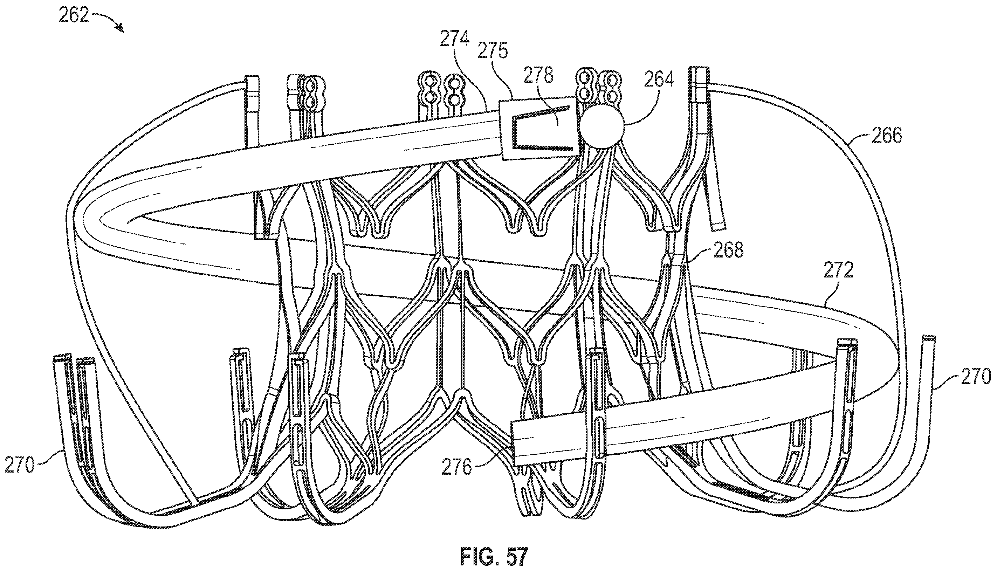

The one or more strands 250 may have a variety of forms and may comprise one or more wires or other form of strand as desired. For example, the one or more strands 250 may comprise a strand of spacer bodies as shown in FIGS. 57-60, or may have another configuration. FIGS. 50-56 illustrate an embodiment in which the one or more strands 250 comprise a wire. Referring to FIG. 52, the wire may include a center core wire 263 in embodiments that may be surrounded with a coil 265. In embodiments, other configurations of wires or other forms of strands may be utilized as desired.

The one or more strands 250 may be configured to slide within the one or more channels 252 and may have a shape that matches a shape of the one or more channels 252. For example, as shown in FIGS. 50-53B, the one or more strands 250 may form a coil that extends around the plurality of prosthetic valve leaflets 16. The coil may comprise a helical coil, extending both circumferentially about the sealing body 246 and axially with respect to the sealing body 246. In embodiments, other configurations may be utilized as desired.

The one or more strands 250 may be configured to be adjusted to adjust the outer diameter 248 of the sealing body 246. For example, the one or more strands 250 may be configured to have a length of the one or more strands 250 adjusted relative to the sealing body 246. A greater length of the one or more strands 250 within the sealing body 246 may increase the outer diameter 248 of the sealing body 246 and a lesser length of the one or more strands 250 within the sealing body 246 may decrease the outer diameter 248 of the sealing body 246. The one or more strands 250 may be configured to be advanced or retracted from the sealing body 246 to adjust the length of the one or more strands 250. The one or more strands 250 may be slid within the one or more channels 252 to advance or retract from the sealing body 246. The one or more strands 250 may be advanced to expand the sealing body 246 and increase a size or diameter of the gap 256 between the sealing body 246 and the valve frame 242.

For example, referring to FIG. 51, a perspective schematic view of the prosthetic valve 240 is shown. The strand 250 is in a retracted position, in which the outer diameter 248 of the sealing body 246 is reduced and the sealing body 246 may be abutted against an outer surface 267 (marked in FIG. 50) of the inner frame 242. As such, the gap 256 between the sealing body 246 and the valve frame 242 may have a reduced size than in the configuration shown in FIG. 50. The one or more strands 250 may form a helical coil within the sealing body 246.

In embodiments, a portion of the one or more strands 250 may extend from the sealing body 246 and external of the sealing body 246. FIG. 52, for example, illustrates a configuration of a strand 250 extending from the channel 252. The strand 250 may be configured to be advanced into the channel 252 or retracted from the channel 252 as desired. Referring to FIGS. 51 and 52, an exit portion 258 of the channel 252 may be positioned on the sealing body 246, or at another point as desired.

The one or more strands 250 may be advanced into the channel 252 to increase the outer diameter 248 of the sealing body 246. FIG. 53A, for example, illustrates the strand 250 having been advanced into the sealing body 246 and expanded the outer diameter 248 from the configuration shown in FIG. 51. The one or more strands 250 may apply a radially outward force to the sealing body 246.

The one or more strands 250 may be advanced to a desired length and then have a length locked with respect to the sealing body 246. For example, referring to FIG. 52, the strand 250 may be advanced through the exit portion 258 to the desired length and then cut and locked with a lock 260 (marked in FIG. 53A) to maintain the length of the strand 250 within the sealing body 246. The lock 260, for example, may comprise an auto-lock mechanism or other form of lock that maintains the length of the strand 250 within the sealing body 246. An end of the strand 250 extending from the lock 260 external to the sealing body 246 may be cut during a deployment procedure.

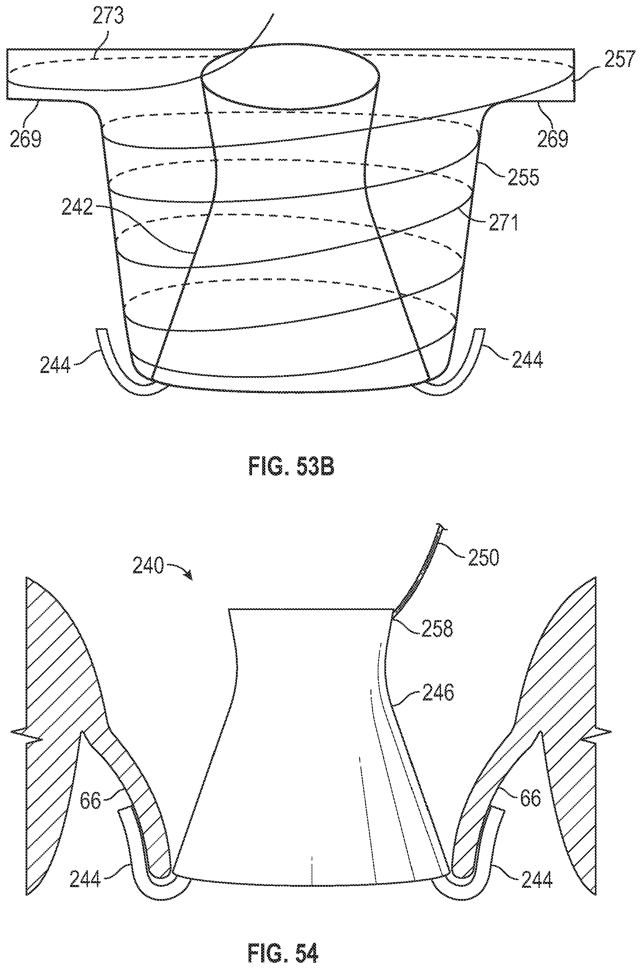

In embodiments, the configuration of the sealing body may be varied from the configuration shown in FIGS. 50-53A. FIG. 53B, for example, illustrates an embodiment in which the sealing body 255 includes a proximal portion 257 having a flange 269 protruding radially outward from the sealing body 255. The flange 269 may protrude radially outward from the sealing body 255 to reduce the possibility of distal movement of the prosthetic valve. The flange 269 may be propped radially outward during the deployment procedure. For example, the strand 271 may prop the flange 269 outward by having a larger diameter coil wrap 273 of the strand 271 within the flange 269. The remining coil wraps of the strand 271 may have a smaller diameter than the larger diameter coil wrap 273 within the flange 269.

The flange 269 may provide improved sealing and retention for the prosthetic valve. In embodiments, the flange 269 may be positioned in an atrium, for a mitral or tricuspid deployment. The flange 269 may contact a proximal or atrial side of a native valve to reduce the possibility of distal movement of the prosthetic valve. Any embodiment of prosthetic valve disclosed herein may include a flange as desired.

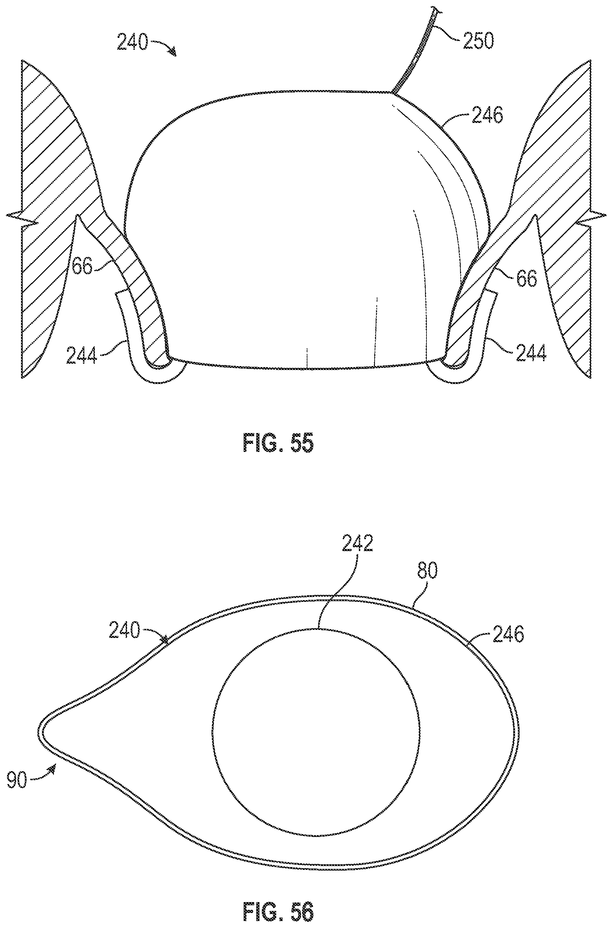

FIG. 54 and 55 illustrate an exemplary deployment procedure. The prosthetic valve 240 shown in FIG. 50 may be advanced to the native valve, with the anchors 244 extending over the native valve leaflets 66. The sealing body 246 may have a reduced outer diameter upon initial deployment, as shown in FIG. 54. An end of the strand 250 may extend from the prosthetic valve 240, for example, from an exit portion 258 of the channel 252.

To adjust the outer diameter of the sealing body 246, the strand 250 may be advanced to the sealing body 246. FIG. 55, for example, illustrates the strand 250 advanced to the sealing body 246 to increase the outer diameter of the sealing body 246. The strand 250 may apply a force radially outward to the sealing body 246 to increase the outer diameter. The strand 250 may seal the sealing body 246 against a portion of the native valve. The sealing body 246, with its expanded diameter, may press against the portion of the native valve, such as the native valve leaflets or the native valve annulus, to seal against the portion of the native valve.

The length of the strand 250 may be adjusted to produce a desired amount of force against the portion of the native valve. The strand 250 may be advanced to press against the portion of the native valve to a desired amount, and then a lock 260 as shown in FIG. 53A or other method may maintain the position of the strand 250 relative to the sealing body 246. The deployment procedure, for example, may be imaged and the outer diameter of the sealing body 246 may be adjusted to a desired size under imaging to seal against the portion of the native valve to a desired amount. In embodiments, a resistive force of the strand 250 may be determined when the strand 250 is advanced to indicate that the size of the sealing body 246 has increased and has sealed against the portion of the native valve. Other methods may be utilized to determine an amount to adjust the outer diameter of the sealing body 246 to.

The adjustment of the outer diameter of the sealing body 246 may occur in vivo, during the deployment procedure. The adjustment may be made by a user during the deployment procedure to provide a desired amount of seal and force applied by the sealing body 246 to the native valve. Further, the adjustment of the outer diameter may account for a variety of sizes of native valves and shapes of the native valve anatomy.

FIG. 56, for example, illustrates a top cross sectional view of the prosthetic valve 240 deployed to a valve annulus 80, in which the diameter of the sealing body 246 has been increased. The sealing body 246 may be flexible, and due to the radially outward force applied by the strand 250 may fit within recesses 90 or other non-circular shapes of the annulus 80. As such, a seal may be provided by the sealing body 246 at a recess 90 and along a non-circular shape of the annulus 80. The inner frame 242, however, may retain a circular shape, with the sealing body 246 expanded to form a non-circular shape.

Various modifications of the sealing body may be provided in embodiments.

FIG. 57 illustrates a cross sectional view of an embodiment of a prosthetic valve 262 in which a plurality of spacer bodies 264 are configured to adjust the outer diameter of the sealing body 266. The prosthetic valve 262 may include a plurality of prosthetic valve leaflets (not shown in FIG. 57) that are supported by a valve frame or inner frame 268. The inner frame 268 may be configured similarly as the inner frame 242 shown in FIG. 50. The prosthetic valve 262 may include one or more anchors 270. The anchors 270 may be configured similarly as the anchors 244 shown in FIG. 50.

The sealing body 266 may comprise a frameless sealing body positioned radially outward from the plurality of prosthetic valve leaflets and configured to seal against a portion of the native valve. The sealing body 266 may be positioned radially outward from the inner frame 268. The sealing body 266 may be configured to have an adjustable outer diameter. The plurality of spacer bodies 264 may form one or more strands that may be configured to adjust the outer diameter of the sealing body 266. The one or more strands may be configured to seal the sealing body 266 against a portion of the native valve.

The sealing body 266 may include a channel 272 that may have a first end or proximal end 274 and a second end or distal end 276. The channel 272, in embodiments, may be configured to receive the one or more strands formed by the plurality of spacer bodies 264, and may form a coil, which may include a helical coil as shown in FIG. 57 or may comprise another shape as desired. In embodiments, the channel 272 and spacer bodies 264 may be positioned interior of a sealing skirt of the sealing body 266. In embodiments, other positions may be utilized. For example, the channel 272 and the spacer bodies 264 may be positioned external to the sealing skirt and may extend along an outer surface of the sealing skirt. Other configurations may be utilized as desired.

The distal end 276 of the channel 272 may comprise a closed end of the channel 272 and the proximal end 274 may include an opening for receiving the spacer bodies 264 in embodiments. The proximal end 274 may include a gate 275 that may allow for entry of the spacer bodies 264 into the channel 272 yet may impede removal of the spacer bodies 264 from the channel. For example, the gate 275 may include one or more arms 278 that are angled to allow for entry through the gate 275 yet impede exit from the gate 275.

The spacer bodies 264 may have a variety of shapes and may comprise beads as shown in FIG. 57, which may be spherical or may have another shape as desired. The spacer bodies 264 may have other configurations that may be configured to fill the channel 272 and adjust the diameter of the sealing body 266.

The number of spacer bodies 264 within the channel 272 may be adjusted to adjust the outer diameter of the sealing body 266. A greater number of spacer bodies 264 within the channel 272 may expand the channel 272 radially outward and adjust the diameter of the sealing body 266 radially outward.

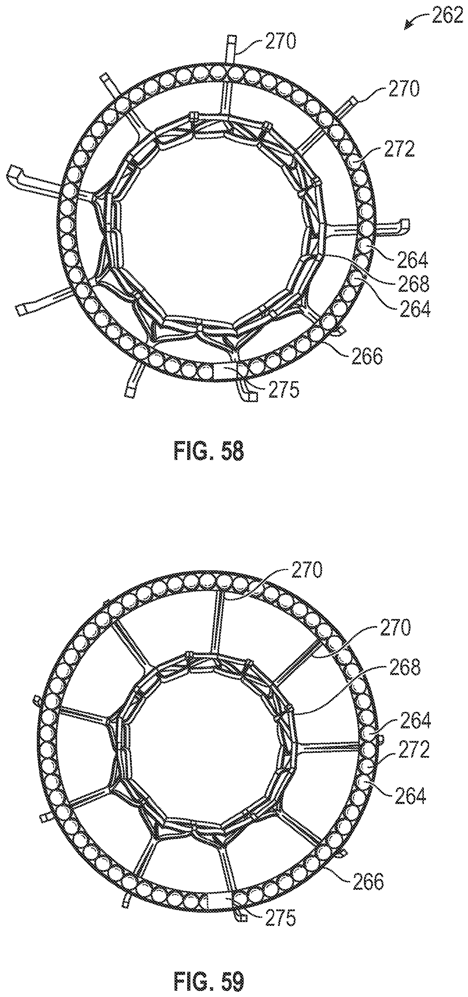

FIG. 58, for example, illustrates a top schematic view of the prosthetic valve 262 in which a relatively low number of spacer bodies 264 are positioned within the channel 272. The spacer bodies 264 may abut against each other within the channel 272 to define an outer diameter of the sealing body 266.

The number of spacer bodies 264 within the channel 272 may be increased by adding more spacer bodies 264 into the channel 272 via the gate 275 for example. FIG. 59 illustrates an increased number of spacer bodies 264 within the channel 272. The outer diameter of the channel 272 has increased and the outer diameter of the sealing body 266 has increased as well. The increased diameter of the sealing body 266 may be determined by a diameter of each individual spacer body 264 that is inserted into the channel 272. For example, the diameter of the sealing body 266 multiplied by "pi" corresponds to the diameter of each individual sealing body 266 multiplied by the number of sealing bodies 266 within the channel 272.

The adjustment of the outer diameter of the sealing body 266 may occur in vivo and during a deployment sequence, similar to the adjustment of the prosthetic valve 240 shown in FIGS. 50-56. A user may determine a number of spacer bodies 264 to advance into the sealing body 266 to increase the diameter of the sealing body 266. The user may sequentially insert the spacer bodies 264 into the channel 272 to increase the diameter of the channel 272 and the diameter of the sealing body 266. A user may determine if a sufficient increase in the outer diameter of the sealing body 266 has occurred and cease adding additional spacer bodies 264 to the channel 272. The gate 275 may impede the removal of the spacer bodies 264 from the channel 272. The force of the spacer bodies 264 against each other may apply a radially outward force to the sealing body 266.

The spacer bodies 264 in embodiments may not be directly connected to each other and may be free to move relative to each other outside of the channel 272. For example, as shown in FIGS. 57-59, the spacer bodies 264 may comprise spheres that are not directly connected to each other and form a strand with a shape defined by the shape of the channel 272. The spheres may be freely separable outside of the channel 272. In embodiments, the spheres may be directly connected to each other via a wire or other form of a connector that extends between the spheres.

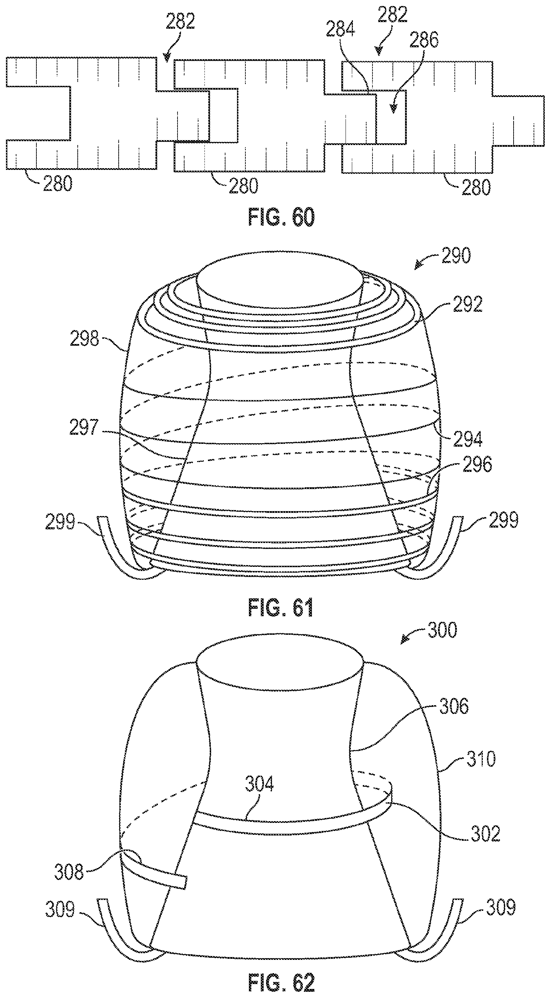

In embodiments, the plurality of spacer bodies may be configured to engage with another one of the plurality of spacer bodies. FIG. 60, for example, illustrates spacer bodies 280 configured to engage each other with interlocking portions 282. The interlocking portions 282 may comprise a protrusion 284 of a first one of the spacer bodies 280 that may be inserted into a cavity 286 of a second one of the spacer bodies 280 that is adjacent. Friction between the protrusion 284 and the cavity 286 may retain the spacer bodies 280 to each other and provide strength for the spacer bodies 280. The spacer bodies 280 may chain together to form a strand that functions in a similar manner as the spacer bodies 264 shown in FIGS. 57-59. The engagement of the spacer bodies 280, however, may exclude the use of a channel 272 as shown in FIGS. 57-59, although a channel may be utilized with the spacer bodies 280 in embodiments if desired. The spacer bodies 280 may extend within a channel 272, similar to the spacer bodies 264 shown in FIGS. 57-59 for example.

A prosthetic valve utilizing a plurality of spacer bodies may have similar benefits as the embodiment shown in FIGS. 50-56. A prosthetic valve utilizing a plurality of spacer bodies may have an adjustable outer diameter for the sealing body, which may be adjusted by the number of spacer bodies advanced to the sealing body. The adjustment may occur in vivo and during deployment. The plurality of sealing bodies may further allow the shape of the sealing body to vary to non-circular shapes (as shown in FIG. 56 for example) to provide a seal with the portion of the native valve.

In embodiments, the spacer bodies may comprise a bioresorbable material. A bioresorbable material may allow for tissue adhesion of the prosthetic valve with the native valve due to the bioresorbable properties of the spacer bodies. In embodiments, all or a portion of the sealing bodies may be bioresorbable to provide an improved seal with the native valve.

FIG. 61 illustrates an embodiment of a prosthetic valve 290 including one or more strands 292, 294, 296 that are configured to adjust the outer diameter of the sealing body 298. The strands 292, 294, 296 may be configured similarly as the strand 250 shown in FIGS. 50-56 and may operate in a similar manner. The one or more strands 292, 294, 296, however, may have varying stiffness. For example, the proximal strand 292 and the distal strand 296 may have a greater stiffness than an intermediate strand 294. The proximal strand 292 and distal strand 296, for example, may be thicker than the intermediate strand 294 or otherwise may be configured with a greater stiffness. The proximal strand 292 and the distal strand 296 may provide for enhanced anchoring of the prosthetic valve 290 due to the enhanced stiffness. The intermediate strand 294 may have a greater compliance to provide for an improved sealing of the sealing body 298 with the native valve.

In embodiments, the strands 292, 294, 296 may comprise separate strands that are inserted into the sealing body 298. In embodiments, the strands 292, 294, 296 may comprise a unitary strand having differing stiffnesses along its length. The configuration of the strands 292, 294, 296 may be varied in embodiments, including the positions and shapes of the strands 292, 294, 296. The valve frame or inner frame 297 of the prosthetic valve 290 may be configured similarly as the inner frame 242 shown in FIG. 50. The prosthetic valve 290 may include one or more anchors 299. The anchors 299 may be configured similarly as the anchors 244 shown in FIG. 50. Other configurations of inner frames and anchors may be utilized as desired.

In embodiments, one or more strands may be biased to apply a radially outward force to a sealing body. FIG. 62, for example, illustrates a prosthetic valve 300 having a strand 302 biased to provide a spring force radially outward. The strand 302 may be shaped as a coil and may have a first end 304 coupled to a valve or inner frame 306 and a second end 308 configured to apply a radially outward force against the sealing body 310.

The strand 302 may comprise a planar coil, or may have another configuration as desired (e.g., a helical coil in embodiments). The ends 304, 308 may be configured to slide relative to each other to allow for expansion of the strand 302 and an increase in diameter of the sealing body 310. The valve frame or inner frame 306 of the prosthetic valve 300 may be configured similarly as the inner frame 242 shown in FIG. 50. The prosthetic valve 300 may include one or more anchors 309. The anchors 309 may be configured similarly as the anchors 244 shown in FIG. 50. Other configurations of inner frames and anchors may be utilized as desired.

The strand 302, in embodiments may be advanced into the sealing body 310 during deployment. In embodiments, the strand 302 may be positioned within the sealing body 310 prior to deployment. For example, the strand 302 may be coupled to the prosthetic valve 300 and placed in a compressed state and then allowed to expand radially outward upon deployment of the prosthetic valve 300.

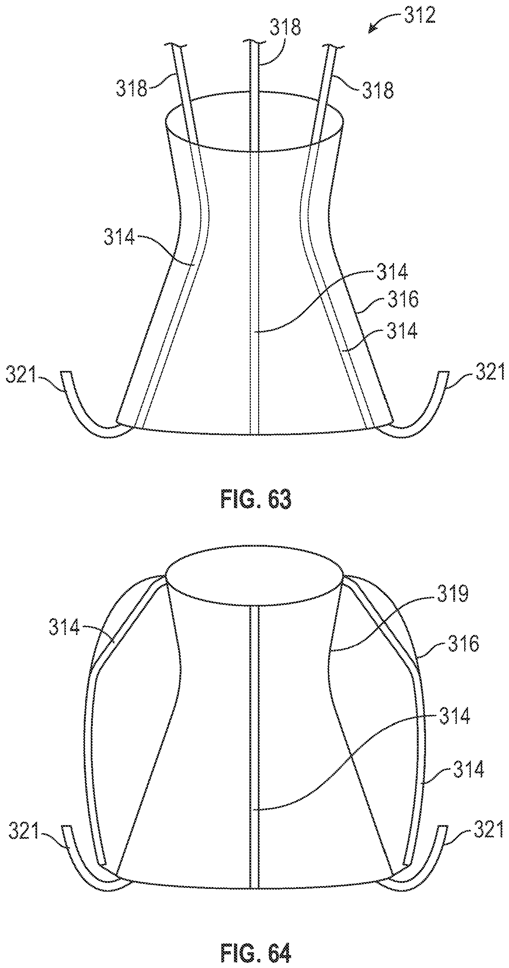

In embodiments, the one or more strands may have a variety of configurations. FIG. 63, for example, illustrates an embodiment of a prosthetic valve 312 in which the one or more strands 314 are configured to extend axially relative to the prosthetic valve leaflets. The one or more strands 314 may be advanced axially along a longitudinal axis of the prosthetic valve 312 and into the sealing body 316. A length of the strands 314 within the sealing body 316 may be adjusted to adjust the outer diameter of the sealing body 316, in a similar manner as the strand shown in FIGS. 50-53 for example. A portion 318 of the strands 314 may be positioned exterior of the sealing body 316 and advanced into the sealing body 316 to increase the diameter of the sealing body 316.

FIG. 64, for example, illustrates the strands 314 having been advanced axially into the sealing body 316 to increase the diameter of the sealing body 316. The length of the strands 314 that is inserted into the sealing body 316 may be adjusted to provide a desired diameter of the sealing body 316. The strands 314 may have their lengths locked, similar to the embodiment of FIGS. 50-56, and a remaining portion 318 of the strands 314 may be cut away if desired. The strands 314 may apply a force to the sealing body 316 to move the sealing body 316 radially outward from the inner frame 319. The valve frame or inner frame 319 of the prosthetic valve 312 may be configured similarly as the inner frame 242 shown in FIG. 50. The prosthetic valve 312 may include one or more anchors 321. The anchors 321 may be configured similarly as the anchors 244 shown in FIG. 50. Other configurations of inner frames and anchors may be utilized as desired.

FIGS. 65-68 illustrate an embodiment of a prosthetic valve 323 in which the sealing body 320 includes a braid material 322. The braid material 322 may be configured to pass axially into the sealing body 320 to cause an outer diameter of the sealing body 320 to expand. FIG. 65, for example, illustrates the braid material 322 being slid axially into the sealing body 320, with the braid material 322 forming a tube that passes into the sealing body 320.

The length of the braid material 322 that passes into the sealing body 320 may be set to provide a desired amount of expansion of the sealing body 320. An outer diameter of the sealing body 320 may be adjusted based on the length of the braid material 322 that passes into the sealing body 320.

FIG. 66, for example, illustrates the braid material 322 having passed into the sealing body 320 and having expanded the outer diameter of the sealing body 320. The braid material 322 may apply an outward expansion force to the sealing body 320 that may provide for improved sealing of the sealing body 320 with the native valve.

In embodiments, the length of the braid material 322 that passes into the sealing body 320 may be set based on a desired amount of expansion of the sealing body 320. The length may be set in vivo, during a deployment procedure. For example, a user may pass a desired length of the braid material 322 into the sealing body 320 and then cut a remaining portion of the braid material 322 that extends out of the sealing body 320 to remove such a portion. An end 324 of the braid material 322 may be locked in position at an exit portion 326 of the sealing body 320 via sutures or another method of locking.

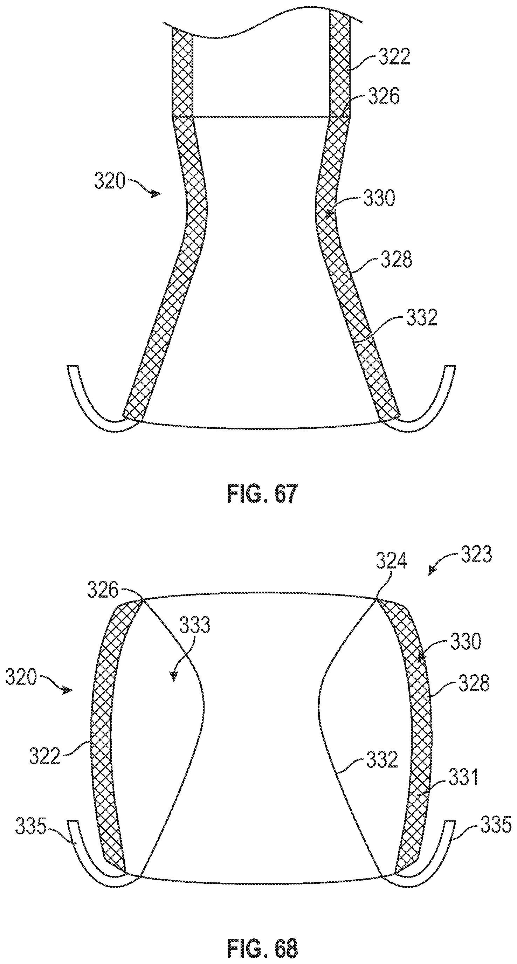

Referring to FIG. 67, the braid material 322 may be positioned radially inward of a sealing skirt 328 and may be positioned within a channel of the sealing skirt 328 for receiving the braid material 322 in embodiments. FIG. 67 illustrates a schematic cross sectional view of the braid material 322 being passed into the sealing body 320 axially. The braid material 322 may pass into a channel 330 that may be positioned radially inward of the sealing skirt 328 in embodiments. The braid material 322 may form a tube around the inner frame 332 and the prosthetic valve leaflets (not shown). The braid material 322 may be configured to be inserted axially between the sealing skirt 328 and the prosthetic valve leaflets.

FIG. 68 illustrates a schematic cross sectional view of the braid material 322 being passed into the sealing body 320, in the configuration shown in FIG. 66. FIG. 68 illustrates the braid material 322 positioned within the channel 330. The end 324 of the braid material 322 may be locked in position at an exit portion 326 of the sealing body 320. The braid material 322 may form an axially extending tube having an outer wall 331 surrounding a central cavity 333. The plurality of prosthetic valve leaflets and the inner frame 332 may be positioned within the central cavity 333. The braid material 322 may form an axially extending tube positioned between the sealing skirt 328 and the inner frame 332.

The valve frame or inner frame 332 of the prosthetic valve 323 may be configured similarly as the inner frame 242 shown in FIG. 50. The prosthetic valve 323 may include one or more anchors 335. The anchors 335 may be configured similarly as the anchors 244 shown in FIG. 50. Other configurations of inner frames and anchors may be utilized as desired.

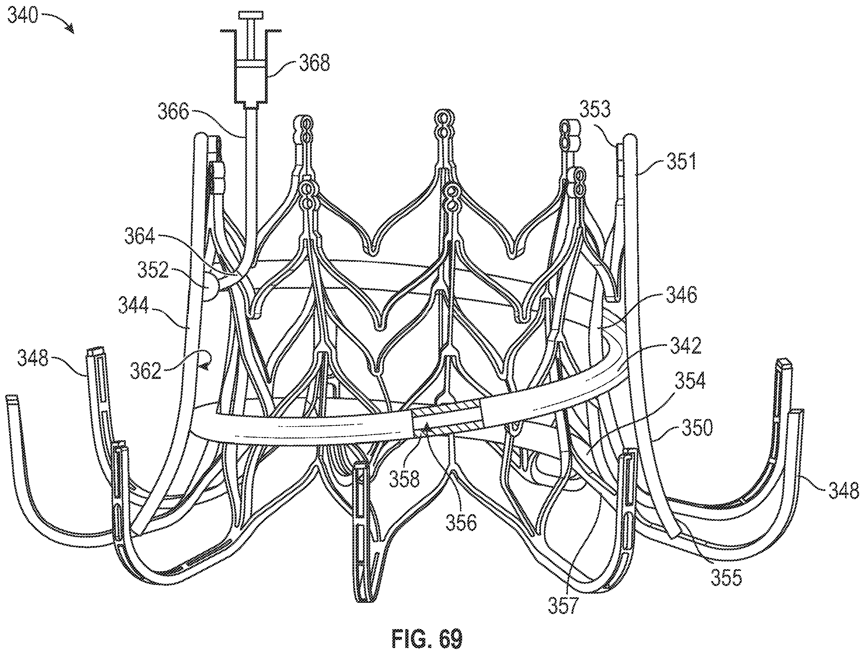

FIG. 69 illustrates a cross sectional view of an embodiment of a prosthetic valve 340 in which an inflatable body 342 is coupled to a plurality of prosthetic valve leaflets (not shown in FIG. 69) and is configured to seal against a portion of a native valve. The inflatable body 342 may be configured to adjust the outer diameter of the sealing body 344. The plurality of prosthetic valve leaflets may be supported by a valve frame or inner frame 346. The valve frame or inner frame 346 may be configured similarly as the inner frame 242 shown in FIG. 50. The prosthetic valve 340 may include one or more anchors 348. The one or more anchors 348 may comprise distal anchors as disclosed herein, which may be configured to extend over a distal tip of a native valve leaflet in embodiments. The anchors 348 may be configured similarly as the anchors 244 shown in FIG. 50. Other forms of anchors may be utilized in embodiments.

The sealing body 344 may comprise a frameless sealing body positioned radially outward from the plurality of prosthetic valve leaflets and configured to seal against a portion of the native valve. The sealing body 344 may be configured to have an adjustable outer diameter. The inflatable body 342 may be configured to be inflated to adjust the outer diameter of the sealing body 344. At least a portion of the frameless sealing body may include the inflatable body 342.

The inflatable body 342 may be positioned radially outward of the plurality of prosthetic valve leaflets and may be positioned radially outward of the inner frame 346 in embodiments. The inflatable body 342 may be positioned between a sealing skirt 350 and the inner frame 346. In embodiments, the inflatable body 342 may have another position as desired.

In embodiments, the inflatable body 342 may be positioned interior of the sealing skirt 350 of the sealing body 344. In embodiments, other positions may be utilized. For example, the inflatable body 342 may be positioned external to the sealing skirt 350 and may extend along an outer surface of the sealing skirt. Other configurations may be utilized as desired.

The sealing skirt 350 may have a proximal end 351 coupled to a proximal portion 353 of the inner frame 346 and may have a distal end 355 coupled to a distal portion 357 of the inner frame 346. The sealing skirt 350 may form a cylindrical outer covering for the inner frame 346 or may have another configuration in embodiments.

The inflatable body 342 may be configured to extend circumferentially about the inner frame 346 and the plurality of prosthetic valve leaflets. The inflatable body 342 may be configured to form a ring about the inner frame 346 and the plurality of prosthetic valve leaflets. In embodiments, the inflatable body 342 may form a coil extending around the inner frame 346 and the plurality of prosthetic valve leaflets. The inflatable body 342 may include a first end 352 and a second end 354 with the inflatable body 342 forming a coil between the ends. As shown in FIG. 69, the coil may comprise a helical coil extending both axially and circumferentially. The first end 352 of the helical coil may be positioned at a height relative to the second end 354 of the helical coil. The coil may extend around the inner frame 346.

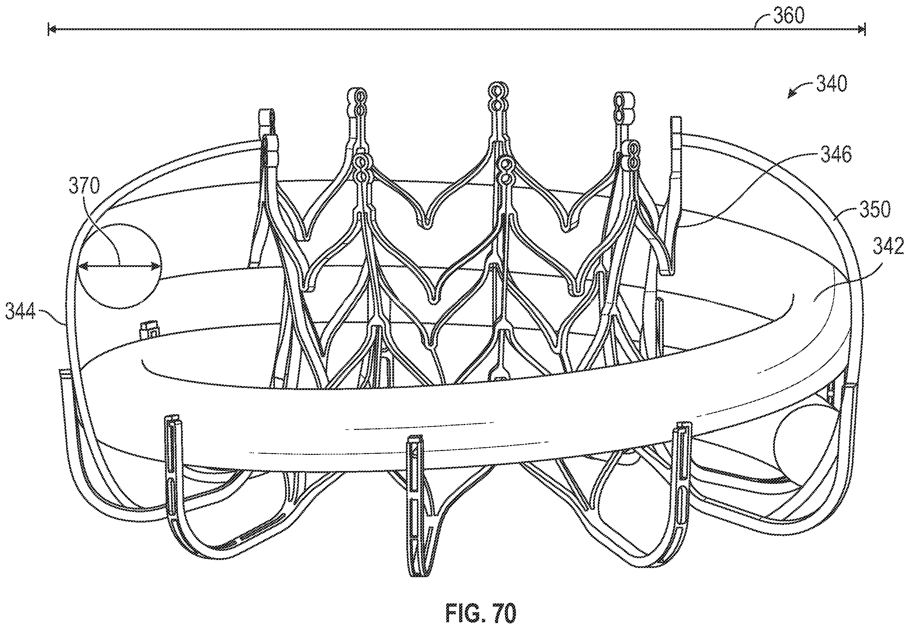

The inflatable body 342 may include an interior chamber 356 (shown in cross sectional view in FIG. 69) that may be configured to fill with fill material. The interior chamber 356 may be surrounded by a wall 358 that may be configured to expand in size upon the fill material filling the interior chamber 356. A diameter of the wall 358 may increase upon the fill material filling the interior chamber 356. The increased diameter of the wall 358 may cause the diameter of the coiled inflatable body 342 to increase, thus increasing the diameter 360 of the sealing body 344 (marked in FIG. 70 for example). The inflatable body 342 may increase in diameter and press against an interior surface 362 of the sealing skirt 350 to cause the diameter of the sealing body 344 to increase.

In embodiments, the inflatable body 342 may include an inflation port 364 for receiving the fill material. The inflation port 364 may be coupled to a conduit 366 for the fill material and may be coupled to an inflation device 368 that may be configured to add or remove fill material from the inflation port 364 and the inflatable body 342. The inflation port 364 may include a one-way valve or other form of valve. In embodiments the inflation port 364 may allow for addition or removal of the fill material from the inflatable body 342. The inflation device 368 may comprise a syringe or other form of device for adding or removing fill material from the inflation port 364 and the inflatable body 342.

In embodiments the diameter 360 of the sealing body 344 may be adjusted during deployment or in vivo. For example, the anchors 348 and inner frame 346 may be deployed to a desired implantation site and the inflatable body 342 may then be inflated to a desired diameter. The conduit 366 for example, may be coupled to the inflation port 364 and the inflation device 368 may be utilized to fill the inflatable body 342 with fill material via the inflation port 364. The inflation device 368, for example, may be exterior to the patient's body and controlled exterior to the patient's body, or may be positioned within the patient's body during a procedure if desired.

The inflatable body 342 may be inflated during the deployment procedure. The deployment procedure, for example, may be imaged and the outer diameter of the sealing body 344 may be adjusted to a desired size under imaging to seal against the portion of the native valve to a desired amount. In embodiments, a resistive force of the inflatable body 342 may be determined to indicate that the size of the sealing body 344 has increased and has sealed against the portion of the native valve. Other methods may be utilized to determine an amount to adjust the outer diameter of the sealing body 344 to.

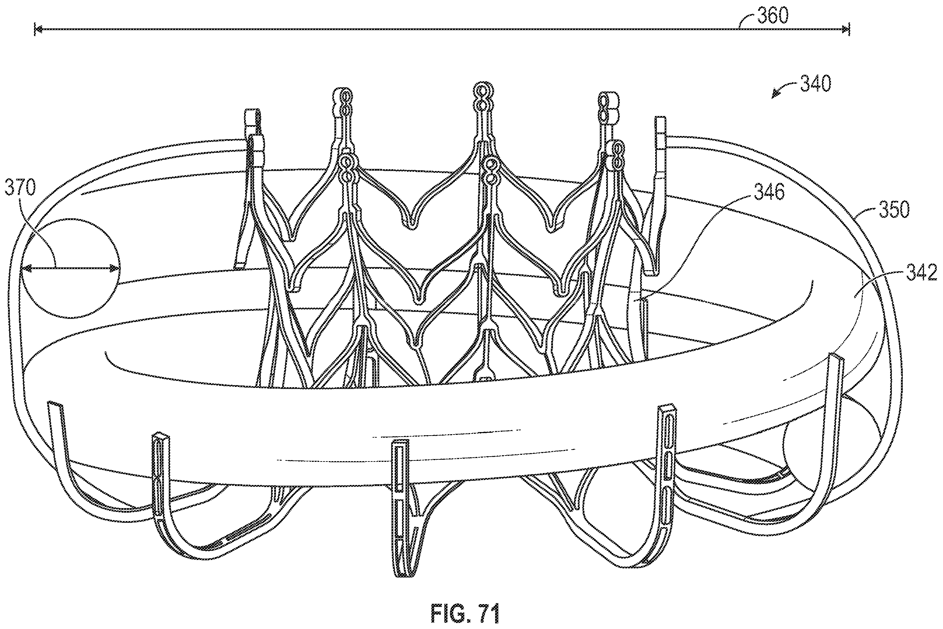

FIG. 70 illustrates the increase in diameter 370 of the wall 358 of the inflatable body 342 and the increase in diameter 360 of the sealing body 344. The conduit 366 and the inflation device 368 are not shown in FIG. 70, although they may be utilized to inflate the inflatable body 342. The size of the inflatable body 342 may increase as shown in FIG. 71, with the diameter 360 increased further than shown in FIG. 70. The inflatable body 342 may allow the shape of the sealing body 344 to vary to non-circular shapes (as shown in FIG. 56 for example) to provide a seal with the portion of the native valve. Further, in embodiments, the inflatable body 342 may be biased to expand radially outward, thus allowing for expansion and reduction of the diameter of the inflatable body 342 with a corresponding expansion or reduction of the diameter of the valve annulus.

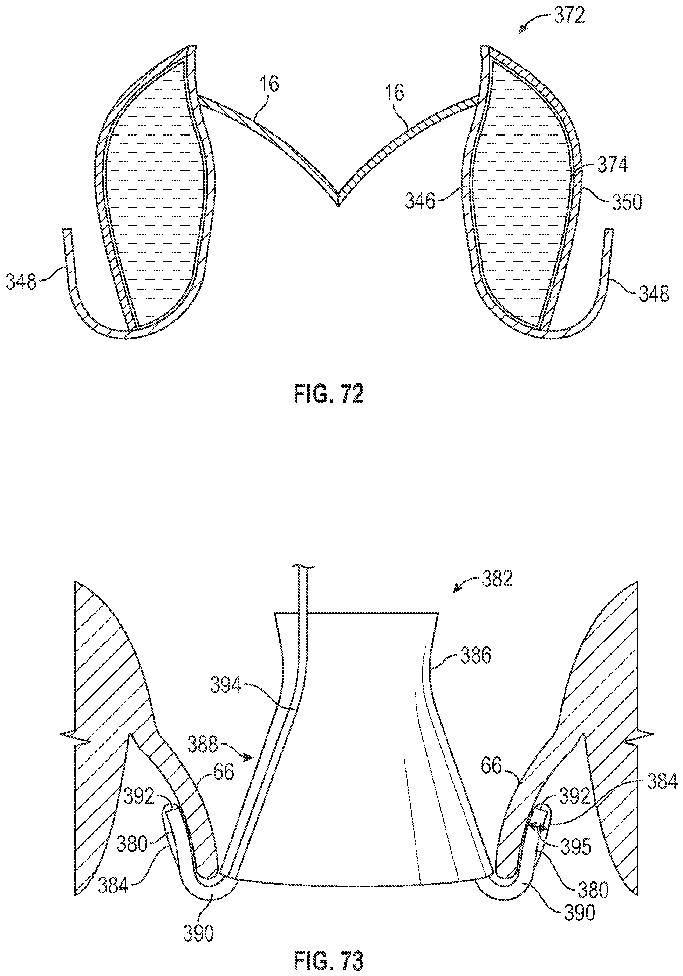

The inflatable body may have a variety of configurations in embodiments. FIG. 72, for example, illustrates a cross sectional schematic view of a prosthetic valve 372 including an inflatable body 374 forming a tube about the inner frame 346 and the plurality of prosthetic valve leaflets 16. The inflatable body 374 may extend circumferentially about the inner frame 346 and the plurality of prosthetic valve leaflets 16. The inflatable body 374 may be positioned between the inner frame 346 and the sealing skirt 350. The inflatable body 374 may include an inflation port, which may be configured similarly as the inflation port 364, and configured to couple to a conduit 366 and an inflation device 368. The inflation port may be for receiving a fill material.

The inflatable body 374 may be configured to fill with fill material to cause the inflatable body 374 to apply a radially outward force against the sealing skirt 350, in a similar manner as the inflatable body 342 having a coil shape as shown in FIGS. 69-71. The inflatable body 374 may be configured to press against the outer surface of the inner frame 346 and the inner surface of the sealing skirt 350 to cause the sealing body 344 to increase in diameter.

In embodiments, one or more anchors may each have a diameter configured to increase. FIG. 73, for example, illustrates one or more anchors 380 of a prosthetic valve 382 configured to anchor a valve body 386 to a native valve and that each have a diameter configured to increase. The one or more anchors 380 each include an inflatable body 384 that may be configured to increase a diameter of the one or more anchors 380.

The prosthetic valve 382 may include a plurality of prosthetic valve leaflets (not shown in FIG. 73) and a valve body 386 supporting the plurality of prosthetic valve leaflets. The valve body 386 may include an outer surface 388 configured to seal against a portion of a native valve. For example, the valve body 386 may include a valve frame and may include a sealing body that forms the outer surface 388 of the valve body 386. The sealing body may include a sealing skirt that forms the outer surface 388 of the valve body 386 or in embodiments another portion of the valve body 386 may form the outer surface 388.

The one or more anchors 380 may be configured to anchor the valve body 386 to the native valve. The one or more anchors 380, for example, may be configured to extend over a distal tip of a native valve leaflet to anchor the valve body 386 to the native valve. The one or more anchors 380 may each include an arm 390 extending to a distal tip 392, with the arm 390 extending proximally to the distal tip 392. The arm 390 may be configured to extend over a distal tip of a native valve leaflet. The distal tip 392 in embodiments may have a diameter configured to increase.

The inflatable body 384 may be coupled to the respective arm 390 of the one or more anchors 380 and may be positioned at the distal tip 392 of the respective anchor 380 or at another position as desired. In embodiments, one or more of the inflatable bodies 384 may be coupled to a conduit 394, which may be configured similarly as the conduit 366. The conduit 394 may be configured to fill the one or more inflatable bodies 384 with a fill material to inflate the inflatable body 384 or to remove the fill material from the inflatable body 384 to deflate the inflatable body 384 as desired. The conduit 394 may be configured to couple to an inflation device, which may be similar to the inflation device 368 discussed in regard to FIG. 69. The one or more inflatable bodies 384 may include an inflation port for receiving the fill material.

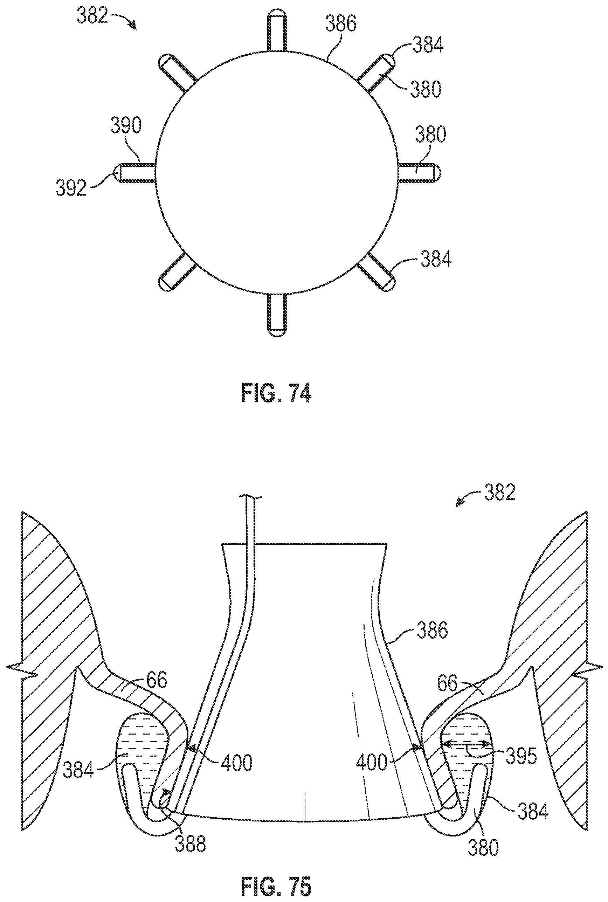

A plurality of the anchors 380 may be spaced circumferentially from each other. FIG. 74, for example, illustrates a top schematic view of the prosthetic valve 382 showing the anchors 380 extending radially outward from the valve body 386.

In embodiments, each of the inflatable bodies 384 may be configured to inflate radially inward to increase a diameter 395 (marked in FIG. 73) of the respective anchor 380. FIG. 75, for example, illustrates the inflatable bodies 384 each having inflated radially inward due to the inflation of the respective inflatable bodies 384. The diameter of the respective anchor 380 increases.

The inflatable bodies 384 may inflate radially inward to press the native valve leaflet 66 against the valve body 386 to seal against the portion of the native valve. The inflatable bodies 384, for example, may extend to press an inward facing surface 400 of the native valve leaflets 66 against the outer surface 388 of the valve body 386. The pressure applied by the inflatable bodies 384 against the outer surface 388 of the valve body 386 may form a fluid seal to reduce the possibility of fluid leakage therethrough. The native valve leaflet 66 may be pinched between the inflatable bodies 384 and the outer surface 388 of the valve body 386.

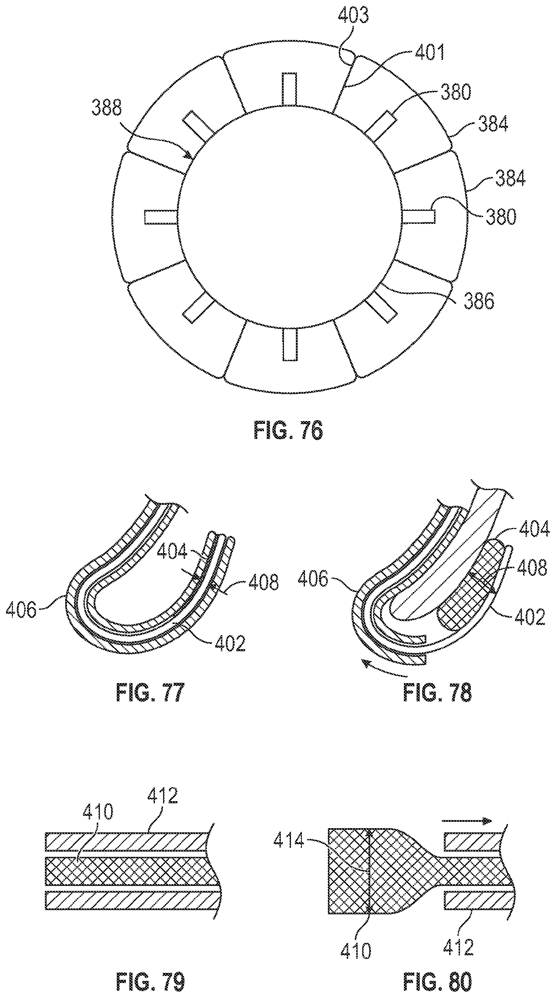

FIG. 76 illustrates a top schematic view of the inflatable bodies 384 inflated, in a configuration as shown in FIG. 75. In embodiments, side surfaces 401, 403 of adjacent inflatable bodies 384 may press against each other and contact each other to form a seal between adjacent inflatable bodies 384. An increase in diameter of the anchors 380 may accordingly be circumferential as shown in FIG. 76, as well as radial as shown in FIG. 75. The inflatable bodies 384 may form a ring about the valve body 386, positioned outside of the native valve leaflets 66. The ring may reduce fluid flow along the outer surface 388 of the valve body 386.

In embodiments, other methods may be utilized to expand a diameter of the anchors. For example, referring to FIGS. 77 and 78, one or more anchors 402 may be self-expandable to increase the diameter of the respective anchor 402. For example, the anchors 402 may each include a self-expandable body 404 that may be configured to expand upon a restraining force being removed from the respective body 404. The self-expandable body 404 may be made of a shape memory or may have another configuration that causes the self-expandable body 404 to expand. A shape memory material may comprise nitinol or may have another form in embodiments as desired. Other forms of self-expandable bodies may include foam bodies or spring bodies that are configured to expand.

An anchor with a diameter configured to increase may be deployed in a small diameter configuration to allow the anchors to slip through chordae or other structures of the heart. The anchors may then have the diameter increased to improve sealing as desired.

Referring to FIG. 77, a restraining body, such as a sleeve 406 or other form of restraining body may extend over the self-expandable body 404 to prevent the self-expandable body 404 from expanding. The self-expandable body may be in a reduced diameter state. Referring to FIG. 78, the restraining body may be removed from the self-expandable body 404, with the sleeve 406 retracted. The self-expandable body 404 may expand to increase the diameter 408 of the anchor 402. The diameter 408 of the anchor 402 may increase radially inward and circumferentially, which may provide the resulting configuration shown in FIGS. 75 and 76 for example. The increased diameter of the anchors 402 may provide for enhanced sealing against the outer surface 388 of the valve body 386.

In embodiments, one or more strands disclosed herein may be self-expandable. A diameter of the one or more strands may be configured to increase. Referring to FIG. 79, a strand 410 may be restrained by a restraining body 412, such as a sleeve. The restraining body 412 may be retracted or otherwise removed from the strand 410 to allow the diameter 414 of the strand 410 to increase, as shown in FIG. 80. The strand 410 may be utilized as a strand in any of the embodiments shown in FIGS. 50-64, for example, or another embodiment of prosthetic valve as desired. The strand 410 may comprise a braid, a shape memory material, a spring body, a foam body, or other structure configured to expand.

A fill material that may be utilized in embodiments herein may comprise a fluid such as saline or other forms of fluid. The fill material may be a liquid, foam, epoxy, gas, or other material. The fill material may be liquid to result in a hydraulic inflation of the inflatable bodies disclosed herein. A fill material in the form of a gas may comprise carbon dioxide or helium, among other forms of gas.

In embodiments, the fill material may comprise a hardenable material. The fill material may be configured to harden over time to enhance the sealing of the sealing body. The hardenable material that may be introduced into an inflatable body at a first, relatively low viscosity and converted to a second, relatively high viscosity. Viscosity enhancement may be accomplished through a variety of UV initiated or catalyst initiated polymerization reactions, or other chemical system. The end point of the viscosity enhancing process may result in a hardness anywhere from a gel to a rigid structure, depending on the desired performance.

A hardenable material may comprise an epoxy. The epoxy may be hardened by mixing materials that harden when combined. The hardening catalyst may be delivered during implantation or later. The hardenable material may be biocompatible and able to conform to the shape of the local native valve. In embodiments, the hardenable material may be bioresorbable.

In embodiments, the fill material may be radiopaque for visualization during implantation. A radiopaque material may be added during filling, as part of a hardening process for example. In embodiments, a portion of a sealing body, such as a sealing skirt may be radiopaque to allow for visualization during implantation.

In embodiments, the fill material may comprise a gel or a foam, which may be biocompatible, and may be configured to harden over time. A gel or foam may be inserted into the sealing body, or may be provided in capsules that dissolve upon implantation to allow for expansion.

In embodiments, a gel may be utilized that may be made via polymer precipitation from biocompatible solvents. Various siloxanes may be utilized as inflation gels as well. Other gel systems that may be utilized may include phase change systems that gel upon heating or cooling from their initial liquid or thixotropic state. Gels may also comprise thixotropic material that undergo sufficient shear-thinning so that they may be readily injected through a fluid conduit yet are also gel-like at zero or low shear rates.

In embodiments, a fill material may contain a foaming agent. The foaming agent may generate pressure within the inflatable body.

Any of the fill materials disclosed herein may be biocompatible in embodiments and may be bioresorbable if desired. A bioresorbable sealing body may improve sealing through tissue adhesion with the native valve.

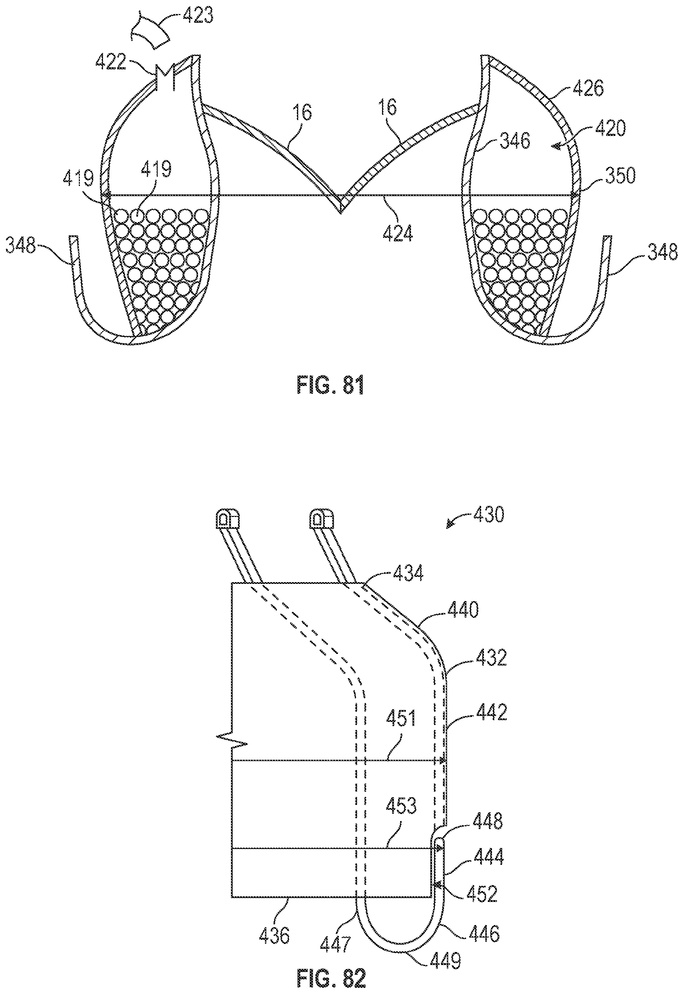

In embodiments, a fill material may comprise filler bodies 419 that may be configured to increase a diameter of a sealing body. The diameter of the sealing body may be an adjustable outer diameter. The plurality of filler bodies may be configured to fill the chamber 420 to adjust the adjustable outer diameter. For example, referring to FIG. 81, the filler bodies 419 may comprise beads or other forms of individual bodies that may fill a chamber 420. The chamber 420 may be positioned between an inner frame 346 and a sealing skirt 350 or may have another position as desired. The chamber 420 may include a port 422 that may be configured to receive the filler bodies 419. A dispensing device 423, for example, may couple to the port 422 to fill the port 422 and the chamber 420 with the filler bodies 419. The chamber 420 may be filled with the filler bodies 419 to increase the diameter 424 of the sealing body 426. The chamber 420 may be filled until a desired diameter 424 of the sealing body 426 is produced.

In the embodiments of FIGS. 50-81, the sealing body comprises a frameless sealing body. In embodiments, a frame may be incorporated with the sealing bodies as desired. The bodies for increasing the diameter of the sealing body may be positioned radially inward of a sealing skirt or may be positioned radially outward of a sealing skirt in embodiments as desired.

Combinations of features of the embodiments of FIGS. 50-81, as well as features of other embodiments herein, may be provided as desired. The configurations of the embodiments of FIGS. 50-81 may be utilized solely or may be utilized in combination with other embodiments disclosed herein.

The embodiments of prosthetic valves may be configured to be deployed to a mitral valve or a tricuspid valve, although other implantation sites may be utilized including an aortic or pulmonary valve as desired. Other implantation sites may be utilized in embodiments. The prosthetic valves may be delivered to the native valve in a similar manner as the prosthetic valve 10 shown in FIG. 4. For example, a delivery apparatus may be utilized to deploy the valve to the native valve. The distal anchors may extend over the distal tip of the leaflets of the native valve.

FIG. 82 illustrates an embodiment of a prosthetic valve 430 configured to be deployed to a native valve. The prosthetic valve 430 may include a plurality of prosthetic valve leaflets (not shown in FIG. 82).

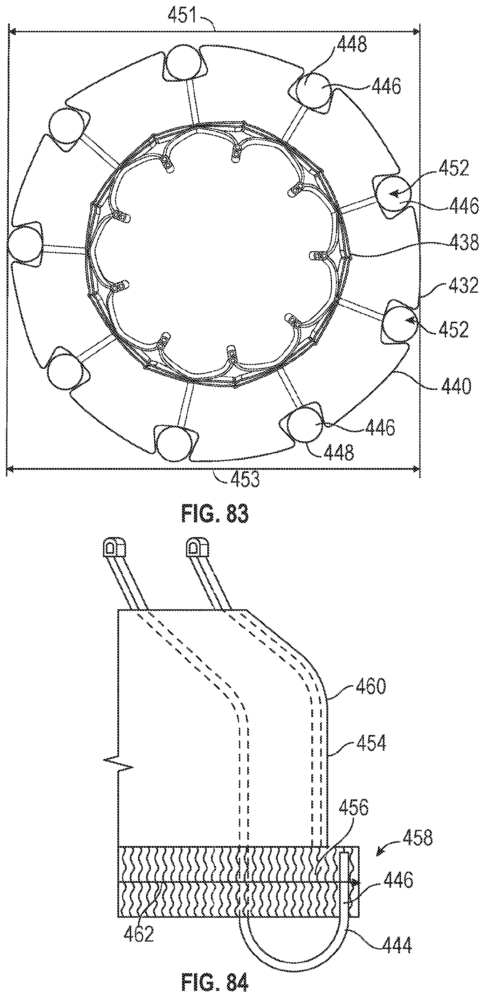

The prosthetic valve 430 may include a valve body 432 having a proximal portion 434 and a distal portion 436 and supporting the plurality of prosthetic valve leaflets. The valve body 432 may include a valve frame or inner frame 438 (marked in FIG. 83) in embodiments, and may include a sealing body 440 that may be configured to form a seal with a portion of a patient's heart. The sealing body 440 may include an outer frame and/or a sealing skirt 442 for forming the seal with the portion of the patient's heart. The sealing body 440 may be positioned radially outward of the inner frame 438 and may surround the inner frame 438.

The prosthetic valve 430 may include one or more anchors 444 positioned at the distal portion 436 of a valve body 432. Each of the one or more anchors 444 has a first portion 446 that extends proximally to a tip 448 of the respective anchor 444. The first portion 446 passes through at least a portion of the valve body 432. Each of the one or more anchors 444 may be configured similarly as the anchors 17 shown in FIG. 3 for example. The one or more anchors 444 may be configured to hook around a distal end of a native leaflet of a native valve, with the first portion 446 positioned radially outward of the native leaflet. The anchors 444 may be configured to retain the native leaflet between the anchors 444 and the valve body 432. The anchors 444 in embodiments may be configured to pinch the native leaflet between the respective anchor 444 and the valve body 432. The anchors 444 may each include a second portion 447 that extends distally from the inner frame 438 to a bend portion 449. The bend portion 449 may bend to direct the anchors 444 from a distal direction to a proximal direction. The first portion 446 may extend proximally from the bend portion 449 to the tip 448 of the respective anchor 444.

Referring to FIG. 83, a plurality of the anchors 444 may extend radially outward from the inner frame 438 and may be spaced circumferentially from each other. The plurality of anchors 444 may form an outer diameter 453. The valve body 432 may form an outer diameter 451 that may be at or greater than the outer diameter 453 of the plurality of anchors 444. The first portion 446 of each of the anchors 444 may be positioned at least partially within the valve body 432. For example, as shown in FIGS. 82 and 83, the first portion 446 may pass at least partially through the sealing body 440. The tip 448 of the respective anchor 444 may be positioned at least partially within the valve body 432.

Referring to FIGS. 82 and 83, in embodiments, the valve body 432 may include pockets 452 that are configured to receive the first portion 446 of the anchors 444. The sealing body 440 may be configured to include pockets 452 shaped to receive the anchors 444. In embodiments, a sealing skirt 442 may be stitched to include the pockets 452.

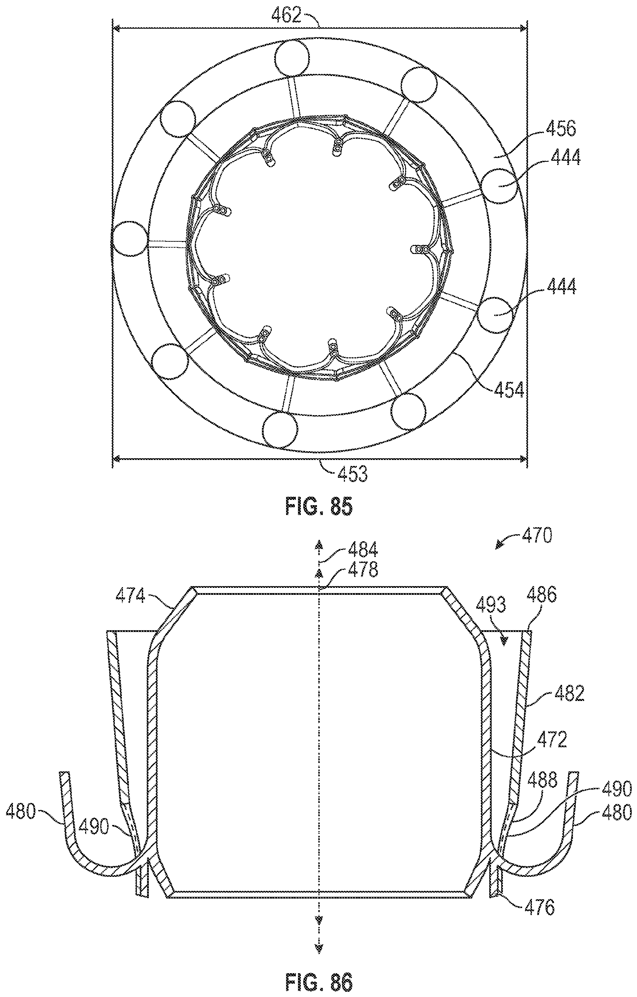

Other configurations of valve bodies may be utilized in embodiments. FIGS. 84 and 85, for example, illustrate an embodiment of a valve body 454 including a skirt 456 having a ring shape extending circumferentially about a distal portion 458 of the valve body 454. The skirt 456 may form a portion or all of a sealing body 460 of the valve body 454, and may comprise the sealing skirt for the valve body 454. The skirt 456 may comprise a fuzzy skirt that the first portion 446 of the anchor 444 passes through.

Referring to FIG. 85, an outer diameter 462 of the skirt 456 may be at or greater than the outer diameter 453 formed by the plurality of anchors 444.

In embodiments, the outer profile of the prosthetic valve may be equal to the outer diameter of the sealing body.

The first portions of the anchors 444 may pass through at least a portion of the valve body to reduce the overall diameter of the prosthetic valve, and to reduce the possibility of contact between the anchors 444 and a wall of the heart. Such a configuration may reduce the possibility of electrical conduction interference of the heart due to the presence of the prosthetic valve. The overall outer profile of the prosthetic valve may be reduced, to reduce the possibility of contact and interference with a wall of the heart in embodiments. A reduced overall outer profile of the prosthetic valve may further allow for ventricular remodeling, including right ventricular remodeling.

The embodiments of prosthetic valves may be configured to be deployed to a mitral valve or a tricuspid valve, although other implantation sites may be utilized including an aortic or pulmonary valve as desired. Other implantation sites may be utilized in embodiments. The prosthetic valves may be delivered to the native valve in a similar manner as the prosthetic valve 10 shown in FIG. 4. For example, a delivery apparatus may be utilized to deploy the valve to the native valve. The distal anchors may extend over the distal tip of the leaflets of the native valve.

Combinations of features of the embodiments of FIGS. 82-85, as well as features of other embodiments herein, may be provided as desired. The configurations of the embodiments of FIGS. 82-85 may be utilized solely or may be utilized in combination with other embodiments disclosed herein.

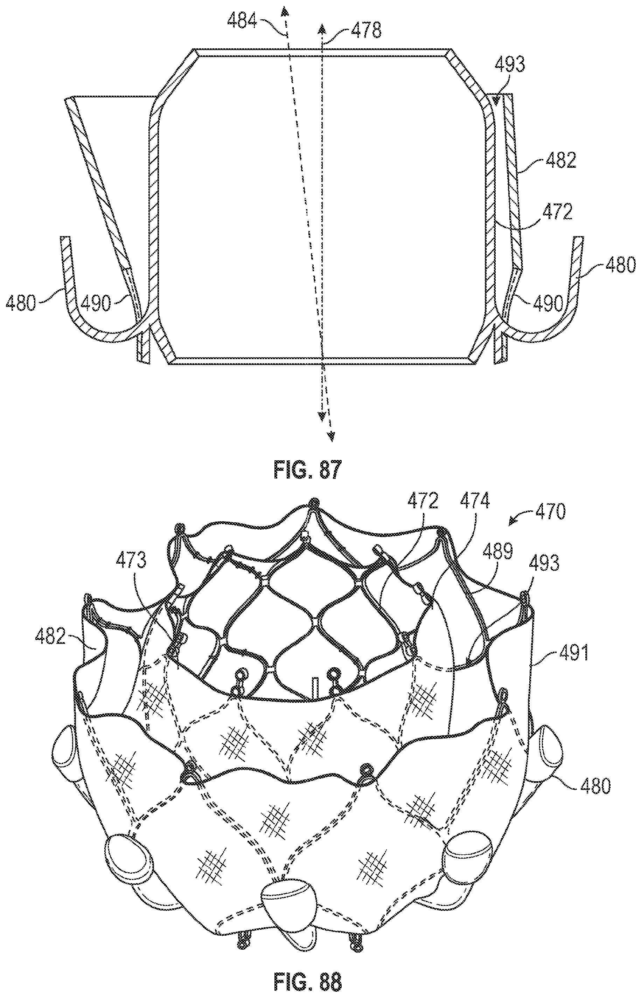

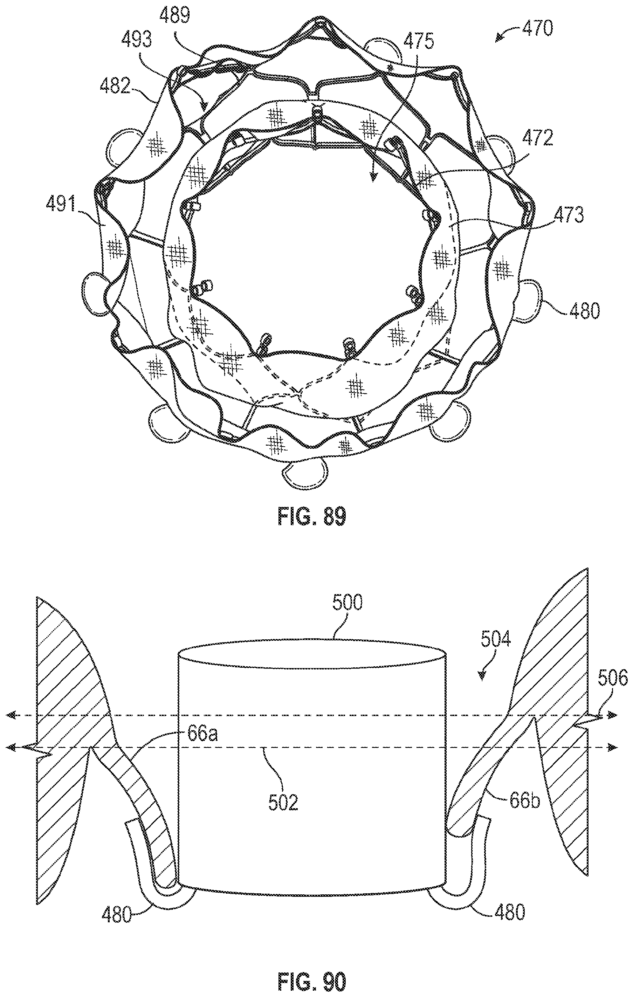

FIG. 86 illustrates an embodiment of a prosthetic valve 470 including a valve frame or inner frame 472 supporting a plurality of prosthetic valve leaflets (not shown) and having a proximal portion 474 and a distal portion 476 and extending along a first axis 478. The inner frame 472 may include a skirt 473 (marked in FIG. 88) coupled thereto. The inner frame 472 may surround a flow channel 475 (marked in FIG. 89). Other configurations of inner frames 472 may be utilized in embodiments.

A plurality of anchors 480 may be positioned at the distal portion 476 of the inner frame 472 and may be configured to couple to a native valve. Each of the anchors 480 may be configured similarly as the anchors 17 shown in FIG. 3 for example. The anchors 480 may be configured to hook around a distal end of a native leaflet of a native valve. The anchors 480 may be configured to retain the native leaflet between the anchors 480 and a sealing body 482.

The sealing body 482 may be positioned radially outward from the inner frame 472 and may have a proximal end 486 and a distal end 488 that is coupled to the distal portion 476 of the inner frame 472 such that the sealing body 482 is configured to move with respect to the inner frame 472. In embodiments, the prosthetic valve 470 may include one or more flexible bodies 490 that may couple the distal end 488 of the sealing body 482 to the distal portion 476 of the inner frame 472 and that allow the sealing body 482 to move with respect to the inner frame 472. The one or more flexible bodies 490 may extend from the inner frame 472 to one or more connection points on the sealing body 482. The one or more flexible bodies 490 may be shaped as arms in embodiments or may have another shape as desired. The one or more flexible bodies 490 may comprise a fabric or a shape memory material (such as Nitinol) in embodiments or may have another configuration as desired.

The sealing body 482 may be configured to seal against a portion of a patient's heart valve. The sealing body 482 may include a frame (which may be an outer frame) 489 (as marked in FIG. 88) and may include a sealing skirt 491 (as marked in FIG. 88) in embodiments, although other configurations may be utilized as desired. The sealing body 482 may be tapered radially inward in a direction from the proximal end 486 towards the distal end 488, although other configurations may be utilized as desired. The sealing body 482 may extend about a second axis 484.

The sealing body 482 may be spaced from the inner frame 472 with an annular gap 493. The proximal end 486 of the sealing body 482 may form an opening for the annular gap 493. The annular gap 493 may be closed at the distal end 488 of the sealing body 482. For example, the flexible bodies 490 may comprise a closure of the space between the distal end 488 of the sealing body 482 and the inner frame 472. In an embodiment in which the flexible bodies 490 comprise a cloth material, the cloth material may form a seal of the distal end 488 of the sealing body 482 for example.

The distal end 488 of the sealing body 482 may be coupled to the distal portion 476 of the inner frame 472 such that the sealing body 482 may be configured to move axially with respect to the inner frame 472. For example, the sealing body 482 may move distally along the axis 484 or proximally along the axis 484 relative to the inner frame 472. In embodiments, the distal end 488 of the sealing body 482 may be coupled to the distal portion 476 of the inner frame 472 such that the sealing body 482 is configured to tilt with respect to the inner frame 472 to offset the first axis 478 from the second axis 484.

FIG. 87, for example, illustrates a tilt of the sealing body 482 with respect to the inner frame 472. The second axis 484 is offset from the first axis 478. The flexible bodies 490 have deflected to allow the sealing body 482 to move with respect to the inner frame 472.

FIG. 88 illustrates a perspective view of the prosthetic valve 470. FIG. 89 illustrates a top view of the prosthetic valve 470.

Upon deployment, the sealing body 482 may be configured to move with respect to the inner frame 472 to allow the sealing body 482 to conform to a variety of configurations of native valves. FIGS. 90-92 illustrate an exemplary deployment sequence. In FIG. 90, the valve may be retained within a capsule 500 of a delivery apparatus. The native valve, notably, has an offset height of one native valve leaflet 66a relative to another native valve leaflet 66b. A plane 502 of the annulus 504 at the native valve leaflet 66a is offset from the plane 506 of the annulus 504 at the other native valve leaflet 66b.

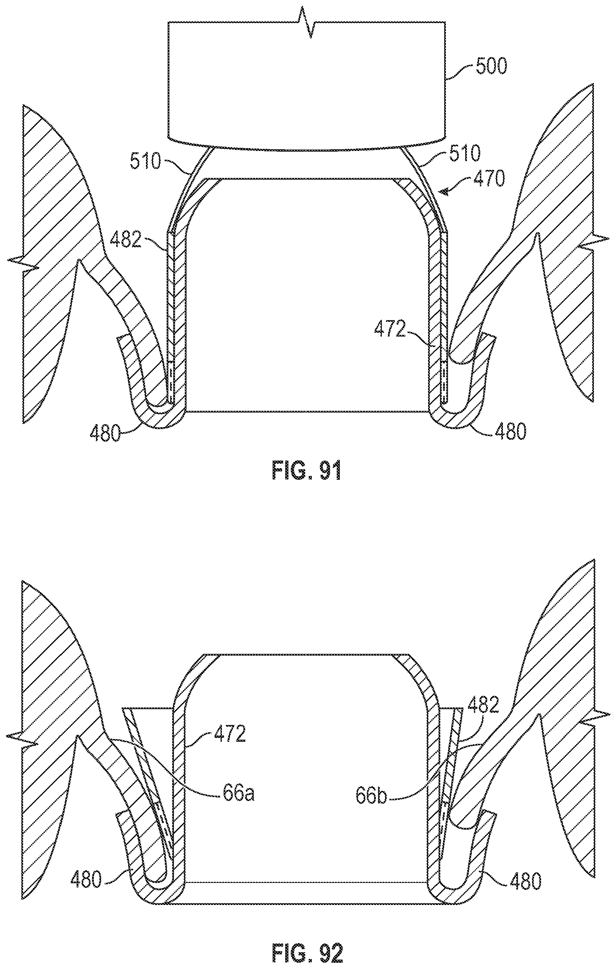

FIG. 91 illustrates a deployment of the prosthetic valve 470. The capsule 500 has retracted. The inner frame 472 and anchors 480 have been released by the capsule 500. In embodiments, couplers 510 such as sutures may retain the sealing body 482 in a reduced diameter configuration. The sealing body 482 for example may be pressed against the outer surface of the inner frame 472, with the size of the gap 493 reduced. The anchors 480 may have a large leaflet capture window due to the sealing body 482 being held towards the inner frame 472.

Referring to FIG. 92, in a next step the couplers 510 may be released and the sealing body 482 may be deployed. The deployment of the sealing body 482 may seat the prosthetic valve 470 at the native valve. The sealing body 482 may be configured to move with respect to the inner frame 472 to account for the offset planes 502, 506 of the annulus 504 shown in FIG. 90. For example, as shown in FIG. 92, axial movement and a tilt of the sealing body 482 relative to the inner frame 472 may be provided. Improved sealing with the native valve may result. Sealing to non-circular or non-planar valve annuli may be provided in embodiments.

The anchors 480 may resist proximal or atrial migration and the tapered shape of the sealing body 482 may resist distal or ventricular migration. The leaflets may be pinched between the anchors 480 and the sealing body 482. The sealing body 482 may be configured to move to adapt to the local shape of the native valve to provide improved sealing, and may be conformable to conform to the local shape of the native valve. In embodiments, the sealing body 482 may accommodate various valve positions due to chordal tension.

The embodiments of prosthetic valves may be configured to be deployed to a mitral valve or a tricuspid valve, although other implantation sites may be utilized including an aortic or pulmonary valve as desired. Other implantation sites may be utilized in embodiments. The prosthetic valves may be delivered to the native valve in a similar manner as the prosthetic valve 10 shown in FIG. 4.

Combinations of features of the embodiments of FIGS. 86-92, as well as features of other embodiments herein, may be provided as desired. The configurations of the embodiments of FIGS. 86-92 may be utilized solely or may be utilized in combination with other embodiments disclosed herein.

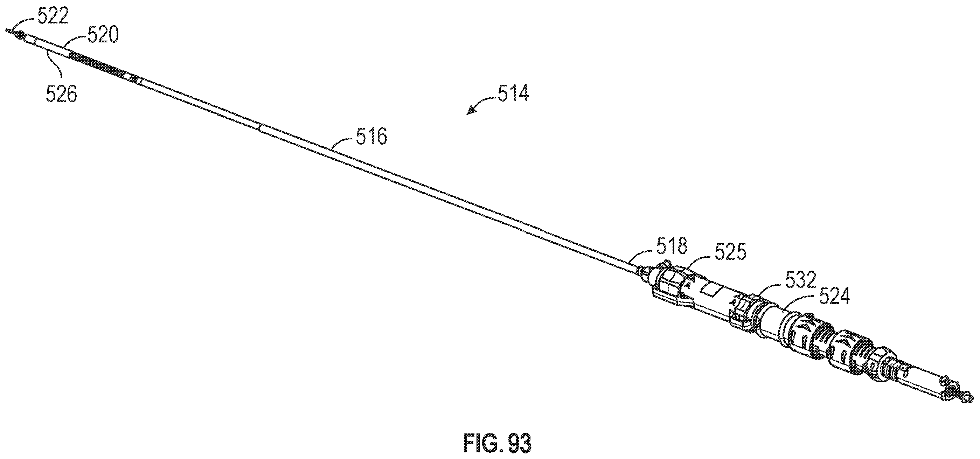

FIG. 93 illustrates a perspective view of a delivery apparatus 514 that may be utilized in embodiments herein. The delivery apparatus 514 may be utilized in combination with the features of embodiments disclosed herein or may be utilized solely, as desired. The delivery apparatus 514 may be utilized in a deployment procedure, which may be similar to the deployment procedure shown in FIG. 4, for example. The delivery apparatus 514 may include an elongate shaft 516 that may be configured for insertion into a portion of a patient's body. The elongate shaft 516 may include a proximal end 518 and a distal end 520. The distal end 520 of the elongate shaft 516 may include a tip 522. The proximal end 518 may couple to a housing, which may be in the form of a handle 524. The handle 524 may be configured for a user to grip and control during a deployment procedure.

In embodiments, at least a portion of the elongate shaft 516 may be deflectable. The delivery apparatus 514 may include a deflection mechanism, which may be controlled via a control device 525 at the handle 524 or at another position as desired. The control device 525 may comprise a knob or may have another form in embodiments as desired. The elongate shaft 516 may be configured to deflect in one or more planes.

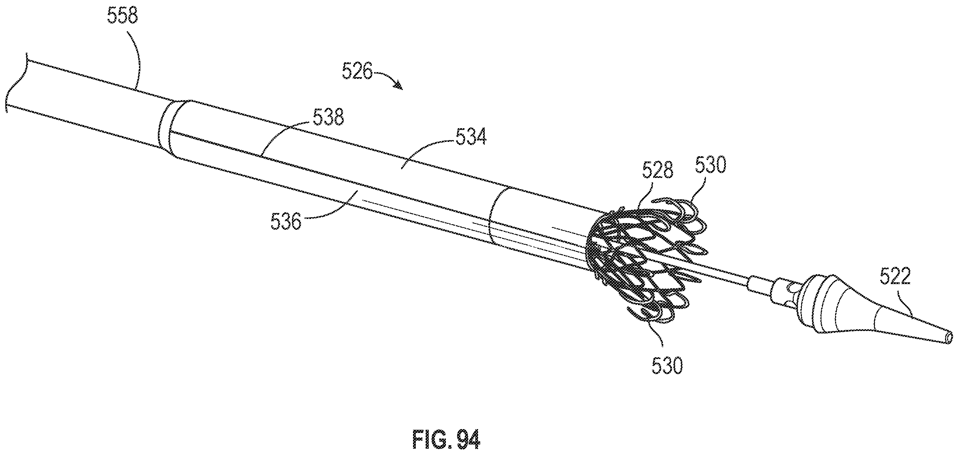

A capsule 526 may be provided at the distal end 520 of the elongate shaft 516 in embodiments. FIG. 94, for example, illustrates a perspective view of the capsule 526. The capsule 526 may be coupled to the elongate shaft 516 and configured to, at least partially, release a prosthetic valve 528. A frame of a prosthetic valve 528 is shown in FIG. 94, along with anchors 530 of the prosthetic valve 528.

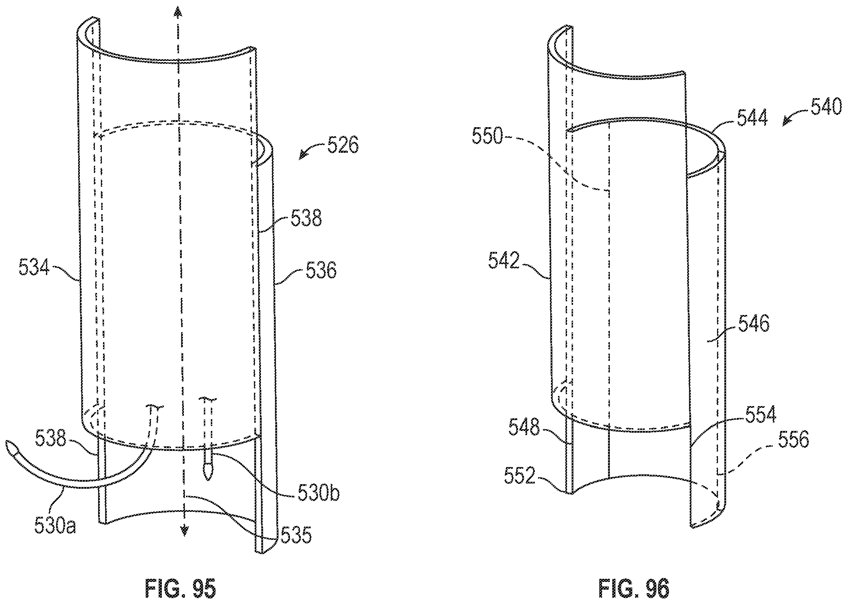

The capsule 526 is shown partially deploying the prosthetic valve 528 in FIG. 94. A portion of the capsule 526 may be configured to be actuated. For example, the delivery apparatus 514 may include an actuation mechanism that may be configured to retract a portion of the capsule 526. A control device 532 may be positioned at the handle 524 or at another portion of the delivery apparatus 514 for retracting a portion of the capsule 526. The control device 532 may comprise a knob or may have another form as desired. In FIG. 94, both sides 534, 536 of the capsule 526 are retracted, however, as shown in FIG. 95, the sides 534, 536 may be retracted individually.

FIG. 95 illustrates the capsule 526 extending along an axis 535. The capsule 526 may include a least two sides (a first side 534 and a second side 536) surrounding the axis 535. The first side 534 may comprise a half of the capsule 526 and the second side 536 may comprise a half of the capsule 526. A split 538 may extend along the capsule 526 and may separate the first side 534 from the second side 536. The split 538 may extend along an axial plane of the capsule 526, and the axis 535 may extend along the axial plane. In embodiments, other configurations of splits 538 may be utilized as desired.

The first side 534 may be configured to retract axially relative to the second side 536. The retraction may allow the capsule 526 to, at least partially, release the prosthetic valve. In embodiments, the first side 534 may be configured to retract independently of the second side 536. The actuation mechanism and a control device 532 such as a control device shown in FIG. 93 may be utilized to retract the first side 534 relative to the second side 536.

In embodiments, the capsule 526 may be configured as a cylinder, with the first side 534 comprising a half-cylinder and the second side 536 comprising a half-cylinder. Other shapes and configurations of the capsule 526 may be provided as desired.

FIG. 96 illustrates an embodiment in which a capsule 540 may include more than two sides (sides 542, 544, 546, 548), each separated from each other with a split 550, 552, 554, 556. Each of the sides 542, 544, 546, 548 may be configured to retract independently of each other.

Referring again to FIG. 95, the sides 534, 536 may be configured to retract independently of each other to control a deployment of a prosthetic valve 528. In embodiments, the sides 534, 536 may be configured to retract independently of each other to independently release anchors 530a, b of the prosthetic valve 528. The first side 534 for example may be configured to retract to release a first anchor 530a, and the second side 536 may be configured to retract to independently release a second anchor 530b. As shown in FIG. 95, the first side 534 has retracted relative to the second side 536 and an anchor 530a of a prosthetic valve is deployed. The second side 536 may retain another anchor 530b with the capsule 526. As such, independent deployment of the anchors 530a, 530b may result.

Referring to FIG. 94, in embodiments, the elongate shaft 516 may include a pivotal portion 558 that the capsule 526 may be configured to pivot about. The capsule 526 may be configured to pivot in one or more planes of the axis 535 shown in FIG. 95 in embodiments.

The use of the sides 534, 536 and a pivotal portion 558 may allow for improved control of deployment of anchors of a prosthetic valve 528. The improved control may account for a variety of shapes of native valves that the prosthetic valve 528 may be deployed to. For example, a non-planar annulus or an irregular leaflet geometry may be accounted for. Irregular leaflet geometry may be provided due to congenital reasons or due to annular dilation. The improved control may also account for a variety of delivery approaches to a native valve provided by the delivery apparatus 415.

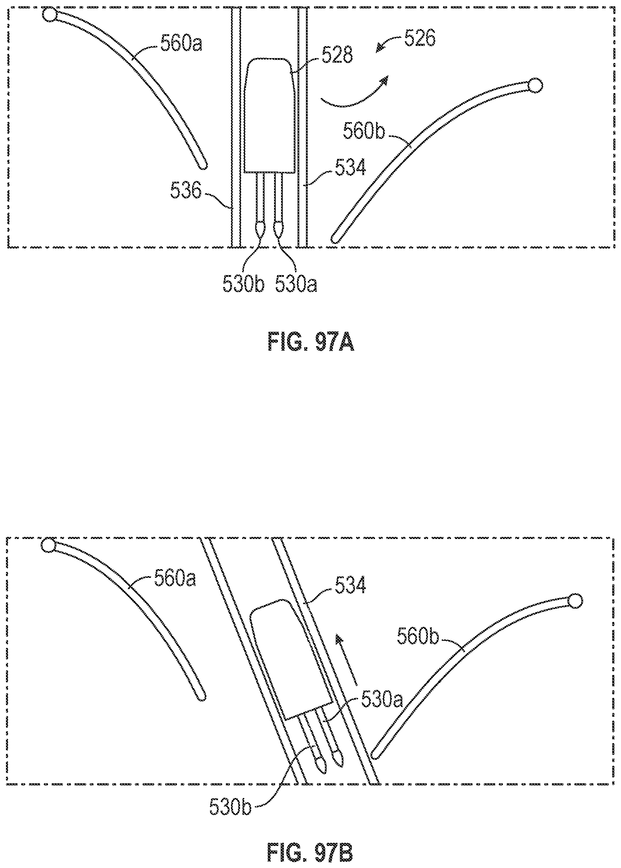

An exemplary deployment sequence is shown in FIGS. 97A-97F. In FIG. 97A, the capsule 526 may be positioned relative to the native valve leaflets 560a, 560b. The native valve leaflets 560a, 560b may have an offset position from each other, with the leaflet 560a being positioned in an offset plane from the leaflet 560b. The prosthetic valve 528 is shown within the capsule 526.

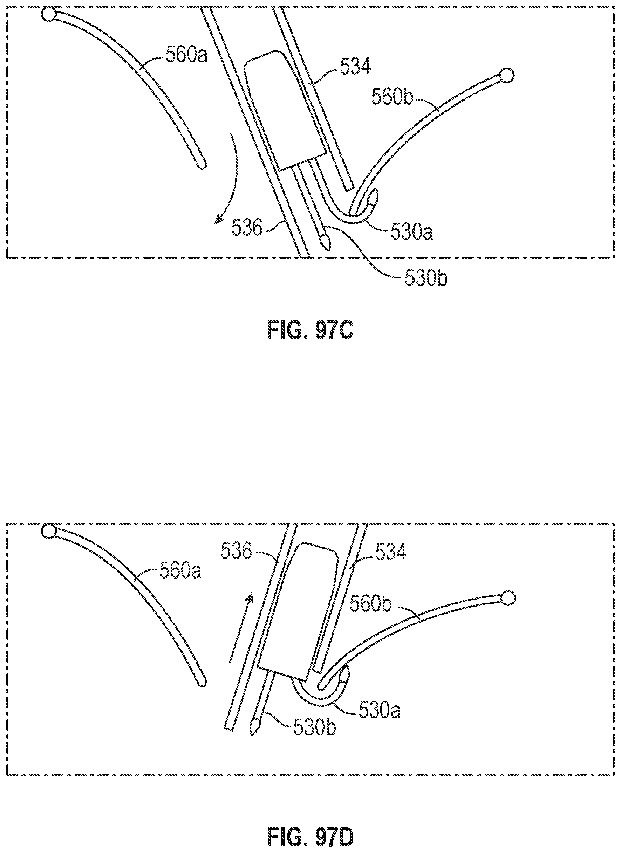

The capsule 526 may be rotated relative to the native valve to position a side 534 of the capsule 526 proximate the leaflet 560b. The capsule 526 may be rotated about the pivotal portion 558 or another method may be utilized to rotate the capsule 526. Referring to FIGS. 97B and 97C, the side 534 may be retracted to deploy the anchor 530a. The anchor 530a may hook around the distal tip of the leaflet 560b.

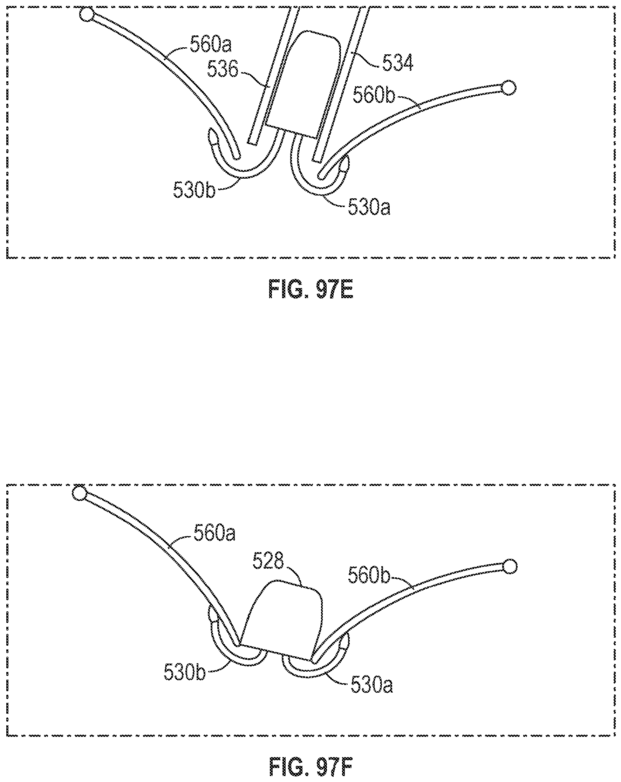

Referring to FIG. 97D, with the anchor 530a deployed, the capsule 526 may be rotated in an opposite direction to position the side 536 proximate the leaflet 560a. Referring to FIG. 97E, the side 536 may be retracted to release the anchor 530b. The anchor 530b may hook around the distal tip of the leaflet 560a. Referring to FIG. 97F, the capsule 500 may be fully retracted, with the prosthetic valve 528 deployed.

The embodiments of prosthetic valves may be utilized in a mitral valve as disclosed herein, or may be utilized in other deployment locations such as a native tricuspid valve, or other deployment locations. The number of anchors deployed by each side of the capsule may be a single anchor, or multiple anchors as desired.

In a tricuspid deployment, a portion of the anchors (e.g., four anchors) may be deployed on an anterior leaflet (which is typically the largest of the three leaflets and usually takes the shape of a semicircle). The remaining anchors (e.g., five anchors) may be deployed with another side of the capsule to the posterior and septal leaflets. The capsule may be pivoted towards the commissures of the leaflets for deployment. Other forms of deployment may be provided in embodiments.

Combinations of features of the embodiments of FIGS. 93-97F, as well as features of other embodiments herein, may be provided as desired. The configurations of the embodiments of FIGS. 93-97F may be utilized solely or may be utilized in combination with other embodiments disclosed herein.

Various modifications of the embodiments disclosed herein may be provided. Features of embodiments may be modified, substituted, excluded, or combined across embodiments as desired. Combinations of features across embodiments may be provided as desired. Combinations of features may be provided across embodiments with other features of such embodiments being excluded if desired.

The various embodiments of sealing skirts disclosed herein may have a variety of forms, including cloth skirts, foam skirts, or braided skirts as desired. Various materials may be utilized as desired.

The implants disclosed herein may include prosthetic heart valves or other forms of implants, such as stents or filters, or diagnostic devices, among others. The implants may be expandable implants configured to move from a compressed or undeployed state to an expanded or deployed state. The implants may be compressible implants configured to be compressed inward to have a reduced outer profile and to move the implant to the compressed or undeployed state.

Various forms of delivery apparatuses may be utilized with the embodiments disclosed herein. The delivery apparatuses as disclosed herein may be utilized for aortic, mitral, tricuspid, and pulmonary replacement and repair as well. The delivery apparatuses may comprise delivery apparatuses for delivery of other forms of implants, such as stents or filters, or diagnostic devices, among others.

The implants and the systems disclosed herein may be used in transcatheter mitral or tricuspid implantation, as well as aortic valve implantation (TAVI) or replacement of other native heart valves (e.g., pulmonary valves). The delivery apparatuses and the systems disclosed herein may be utilized for transarterial access, including transfemoral access, to a patient's heart. The delivery apparatuses and systems may be utilized in transcatheter percutaneous procedures, including transarterial procedures, which may be transfemoral or transjugular. Transapical procedures, among others, may also be utilized. Other procedures may be utilized as desired.

In addition, the methods herein are not limited to the methods specifically described and may include methods of utilizing the systems and apparatuses disclosed herein. The steps of the methods may be modified, excluded, or added to, with systems, apparatuses, and methods disclosed herein. The embodiments disclosed herein may comprise systems for implantation within a human body in embodiments.

In a first aspect, a prosthetic valve. The prosthetic valve may be configured to be deployed to a native valve. The prosthetic valve may include a plurality of prosthetic valve leaflets. The prosthetic valve may include a first anchor coupled to the plurality of prosthetic valve leaflets and configured to be positioned radially outward of an outward facing surface of a leaflet of the native valve. The prosthetic valve may include a second anchor coupled to the plurality of prosthetic valve leaflets and configured to abut against an inward facing surface of the leaflet of the native valve.

Implementations of the embodiments may include one or more of the following. The first anchor and the second anchor may be configured to retain the leaflet of the native valve between the first anchor and the second anchor. The first anchor and the second anchor may pinch the leaflet of the native valve between the first anchor and the second anchor. The first anchor may be configured to extend around a distal tip of the leaflet of the native valve. The second anchor may comprise a protrusion configured to extend towards the first anchor. A frame may be coupled to the plurality of prosthetic valve leaflets, wherein the first anchor is coupled to a distal portion of the frame and the second anchor is coupled to the distal portion of the frame. The frame may include an inner frame supporting the plurality of prosthetic valve leaflets and includes an outer frame positioned radially outward of the inner frame, and the first anchor is coupled to the inner frame and the second anchor is coupled to the outer frame. A sealing skirt may be coupled to the outer frame. The outer frame may comprise at least a portion of a sealing body configured to apply a seal to a portion of a heart. One or more of the first anchor or the second anchor may be biased to provide a force towards each other. The first anchor and the second anchor may be positioned at the same circumferential position. The first anchor and the second anchor may each be configured to be positioned radially outward from the plurality of prosthetic valve leaflets. The prosthetic valve may be configured to move from an undeployed configuration to a deployed configuration, and the first anchor is configured to move relatively towards the second anchor when the prosthetic valve moves from the undeployed configuration to the deployed configuration. A plurality of anchors may be coupled to the plurality of prosthetic valve leaflets and configured to be positioned radially outward of outward facing surfaces of leaflets of the native valve, and a plurality of anchors coupled to the plurality of prosthetic valve leaflets and configured to abut against inward facing surfaces of leaflets of the native valve. The prosthetic valve may be configured to be deployed to a mitral valve or a tricuspid valve.

In a second aspect, a method comprising delivering a prosthetic valve to a native valve, the prosthetic valve including a plurality of prosthetic valve leaflets and a first anchor and a second anchor; and retaining a leaflet of the native valve between the first anchor positioned radially outward of an outward facing surface of the leaflet and the second anchor abutting against an inward facing surface of the leaflet of the native valve.

Implementations of the embodiments may include one or more of the following. The method may include utilizing the first anchor and the second anchor to anchor the prosthetic valve to the native valve. The method may include pinching the leaflet of the native valve between the first anchor and the second anchor. The method may include deploying the first anchor and the second anchor sequentially. The method may include first deploying the first anchor and then deploying the second anchor. The prosthetic valve may include a frame coupled to the plurality of prosthetic valve leaflets, and wherein the first anchor is coupled to a distal portion of the frame and the second anchor is coupled to the distal portion of the frame. The frame may include an inner frame supporting the plurality of prosthetic valve leaflets and includes an outer frame positioned radially outward of the inner frame, and the first anchor is coupled to the inner frame and the second anchor is coupled to the outer frame. One or more of the first anchor or the second anchor may be biased to provide a force towards each other. The first anchor and the second anchor may be positioned at the same circumferential position. The native valve may be a mitral valve or a tricuspid valve.

In a third aspect, a prosthetic valve. The prosthetic valve may be configured to be deployed to a native valve. The prosthetic valve may include a plurality of prosthetic valve leaflets. The prosthetic valve may include a frame coupled to the plurality of prosthetic valve leaflets. The prosthetic valve may include one or more sealing prongs protruding radially outward from the frame and each configured to reduce fluid flow at a portion of the native valve.

Implementations of the embodiments may include one or more of the following. A sealing skirt may be coupled to the one or more sealing prongs and may extend circumferentially about the prosthetic valve. The frame may include an inner frame supporting the plurality of prosthetic valve leaflets and includes an outer frame positioned radially outward of the inner frame, and the one or more sealing prongs protrude radially outward from the outer frame. The outer frame may be configured to deflect into an oval shape. The one or more sealing prongs may be configured to deflect to accommodate a surface of the native valve. The one or more sealing prongs may be configured to deflect radially inward. The one or more sealing prongs may be configured to deflect circumferentially. The one or more sealing prongs may be configured to angle in a distal direction. One or more distal anchors may each be configured to extend around one or more leaflets of the native valve. The prosthetic valve may be configured to be deployed to a mitral valve or a tricuspid valve.

In a fourth aspect, a method comprising deploying a prosthetic valve to a native valve. The prosthetic valve may include a plurality of prosthetic valve leaflets, a frame coupled to the plurality of prosthetic valve leaflets, and one or more sealing prongs each configured to protrude radially outward from the frame and each configured to reduce fluid flow at a portion of the native valve.

Implementations of the embodiments may include one or more of the following. A sealing skirt may be positioned at the one or more sealing prongs and may extend circumferentially about the prosthetic valve. The frame may include an inner frame supporting the plurality of prosthetic valve leaflets and includes an outer frame positioned radially outward of the inner frame, and the one or more sealing prongs are each configured to protrude radially outward from the outer frame. The method may include deflecting the one or more sealing prongs with a portion of the native valve. The method may include deflecting the one or more sealing prongs radially inward. The method may include deflecting the one or more sealing prongs circumferentially. The method may include deflecting the one or sealing prongs to conform to a shape of an annulus of the native valve. The method may include deflecting a shape of the frame to conform to a shape of an annulus of the native valve. The method may include deflecting the shape of the frame to an oval shape. The native valve may be a mitral valve or a tricuspid valve.

In a fifth aspect, a prosthetic valve. The prosthetic valve may be configured to be deployed to a native valve. The prosthetic valve may include a plurality of prosthetic valve leaflets. The prosthetic valve may include one or more distal anchors coupled to the plurality of prosthetic valve leaflets and configured to extend over a distal tip of a leaflet of the native valve and horizontally from the distal tip of the leaflet to a tip of the respective distal anchor.

Implementations of the embodiments may include one or more of the following. The prosthetic valve may extend around a central axis, and the one or more distal anchors extend perpendicular with respect to the central axis. A frame may support the plurality of prosthetic valve leaflets and having a distal portion, and the one or more distal anchors are coupled to the distal portion of the frame. The one or more distal anchors may be configured to be positioned radially outward from the frame. The tip of the respective distal anchor may be configured to contact a wall of a heart. The tip of the respective distal anchor may be configured to be flexible. The respective distal anchor may include an undulating feature. The tip of the respective distal anchor may include a puncturing tip. The tip of the respective distal anchor may be configured to contact a ventricular wall of the heart. The prosthetic valve may be configured to be deployed to a mitral valve or a tricuspid valve.

In a sixth aspect, a method comprising deploying a prosthetic valve to a native valve. The prosthetic valve may include a plurality of prosthetic valve leaflets, and one or more distal anchors coupled to the plurality of prosthetic valve leaflets and configured to extend over a distal tip of a leaflet of the native valve and horizontally from the distal tip of the leaflet to a tip of the respective distal anchor.

Implementations of the embodiments may include one or more of the following. The method may include positioning the tip of the respective distal anchor between the leaflet of the native valve and a heart wall. The method may include contacting the tip of the respective distal anchor to a heart wall. The heart wall may be a ventricular heart wall. A frame may support the plurality of prosthetic valve leaflets and having a distal portion, and the one or more distal anchors are coupled to the distal portion of the frame. The prosthetic valve may extend around a central axis, and the one or more distal anchors extend perpendicular with respect to the central axis. The tip of the respective distal anchor may be configured to be flexible. The respective distal anchor may include an undulating feature. The tip of the respective distal anchor may include a puncturing tip. The native valve may be a mitral valve or a tricuspid valve.

In a seventh aspect, a prosthetic valve. The prosthetic valve may be configured to be deployed to a native valve. The prosthetic valve may include a plurality of prosthetic valve leaflets. The prosthetic valve may include one or more distal anchors coupled to the plurality of prosthetic valve leaflets and configured to extend over a distal tip of a leaflet of the native valve and couple to a ventricular heart wall.

Implementations of the embodiments may include one or more of the following. Each of the one or more distal anchors may include a puncturing tip for puncturing the ventricular heart wall. The puncturing tip may be angled to resist a force to the prosthetic valve in a ventricular direction. The one or more distal anchors may anchor to the leaflet of the native valve to resist a force to the prosthetic valve in an atrial direction. The one or more distal anchors may include a plurality of the distal anchors having different lengths. The one or more distal anchors may be flexible. A frameless sealing body may be positioned radially outward from the plurality of prosthetic valve leaflets and configured to seal against a portion of the native valve. The frameless sealing body may comprise a sealing skirt. A frame may support the plurality of prosthetic valve leaflets, and wherein the frameless sealing body is positioned radially outward from the frame. The prosthetic valve may be configured to be deployed to a mitral valve or a tricuspid valve.

In an eighth aspect, a method comprising delivering a prosthetic valve to a native valve, the prosthetic valve including a plurality of prosthetic valve leaflets and one or more distal anchors; extending the one or more distal anchors over a distal tip of a leaflet of the native valve; and coupling the one or more distal anchors to a ventricular heart wall.

Implementations of the embodiments may include one or more of the following. The method may comprise puncturing the ventricular heart wall with one or more puncturing tips of the one or more distal anchors. The one or more puncturing tips may be angled to resist a force to the prosthetic valve in a ventricular direction. The method may comprise abutting a frameless sealing body against a portion of the native valve to reduce fluid flow at the portion of the native valve. The frameless sealing body may comprise a sealing skirt. A frame may support the plurality of prosthetic valve leaflets, and wherein the frameless sealing body is positioned radially outward from the frame. The method may comprise anchoring the one or more distal anchors to the leaflet of the native valve to resist a force to the prosthetic valve in an atrial direction. The one or more distal anchors may include a plurality of the distal anchors having different lengths. The one or more distal anchors may be flexible. The native valve may be a mitral valve or a tricuspid valve.

In a ninth aspect, a prosthetic valve. The prosthetic valve may be configured to be deployed to a native valve. The prosthetic valve may include a plurality of prosthetic valve leaflets. The prosthetic valve may include a braid material coupled to the plurality of prosthetic valve leaflets and configured to have a shape that varies to vary an outer diameter of the prosthetic valve.

Implementations of the embodiments may include one or more of the following. The braid material may comprise one or more braided tubes. A frame may support the plurality of prosthetic valve leaflets, and the one or more braided tubes are positioned between the frame and a sealing body. The one or more braided tubes may extend circumferentially relative to the plurality of prosthetic valve leaflets. The one or more braided tubes may extend axially relative to the plurality of prosthetic valve leaflets. The braid material may be positioned radially inward of a frameless sealing body and is configured to have a shape that varies to vary an outer diameter of the frameless sealing body. The frameless sealing body may comprise a sealing skirt surrounding the braid material. The braid material may be configured to conform to a shape of an annulus of the native valve. One or more distal anchors may be configured to anchor to one or more leaflets of the native valve. The prosthetic valve may be configured to be deployed to a mitral valve or a tricuspid valve. The braid material may form an axially extending tube having an outer wall surrounding a central cavity, and the plurality of prosthetic valve leaflets are positioned within the central cavity. The prosthetic valve may include an inner frame, and the braid material forms an axially extending tube having an outer wall surrounding a central cavity, and the inner frame is positioned within the central cavity. The prosthetic valve may include a sealing skirt and the braid material is configured to be inserted axially between the sealing skirt and the plurality of prosthetic valve leaflets. The prosthetic valve may include an inner frame, and the braid material forms an axially extending tube positioned between the sealing skirt and the inner frame. The sealing skirt may include a channel for receiving the braid material.

In a tenth aspect, a method comprising delivering a prosthetic valve to a native valve. The prosthetic valve may include a plurality of prosthetic valve leaflets, and a braid material coupled to the plurality of prosthetic valve leaflets and configured to have a shape that varies to vary an outer diameter of the prosthetic valve.

Implementations of the embodiments may include one or more of the following. The braid material may comprise one or more braided tubes. The one or more braided tubes may extend circumferentially relative to the plurality of prosthetic valve leaflets. The one or more braided tubes may extend axially relative to the plurality of prosthetic valve leaflets. A frame may support the plurality of prosthetic valve leaflets, and the one or more braided tubes are positioned between the frame and a sealing body. The braid material may be positioned radially inward of a frameless sealing body and is configured to have a shape that varies to vary an outer diameter of the frameless sealing body. The frameless sealing body may comprise a sealing skirt surrounding the braid material. The method may include conforming the braid material to a shape of an annulus of the native valve. The method may include conforming the braid material to a non-circular shape. The native valve may be a mitral valve or a tricuspid valve. The braid material may form an axially extending tube having an outer wall surrounding a central cavity, and the plurality of prosthetic valve leaflets are positioned within the central cavity. The prosthetic valve may include an inner frame, and the braid material forms an axially extending tube having an outer wall surrounding a central cavity, and the inner frame is positioned within the central cavity. The prosthetic valve may include a sealing skirt and the braid material is configured to be inserted axially between the sealing skirt and the plurality of prosthetic valve leaflets. The prosthetic valve may include an inner frame, and the braid material forms an axially extending tube positioned between the sealing skirt and the inner frame. The sealing skirt may include a channel for receiving the braid material.

In an eleventh aspect, a prosthetic valve. The prosthetic valve may be configured to be deployed to a native valve. The prosthetic valve may include a plurality of prosthetic valve leaflets. The prosthetic valve may include an anchor coupled to the plurality of prosthetic valve leaflets and configured to couple to a portion of a heart. The prosthetic valve may include an indicator mechanism configured to indicate the coupling of the anchor to the portion of the heart.

Implementations of the embodiments may include one or more of the following. The indicator mechanism may be positioned on the anchor. The indicator mechanism may be configured to indicate the coupling under imaging comprising one or more of fluoroscopy or ultrasound. The indicator mechanism may be configured to move to indicate the coupling of the anchor to the portion of the heart. The indicator mechanism may be configured to move to enhance or reduce an edge. The indicator mechanism may include a protrusion configured to be depressed. The indicator mechanism may include a spring or a skirt. The portion of the heart may be a leaflet of the native valve. The anchor may comprise a distal anchor configured to extend over a portion of a leaflet of the native valve. The prosthetic valve may be configured to be deployed to a mitral valve or a tricuspid valve. The indicator mechanism may comprise a flexible portion of the anchor. The flexible portion may be configured to deflect the anchor outward upon coupling of the anchor to a leaflet of the native valve. The flexible portion of the anchor may include an undulating feature. The indicator mechanism may comprise a flexible tip of the anchor. The indicator mechanism may be configured to move in response to an anatomic force.

In a twelfth aspect, a method comprising visualizing an indicator mechanism coupled to a prosthetic valve including a plurality of prosthetic valve leaflets; and determining a coupling of an anchor of the prosthetic valve to a portion of a patient's heart based on the visualized indicator mechanism.

Implementations of the embodiments may include one or more of the following. The indicator mechanism may be positioned on the anchor. The coupling may include positioning the anchor radially outward of an outward facing surface of a leaflet of a native valve. The indicator mechanism may be positioned radially outward of the outward facing surface. The method may include visualizing the indicator mechanism with one or more of fluoroscopy or ultrasound. The indicator mechanism may move to indicate the coupling of the anchor to the portion of the patient's heart. The indicator mechanism may be configured to move to enhance or reduce an edge. The indicator mechanism may include a protrusion configured to be depressed. The indicator mechanism may include a spring or a skirt. The prosthetic valve may be deployed to a mitral valve or a tricuspid valve. The indicator mechanism may comprise a flexible portion of the anchor. The flexible portion may deflect the anchor outward upon coupling of the anchor to a leaflet of a native valve. The flexible portion of the anchor may include an undulating feature. The indicator mechanism may comprise a flexible tip of the anchor. The indicator mechanism may move in response to an anatomic force.

In a thirteenth aspect, a prosthetic valve. The prosthetic valve may be configured to be deployed to a native valve. The prosthetic valve may include a plurality of prosthetic valve leaflets. The prosthetic valve may include an inner frame supporting the plurality of prosthetic valve leaflets. The prosthetic valve may include a sealing body positioned radially outward from the inner frame and configured to seal against a portion of the native valve and configured to have an adjustable outer diameter.

Implementations of the embodiments may include one or more of the following. At least a portion of the sealing body may include an inflatable body that is configured to be inflated to adjust the adjustable outer diameter. The inflatable body may be configured to extend circumferentially about the inner frame. The inflatable body may be configured to form a ring about the inner frame. The inflatable body may form a coil extending around the inner frame. The inflatable body may include an inflation port for receiving a fill material. The inflatable body may be positioned between a sealing skirt and the inner frame. At least a portion of the sealing body may include one or more strands configured to adjust the adjustable outer diameter. A length of the one or more strands may be configured to be adjusted to adjust the adjustable outer diameter. The one or more strands may be configured to be advanced to the sealing body to adjust the length of the one or more strands. The sealing body may include a channel configured to receive the one or more strands. The one or more strands may include one or more wires. The one or more strands may include a plurality of spacer bodies. Each of the plurality of spacer bodies may be configured to engage with another one of the plurality of spacer bodies. The one or more strands may form a coil extending around the plurality of prosthetic valve leaflets. The one or more strands may extend axially relative to the plurality of prosthetic valve leaflets. The sealing body may include a braid material. The sealing body may include a sealing skirt and a chamber positioned between the sealing skirt and the inner frame, and the prosthetic valve further comprises a plurality of filler bodies configured to fill the chamber to adjust the adjustable outer diameter. The sealing body may comprise a frameless sealing body. The prosthetic valve may be configured to be deployed to a mitral valve or a tricuspid valve.

In a fourteenth aspect, a method comprising deploying a prosthetic valve to a native valve. The prosthetic valve may include a plurality of prosthetic valve leaflets, an inner frame supporting the plurality of prosthetic valve leaflets, and a sealing body positioned radially outward from the inner frame and configured to seal against a portion of the native valve and configured to have an adjustable outer diameter.

Implementations of the embodiments may include one or more of the following. At least a portion of the sealing body may include an inflatable body that is configured to be inflated to adjust the adjustable outer diameter. The inflatable body may be positioned between a sealing skirt and the inner frame. At least a portion of the sealing body may include one or more strands configured to adjust the adjustable outer diameter. The one or more strands may include one or more wires. The one or more strands may include a plurality of spacer bodies. The sealing body may include a braid material. The sealing body may include a sealing skirt and a chamber positioned between the sealing skirt and the inner frame, and the prosthetic valve further comprises a plurality of filler bodies configured to fill the chamber to adjust the adjustable outer diameter. The sealing body may comprise a frameless sealing body. The native valve may be a mitral valve or a tricuspid valve.

In a fifteenth aspect, a prosthetic valve. The prosthetic valve may be configured to be deployed to a native valve. The prosthetic valve may include a plurality of prosthetic valve leaflets. The prosthetic valve may include an inner frame supporting the plurality of prosthetic valve leaflets. The prosthetic valve may include an inflatable body positioned radially outward from the inner frame and configured to seal against a portion of the native valve.

Implementations of the embodiments may include one or more of the following. The inflatable body may be positioned radially outward of the plurality of prosthetic valve leaflets. The inflatable body may be configured to form a ring about the inner frame. The inflatable body may form a coil extending around the inner frame. The inflatable body may include an inflation port for receiving a fill material. The inflatable body may be positioned between a sealing skirt and the inner frame. The inflatable body may be configured to apply a radially outward force to the sealing skirt. One or more anchors may be configured to extend over a distal tip of a native valve leaflet. The inner frame may include a proximal portion and a distal portion, and the prosthetic valve further comprises one or more anchors coupled to the distal portion of the inner frame. The prosthetic valve may be configured to be deployed to a mitral valve or a tricuspid valve.

In a sixteenth aspect, a method comprising deploying a prosthetic valve to a native valve. The prosthetic valve may include a plurality of prosthetic valve leaflets, an inner frame supporting the plurality of prosthetic valve leaflets, and an inflatable body positioned radially outward from the inner frame and configured to seal against a portion of the native valve.

Implementations of the embodiments may include one or more of the following. The inflatable body may be positioned radially outward of the plurality of prosthetic valve leaflets. The inflatable body may be configured to form a ring about the inner frame. The inflatable body may form a coil extending around the inner frame. The inflatable body may include an inflation port for receiving a fill material. The inflatable body may be positioned between a sealing skirt and the inner frame. The inflatable body may be configured to apply a radially outward force to the sealing skirt. The method may include one or more anchors configured to extend over a distal tip of a native valve leaflet. The inner frame may include a proximal portion and a distal portion, and the prosthetic valve further comprises one or more anchors coupled to the distal portion of the inner frame. The native valve may be a mitral valve or a tricuspid valve.

In a seventeenth aspect, a prosthetic valve. The prosthetic valve may be configured to be deployed to a native valve. The prosthetic valve may include a plurality of prosthetic valve leaflets. The prosthetic valve may include a sealing body positioned radially outward from the plurality of prosthetic valve leaflets and including one or more strands configured to seal the sealing body against a portion of the native valve.

Implementations of the embodiments may include one or more of the following. The one or more strands may include one or more wires. The one or more strands may include a plurality of spacer bodies. Each of the plurality of spacer bodies may be configured to engage with another one of the plurality of spacer bodies. The one or more strands may form a coil extending around the plurality of prosthetic valve leaflets. The one or more strands may extend axially relative to the plurality of prosthetic valve leaflets. A length of the one or more strands may be configured to be adjusted to adjust an outer diameter of the sealing body. The one or more strands may be configured to be advanced to the sealing body to adjust the length of the one or more strands. The sealing body may include a channel configured to receive the one or more strands. The one or more strands may be configured to apply a radially outward force to the sealing body.

In an eighteenth aspect, a method comprising deploying a prosthetic valve to a native valve. The prosthetic valve may include a plurality of prosthetic valve leaflets, and a sealing body positioned radially outward from the plurality of prosthetic valve leaflets and including one or more strands configured to seal the sealing body against a portion of the native valve.

Implementations of the embodiments may include one or more of the following. The one or more strands may include one or more wires. The one or more strands may include a plurality of spacer bodies. Each of the plurality of spacer bodies may be configured to engage with another one of the plurality of spacer bodies. The one or more strands may form a coil extending around the plurality of prosthetic valve leaflets. The one or more strands may extend axially relative to the plurality of prosthetic valve leaflets. A length of the one or more strands may be configured to be adjusted to adjust an outer diameter of the sealing body. The one or more strands may be configured to be advanced to the sealing body to adjust the length of the one or more strands. The sealing body may include a channel configured to receive the one or more strands. The one or more strands may be configured to apply a radially outward force to the sealing body.

In a nineteenth aspect, a prosthetic valve. The prosthetic valve may be configured to be deployed to a native valve. The prosthetic valve may include a plurality of prosthetic valve leaflets. The prosthetic valve may include a frame supporting the plurality of prosthetic valve leaflets. The prosthetic valve may include a frameless sealing body positioned radially outward from the plurality of prosthetic valve leaflets and configured to seal against a portion of the native valve.

Implementations of the embodiments may include one or more of the following. At least a portion of the frameless sealing body includes an inflatable body that is configured to be inflated. The inflatable body may include an inflation port for receiving a fill material. At least a portion of the frameless sealing body may include one or more strands configured to seal the frameless sealing body against the portion of the native valve. A length of the one or more strands may be configured to be adjusted to adjust an outer diameter of the frameless sealing body. The one or more strands may be configured to be advanced to the frameless sealing body to adjust the length of the one or more strands. The one or more strands may be configured to apply a radially outward force to the frameless sealing body. The one or more strands may be biased to apply the radially outward force to the frameless sealing body. The frameless sealing body may include a sealing skirt and a chamber positioned between the sealing skirt and the frame, and the prosthetic valve further comprises a plurality of filler bodies configured to fill the chamber to adjust an outer diameter of the frameless sealing body. The prosthetic valve may be configured to be deployed to a mitral valve or a tricuspid valve.

In a twentieth aspect, a method comprising deploying a prosthetic valve to a native valve. The prosthetic valve may include a plurality of prosthetic valve leaflets, a frame supporting the plurality of prosthetic valve leaflets, and a frameless sealing body positioned radially outward from the plurality of prosthetic valve leaflets and configured to seal against a portion of the native valve.

Implementations of the embodiments may include one or more of the following. At least a portion of the frameless sealing body may include an inflatable body that is configured to be inflated. The inflatable body may include an inflation port for receiving a fill material. At least a portion of the frameless sealing body may include one or more strands configured to seal the frameless sealing body against a portion of the native valve. A length of the one or more strands may be configured to be adjusted to adjust an outer diameter of the frameless sealing body. The one or more strands may be configured to be advanced to the frameless sealing body to adjust the length of the one or more strands. The one or more strands may be configured to apply a radially outward force to the frameless sealing body. The one or more strands may be biased to apply the radially outward force to the frameless sealing body. The frameless sealing body may include a sealing skirt and a chamber positioned between the sealing skirt and the frame, and the prosthetic valve further comprises a plurality of filler bodies configured to fill the chamber to adjust an outer diameter of the frameless sealing body. The native valve may be a mitral valve or a tricuspid valve.

In a twenty-first aspect, a prosthetic valve. The prosthetic valve may be configured to be deployed to a native valve. The prosthetic valve may include a plurality of prosthetic valve leaflets. The prosthetic valve may include a valve body supporting the plurality of prosthetic valve leaflets. The prosthetic valve may include one or more anchors configured to anchor the valve body to the native valve and each having a diameter configured to increase.

Implementations of the embodiments may include one or more of the following. The one or more anchors may include an inflatable body configured to increase the diameter of the one or more anchors. The inflatable body may include an inflation port for receiving a fill material. The one or more anchors may be configured to extend over a distal tip of a native valve leaflet and the inflatable body is configured to press the native valve leaflet against the valve body to seal against a portion of the native valve. The one or more anchors may each comprise an arm extending to a distal tip, and the distal tip of the one or more anchors has the diameter configured to increase. The arm may be configured to extend over a distal tip of a native valve leaflet. The one or more anchors may be selfexpandable to increase the diameter. The valve body may include an outer surface configured to seal against a portion of the native valve. The valve body may include a sealing skirt. The native valve may be a mitral valve or a tricuspid valve.

In a twenty-second aspect, a method comprising deploying a prosthetic valve to a native valve. The prosthetic valve may include a plurality of prosthetic valve leaflets, a valve body supporting the plurality of prosthetic valve leaflets, and one or more anchors configured to anchor the valve body to the native valve and each having a diameter configured to increase.

Implementations of the embodiments may include one or more of the following. The one or more anchors may include an inflatable body configured to increase the diameter of the one or more anchors. The inflatable body may include an inflation port for receiving a fill material. The one or more anchors may be configured to extend over a distal tip of a native valve leaflet and the inflatable body is configured to press the native valve leaflet against the valve body to seal against a portion of the native valve. The one or more anchors may each comprise an arm extending to a distal tip, and the distal tip of the one or more anchors has the diameter configured to increase. The arm may be configured to extend over a distal tip of a native valve leaflet. The one or more anchors may be selfexpandable to increase the diameter. The valve body may include an outer surface configured to seal against a portion of the native valve. The valve body may include a sealing skirt. The native valve may be a mitral valve or a tricuspid valve.

In a twenty-third aspect, a prosthetic valve. The prosthetic valve may be configured to be deployed to a native valve. The prosthetic valve may include a plurality of prosthetic valve leaflets. The prosthetic valve may include a valve body having a proximal portion and a distal portion and supporting the plurality of prosthetic valve leaflets. The prosthetic valve may include one or more anchors positioned at the distal portion of the valve body, each of the one or more anchors having a first portion extending proximally to a tip of the respective anchor, the first portion passing through at least a portion of the valve body.

Implementations of the embodiments may include one or more of the following. The tip of the respective anchor may be positioned at least partially within the valve body. The valve body may include a plurality of pockets configured to receive the first portion of the one or more anchors. The valve body may include a fuzzy skirt and the first portion passes through the fuzzy skirt. The one or more anchors may include a plurality of the anchors forming an outer diameter, and the valve body has an outer diameter that is at or greater than the outer diameter of the plurality of anchors. The plurality of anchors may be spaced circumferentially from each other. The one or more anchors may each be configured to hook around a distal end of a native leaflet of the native valve. The one or more anchors may be configured to retain the native leaflet between the one or more anchors and the valve body. The valve body may include an inner frame and a sealing body positioned radially outward of the inner frame, and the first portion passes through the sealing body. The prosthetic valve may be configured to be deployed to a mitral valve or a tricuspid valve.

In a twenty-fourth aspect, a method comprising deploying a prosthetic valve to a native valve. The prosthetic valve may include a plurality of prosthetic valve leaflets, a valve body having a proximal portion and a distal portion and supporting the plurality of prosthetic valve leaflets, and one or more anchors positioned at the distal portion of the valve body, each of the one or more anchors having a first portion extending proximally to a tip of the respective anchor, the first portion passing through at least a portion of the valve body.

Implementations of the embodiments may include one or more of the following. The tip of the respective anchor may be positioned at least partially within the valve body. The valve body may include a plurality of pockets configured to receive the first portion of the one or more anchors. The valve body may include a fuzzy skirt and the first portion passes through the fuzzy skirt. The one or more anchors may include a plurality of the anchors forming an outer diameter, and the valve body has an outer diameter that is at or greater than the outer diameter of the plurality of anchors. The plurality of anchors may be spaced circumferentially from each other. The one or more anchors may each be configured to hook around a distal end of a native leaflet of the native valve. The one or more anchors may be configured to retain the native leaflet between the one or more anchors and the valve body. The valve body may include an inner frame and a sealing body positioned radially outward of the inner frame, and the first portion passes through the sealing body. The native valve may be a mitral valve or a tricuspid valve.

In a twenty-fifth aspect, a prosthetic valve. The prosthetic valve may be configured to be deployed to a native valve. The prosthetic valve may include a plurality of prosthetic valve leaflets. The prosthetic valve may include an inner frame supporting the plurality of prosthetic valve leaflets and having a proximal portion and a distal portion. The prosthetic valve may include a plurality of anchors positioned at the distal portion of the inner frame and configured to couple to the native valve. The prosthetic valve may include a sealing body positioned radially outward from the inner frame and having a proximal end and a distal end that is coupled to the distal portion of the inner frame such that the sealing body is configured to move with respect to the inner frame.

Implementations of the embodiments may include one or more of the following. One or more flexible bodies may couple the distal end of the sealing body to the distal portion of the inner frame and allow the sealing body to move with respect to the inner frame. The one or more flexible bodies may comprise a fabric or a shape memory material. The inner frame may extend about a first axis and the sealing body extends about a second axis, and the distal end is coupled to the distal portion of the inner frame such that the sealing body is configured to tilt with respect to the inner frame to offset the first axis from the second axis. The distal end may be coupled to the distal portion of the inner frame such that the sealing body is configured to move axially with respect to the inner frame. The sealing body may be spaced from the inner frame with an annular gap and the proximal end of the sealing body forms an opening for the annular gap. The inner frame may comprise a first frame and the sealing body includes a second frame and a sealing skirt. The sealing body may be tapered radially inward in a direction from the proximal end towards the distal end. The plurality of anchors may be configured to hook around a distal end of a native leaflet of the native valve and retain the native leaflet between the plurality of anchors and the sealing body. The prosthetic valve may be configured to be deployed to a mitral valve or a tricuspid valve.

In a twenty-sixth aspect, a method comprising deploying a prosthetic valve to a native valve. The prosthetic valve may include a plurality of prosthetic valve leaflets, an inner frame supporting the plurality of prosthetic valve leaflets and having a proximal portion and a distal portion, a plurality of anchors positioned at the distal portion of the inner frame and configured to couple to the native valve, and a sealing body positioned radially outward from the inner frame and having a proximal end and a distal end that is coupled to the distal portion of the inner frame such that the sealing body is configured to move with respect to the inner frame.

Implementations of the embodiments may include one or more of the following. One or more flexible bodies may couple the distal end of the sealing body to the distal portion of the inner frame and allow the sealing body to move with respect to the inner frame. The one or more flexible bodies may comprise a fabric or a shape memory material. The inner frame may extend about a first axis and the sealing body extends about a second axis, and the distal end is coupled to the distal portion of the inner frame such that the sealing body is configured to tilt with respect to the inner frame to offset the first axis from the second axis. The distal end may be coupled to the distal portion of the inner frame such that the sealing body is configured to move axially with respect to the inner frame. The sealing body may be spaced from the inner frame with an annular gap and the proximal end of the sealing body forms an opening for the annular gap. The inner frame may comprise a first frame and the sealing body includes a second frame and a sealing skirt. The sealing body may be tapered radially inward in a direction from the proximal end towards the distal end. The plurality of anchors may be configured to hook around a distal end of a native leaflet of the native valve and retain the native leaflet between the plurality of anchors and the sealing body. The native valve may be a mitral valve or a tricuspid valve.

In a twenty-seventh aspect, a system. The system may be for delivery of a prosthetic valve into a portion of a patient's body. The system may include an elongate shaft configured for insertion into a portion of the patient's body. The system may include a capsule coupled to the elongate shaft and configured to at least partially release the prosthetic valve, the capsule extending about an axis and including at least two sides surrounding the axis, a first side of the at least two sides configured to retract axially relative to a second side of the at least two sides.

Implementations of the embodiments may include one or more of the following. The first side may comprise a half of the capsule. The second side may comprise a half of the capsule. The first side may comprise a half-cylinder and the second side comprises a half-cylinder. The first side may be configured to retract independently of the second side. A split may extend along an axial plane of the capsule and separates the first side from the second side. The axis may extend within the axial plane. The capsule may be configured to pivot in one or more planes of the axis. The prosthetic valve may include a plurality of anchors, and wherein the first side is configured to retract to release a first anchor of the plurality of anchors. The second side may be configured to retract to release a second anchor of the plurality of anchors.

In a twenty-eighth aspect, a method comprising utilizing a delivery system to deploy a prosthetic valve to a native valve. The delivery system may include an elongate shaft configured for insertion into a portion of a patient's body, and a capsule coupled to the elongate shaft and configured to at least partially release the prosthetic valve, the capsule extending about an axis and including at least two sides surrounding the axis, a first side of the at least two sides configured to retract axially relative to a second side of the at least two sides.

Implementations of the embodiments may include one or more of the following. The first side may comprise a half of the capsule. The second side may comprise a half of the capsule. The first side may comprise a half-cylinder and the second side comprises a half-cylinder. The first side may be configured to retract independently of the second side. A split may extend along an axial plane of the capsule separates the first side from the second side. The axis may extend within the axial plane. The capsule may be configured to pivot in one or more planes of the axis. The prosthetic valve may include a plurality of anchors, and wherein the first side is configured to retract to release a first anchor of the plurality of anchors. The second side may be configured to retract to release a second anchor of the plurality of anchors.

Any of the features of an embodiment of any of the aspects, including but not limited to any embodiments of any of the first through twenty-eighth aspects referred to above, is applicable to all other aspects and embodiments identified herein, including but not limited to any embodiments of any of the first through twenty-eighth aspects referred to above. Moreover, any of the features of an embodiment of the various aspects, including but not limited to any embodiments of any of the first through twenty-eighth aspects referred to above, is independently combinable, partly or wholly with other embodiments described herein in any way, e.g., one, two, or three or more embodiments may be combinable in whole or in part. Further, any of the features of an embodiment of the various aspects, including but not limited to any embodiments of any of the first through twenty-eighth aspects referred to above, may be made optional to other aspects or embodiments. Any aspect or embodiment of a method can be performed by a system or apparatus of another aspect or embodiment, and any aspect or embodiment of a system or apparatus can be configured to perform a method of another aspect or embodiment, including but not limited to any embodiments of any of the first through twenty-eighth aspects referred to above.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein without departing from the scope of the present specification. Lastly, the terminology used herein is for the purpose of describing particular embodiments only, and is not intended to limit the scope of systems, apparatuses, and methods as disclosed herein, which is defined solely by the claims. Accordingly, the systems, apparatuses, and methods are not limited to that precisely as shown and described.

## Claims

1. A prosthetic valve (240) configured to be deployed to a native valve, the prosthetic valve comprising:
a plurality of prosthetic valve leaflets (16);
an inner frame (242) supporting the plurality of prosthetic valve leaflets; and
a sealing body (246) positioned radially outwardly from the inner frame and configured to seal against a portion of the native valve, wherein the sealing body has a selectively adjustable outer diameter;
wherein at least a portion of the sealing body includes one or more strands (250) configured to adjust the adjustable outer diameter;
wherein a length of the one or more strands is configured to be adjusted to adjust the adjustable outer diameter; and
wherein the one or more strands are configured to be advanced or retracted relative to the sealing body to adjust the adjustable outer diameter.

2. The prosthetic valve of claim 1, wherein at least a portion of the sealing body includes an inflatable body (342) for adjusting the outer diameter.

3. The prosthetic valve of claim 2, wherein the inflatable body is configured to form a ring that encircles the inner frame.

4. The prosthetic valve of claim 2 or claim 3, wherein the inflatable body forms a coil extending around the inner frame.

5. The prosthetic valve of any of claims 2-4, wherein the inflatable body is positioned between a sealing skirt (350) and the inner frame.

6. The prosthetic valve of any of claims 2-5, further comprising a hardenable material for filling the inflatable body.

7. The prosthetic valve of any of claims 1-6, wherein the one or more strands include one or more wires.

8. The prosthetic valve of any of claims 1-7, wherein the one or more strands include a plurality of spacer bodies (264, 280).

9. The prosthetic valve of claim 8, wherein each of the plurality of spacer bodies (280) are configured to engage with another one of the plurality of spacer bodies.

10. The prosthetic valve of any of claims 1-9 , wherein the one or more strands form a coil extending around the plurality of prosthetic valve leaflets.

11. The prosthetic valve of any of claims 1-10, wherein the one or more strands extend axially relative to the plurality of prosthetic valve leaflets.

12. The prosthetic valve of any of claims 1-11, wherein the sealing body includes a sealing skirt (350) and wherein a chamber (420) is positioned between the sealing skirt and the inner frame.

13. The prosthetic valve of claim 12, further comprising a plurality of filler bodies (419) sized to be delivered into the chamber for adjusting the adjustable outer diameter.

## Patentansprüche

1. Klappenprothese (240), gestaltet zum Einsetzen in eine natürliche Klappe, wobei die Klappenprothese umfasst:
eine Vielzahl von Klappenprothesensegeln (16);
einen inneren Rahmen (242), der die Vielzahl von Klappenprothesensegeln trägt; und
einen Dichtungskörper (246), der radial außerhalb des inneren Rahmens angeordnet ist und dafür gestaltet ist, gegen einen Abschnitt der nativen Klappe abzudichten, wobei der Dichtungskörper einen selektiv einstellbaren Außendurchmesser aufweist;
wobei wenigstens ein Teil des Dichtungskörpers einen oder mehrere Stränge (250) enthält, die dafür gestaltet sind, den einstellbaren Außendurchmesser einzustellen;
wobei eine Länge des einen oder der mehreren Stränge dafür gestaltet ist, eingestellt zu werden, um den einstellbaren Außendurchmesser einzustellen; und
wobei der eine oder die mehreren Stränge dafür gestaltet sind, relativ zu dem Dichtungskörper vorgeschoben oder zurückgezogen zu werden, um den einstellbaren Außendurchmesser einzustellen.

2. Klappenprothese nach Anspruch 1, wobei wenigstens ein Abschnitt des Dichtungskörpers einen aufblähbaren Körper (342) zum Einstellen des Außendurchmessers enthält.

3. Klappenprothese nach Anspruch 2, wobei der aufblähbare Körper dafür gestaltet ist, einen Ring zu bilden, der den inneren Rahmen umgibt.

4. Klappenprothese nach Anspruch 2 oder Anspruch 3, wobei der aufblähbare Körper eine Wendel bildet, die um den inneren Rahmen verläuft.

5. Klappenprothese nach einem der Ansprüche 2-4, wobei der aufblähbare Körper zwischen einer Dichtungsschürze (350) und dem inneren Rahmen angeordnet ist.

6. Klappenprothese nach einem der Ansprüche 2-5, ferner umfassend ein härtbares Material zum Füllen des aufblähbaren Körpers.

7. Klappenprothese nach einem der Ansprüche 1-6, wobei der eine oder die mehreren Stränge einen oder mehrere Drähte enthalten.

8. Klappenprothese nach einem der Ansprüche 1-7, wobei der eine oder die mehreren Stränge eine Vielzahl von Abstandshalterkörpern (264, 280) enthalten.

9. Klappenprothese nach Anspruch 8, wobei jeder der Vielzahl von Abstandshalterkörpern (280) zum Eingriff mit einem anderen der Vielzahl von Abstandshalterkörpern gestaltet ist.

10. Klappenprothese nach einem der Ansprüche 1-9, wobei der eine oder die mehreren Stränge eine Wendel bilden, die um die Vielzahl von Klappenprothesensegeln verläuft.

11. Klappenprothese nach einem der Ansprüche 1-10, wobei der eine oder die mehreren Stränge axial relativ zu der Vielzahl von Klappenprothesensegeln verlaufen.

12. Klappenprothese nach einem der Ansprüche 1-11, wobei der Dichtungskörper eine Dichtungsschürze (350) enthält und wobei eine Kammer (420) zwischen der Dichtungsschürze und dem inneren Rahmen angeordnet ist.

13. Klappenprothese nach Anspruch 12, ferner umfassend eine Vielzahl von Füllkörpern (419), die bemessen sind, in die Kammer zum Einstellen des einstellbaren Außendurchmessers abgegeben zu werden.

## Revendications

1. Valvule prothétique (240) configurée pour être déployée sur une valvule native, la valvule prothétique comprenant :
une pluralité de feuillets de valvule prothétique (16) ; un cadre interne (242) supportant la pluralité de feuillets de valvule prothétique ; et
un corps d'étanchéité (246) positionné radialement vers l'extérieur à partir du cadre interne et configuré pour assurer l'étanchéité contre une partie de la valvule native, le corps d'étanchéité ayant un diamètre externe réglable sélectivement ;
au moins une partie du corps d'étanchéité comprenant un ou plusieurs brins (250) configurés pour régler le diamètre externe réglable ;
une longueur du ou des brins étant configurée pour être réglée pour régler le diamètre externe réglable ; et
le ou les brins étant configurés pour être avancés ou rétractés par rapport au corps d'étanchéité pour régler le diamètre externe réglable.

2. Valvule prothétique selon la revendication 1, au moins une partie du corps d'étanchéité comprenant un corps gonflable (342) pour régler le diamètre externe.

3. Valvule prothétique selon la revendication 2, le corps gonflable étant configuré pour former un anneau qui encercle le cadre interne.

4. Valvule prothétique selon la revendication 2 ou la revendication 3, le corps gonflable formant une bobine s'étendant autour du cadre interne.

5. Valvule prothétique selon l'une quelconque des revendications 2 à 4, le corps gonflable étant positionné entre une jupe d'étanchéité (350) et le cadre interne.

6. Valvule prothétique selon l'une quelconque des revendications 2 à 5, comprenant en outre un matériau durcissable pour remplir le corps gonflable.

7. Valvule prothétique selon l'une quelconque des revendications 1 à 6, le ou les brins comprenant un ou plusieurs fils.

8. Valvule prothétique selon l'une quelconque des revendications 1 à 7, le ou les brins comprenant une pluralité de corps d'espacement (264, 280).

9. Valvule prothétique selon la revendication 8, chacun de la pluralité de corps d'entretoise (280) étant configuré pour venir en prise avec un autre corps de la pluralité de corps d'entretoise.

10. Valvule prothétique selon l'une quelconque des revendications 1 à 9, le ou les brins formant une bobine s'étendant autour de la pluralité de feuillets de valvule prothétique.

11. Valvule prothétique selon l'une quelconque des revendications 1 à 10, le ou les brins s'étendant axialement par rapport à la pluralité de feuillets de valvule prothétique.

12. Valvule prothétique selon l'une quelconque des revendications 1 à 11, le corps d'étanchéité comprenant une jupe d'étanchéité (350) et une chambre (420) étant positionnée entre la jupe d'étanchéité et le cadre interne.

13. Valvule prothétique selon la revendication 12, comprenant en outre une pluralité de corps de remplissage (419) dimensionnés pour être délivrés dans la chambre pour régler le diamètre externe réglable.
